(19) Europäisches Patentamt
European Patent Office
Office européen des brevets

(11) EP 3 612 624 B1

(12) EUROPEAN PATENT SPECIFICATION

(45) Date of publication and mention of the grant of the patent:
**25.09.2024 Bulletin 2024/39**

(21) Application number: **18724470.2**

(22) Date of filing: **20.04.2018**

(51) International Patent Classification (IPC):
***C12N 5/0789*** *(2010.01)* ***C12N 5/0786*** *(2010.01)*
***C12N 5/10*** *(2006.01)* ***A61K 31/405*** *(2006.01)*
***A61K 35/15*** *(2015.01)* ***A61K 38/21*** *(2006.01)*
***A61K 39/395*** *(2006.01)* ***A61K 48/00*** *(2006.01)*
***A61P 35/00*** *(2006.01)*

(52) Cooperative Patent Classification (CPC):
(C-Sets available)
**C12N 5/0647; A01K 67/027; A61K 31/405; A61K 38/21; A61K 39/0011; A61K 39/3955; A61K 39/461; A61K 39/4611; A61K 39/4631; A61K 39/464412; A61K 39/464438; A61K 45/06; A61K 48/005; A61K 48/0083; A61P 35/00;** (Cont.)

(86) International application number:
**PCT/EP2018/060238**

(87) International publication number:
**WO 2018/193119 (25.10.2018 Gazette 2018/43)**

(54) **GENE THERAPY**

GENTHERAPIE

THÉRAPIE GÉNIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.04.2017 GB 201706410**
**30.01.2018 GB 201801511**

(43) Date of publication of application:
**26.02.2020 Bulletin 2020/09**

(73) Proprietors:
- **Ospedale San Raffaele S.r.l.**
**20132 Milano (IT)**
- **Fondazione Telethon ETS**
**00185 Roma (IT)**

(72) Inventors:
- **NALDINI, Luigi**
**20132 Milan (IT)**
- **ESCOBAR, Giulia**
**20132 Milan (IT)**
- **GENTNER, Bernhard Rudolf**
**20132 Milan (IT)**
- **MUCCI, Adele**
**20132 Milan (IT)**

(74) Representative: **D Young & Co LLP**
**3 Noble Street**
**London EC2V 7BQ (GB)**

(56) References cited:
**EP-A1- 2 856 876 EP-A1- 2 856 876**

- **ESCOBAR G ET AL: "Genetic Engineering of Hematopoiesis for Targeted IFN-alpha Delivery Inhibits Breast Cancer Progression", SCIENCE TRANSLATIONAL MEDICINE, vol. 6, no. 217, 217RA3, 1 January 2014 (2014-01-01), XP055492866, ISSN: 1946-6234, DOI: 10.1126/scitranslmed.3006353**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- CATARINELLA M ET AL: "IFN.alpha gene/cell therapy curbs colorectal cancer colonization of the liver by acting on the hepatic microenvironment", EMBO MOLECULAR MEDICINE, vol. 8, no. 2, 1 February 2016 (2016-02-01), pages 155 - 170, XP055492868, ISSN: 1757-4676, DOI: 10.15252/emmm.201505395
- GENTNER B ET AL: "Identification of Hematopoietic Stem Cell-Specific miRNAs Enables Gene Therapy of Globoid Cell Leukodystrophy", SCIENCE TRANSLATIONAL MEDICINE, vol. 2, no. 58, 58RA84, 17 November 2010 (2010-11-17), XP055492874, ISSN: 1946-6234, DOI: 10.1126/scitranslmed.3001522
- CHIRIACO M ET AL: "Dual-regulated Lentiviral Vector for Gene Therapy of X-linked Chronic Granulomatosis", MOLECULAR THERAPY, vol. 22, no. 8, 24 June 2014 (2014-06-24), pages 1472 - 1483, XP055492876, ISSN: 1525-0016, DOI: 10.1038/mt.2014.87
- SHARMA P & ALLISON J P: "Immune Checkpoint Targeting in Cancer Therapy: Toward Combination Strategies with Curative Potential", CELL, vol. 161, no. 2, 9 April 2015 (2015-04-09), pages 205 - 214, XP055427087, ISSN: 0092-8674, DOI: 10.1016/j.cell.2015.03.030
- TARHINI A A ET AL: "Safety and Efficacy of Combination Immunotherapy With Interferon Alfa-2b and Tremelimumab in Patients With Stage IV Melanoma", JOURNAL OF CLINICAL ONCOLOGY, vol. 30, no. 3, 20 January 2012 (2012-01-20), pages 322 - 328, XP055492880, ISSN: 0732-183X, DOI: 10.1200/JCO.2011.37.5394
- GENTNER B ET AL: "Autologous CD34+-Enriched Hematopoietic Progenitor Cells Genetically Modified for Human Interferon-Alpha2 (Temferon), Rapidly Engraft and Mature in Patients with Glioblastoma Multiforme (TEM-GBM_001 Study)", MOLECULAR THERAPY, vol. 28, no. 4 Suppl. 1, 1345, 28 April 2020 (2020-04-28), pages 576, XP055978815, ISSN: 1525-0016, DOI: 10.1016/j.ymthe.2020.04.019
- CICERI F ET AL: "Autologous CD34+ enriched hematopoietic progenitor cells genetically modified for human interferon-alpha2, rapidly engraft and mature in patients with glioblastoma multiforme (tem-gbm-001 study)", HEMASPHERE, vol. 4, no. Suppl. 1, S283, June 2020 (2020-06-01), pages 103 - 103, XP055978830, ISSN: 2572-9241, DOI: 10.1097/HS9.0000000000000404
- ESCOBAR G ET AL: "Genetic Engineering of Hematopoiesis for Targeted IFN-alpha Delivery Inhibits Breast Cancer Progression", SCIENCE TRANSLATIONAL MEDICINE, vol. 6, no. 217, 217RA3, 1 January 2014 (2014-01-01), XP055492866, ISSN: 1946-6234, DOI: 10.1126/scitranslmed.3006353

- CATARINELLA M ET AL: "IFN.alpha gene/cell therapy curbs colorectal cancer colonization of the liver by acting on the hepatic microenvironment", EMBO MOLECULAR MEDICINE, vol. 8, no. 2, 1 February 2016 (2016-02-01), pages 155 - 170, XP055492868, ISSN: 1757-4676, DOI: 10.15252/emmm.201505395
- GENTNER B ET AL: "Identification of Hematopoietic Stem Cell-Specific miRNAs Enables Gene Therapy of Globoid Cell Leukodystrophy", SCIENCE TRANSLATIONAL MEDICINE, vol. 2, no. 58, 58RA84, 17 November 2010 (2010-11-17), XP055492874, ISSN: 1946-6234, DOI: 10.1126/scitranslmed.3001522
- CHIRIACO M ET AL: "Dual-regulated Lentiviral Vector for Gene Therapy of X-linked Chronic Granulomatosis", MOLECULAR THERAPY, vol. 22, no. 8, 24 June 2014 (2014-06-24), pages 1472 - 1483, XP055492876, ISSN: 1525-0016, DOI: 10.1038/mt.2014.87
- SHARMA P & ALLISON J P: "Immune Checkpoint Targeting in Cancer Therapy: Toward Combination Strategies with Curative Potential", CELL, vol. 161, no. 2, 9 April 2015 (2015-04-09), pages 205 - 214, XP055427087, ISSN: 0092-8674, DOI: 10.1016/j.cell.2015.03.030
- TARHINI A A ET AL: "Safety and Efficacy of Combination Immunotherapy With Interferon Alfa-2b and Tremelimumab in Patients With Stage IV Melanoma", JOURNAL OF CLINICAL ONCOLOGY, vol. 30, no. 3, 20 January 2012 (2012-01-20), pages 322 - 328, XP055492880, ISSN: 0732-183X, DOI: 10.1200/JCO.2011.37.5394
- GENTNER B ET AL: "Autologous CD34+-Enriched Hematopoietic Progenitor Cells Genetically Modified for Human Interferon-Alpha2 (Temferon), Rapidly Engraft and Mature in Patients with Glioblastoma Multiforme (TEM-GBM_001 Study)", MOLECULAR THERAPY, vol. 28, no. 4 Suppl. 1, 1345, 28 April 2020 (2020-04-28), pages 576, XP055978815, ISSN: 1525-0016, DOI: 10.1016/j.ymthe.2020.04.019
- ESCOBAR G ET AL: "Genetic Engineering of Hematopoiesis for Targeted IFN-alpha Delivery Inhibits Breast Cancer Progression", SCIENCE TRANSLATIONAL MEDICINE, vol. 6, no. 217, 217RA3, 1 January 2014 (2014-01-01), XP055492866, ISSN: 1946-6234, DOI: 10.1126/scitranslmed.3006353
- CATARINELLA M ET AL: "IFNalpha gene/cell therapy curbs colorectal cancer colonization of the liver by acting on the hepatic microenvironment", EMBO MOLECULAR MEDICINE, vol. 8, no. 2, 14 January 2016 (2016-01-14), pages 155 - 170, XP055492868, ISSN: 1757-4676, DOI: 10.15252/emmm.201505395

- **GENTNER B ET AL: "Identification of Hematopoietic Stem Cell-Specific miRNAs Enables Gene Therapy of Globoid Cell Leukodystrophy", SCIENCE TRANSLATIONAL MEDICINE, vol. 2, no. 58, 58RA84, 17 November 2010 (2010-11-17), XP055492874, ISSN: 1946-6234, DOI: 10.1126/scitranslmed.3001522**
- **CHIRIACO M ET AL: "Dual-regulated Lentiviral Vector for Gene Therapy of X-linked Chronic Granulomatosis", MOLECULAR THERAPY, vol. 22, no. 8, 24 June 2014 (2014-06-24), US, pages 1472 - 1483, XP055492876, ISSN: 1525-0016, DOI: 10.1038/mt.2014.87**
- **SHARMA P & ALLISON J P: "Immune Checkpoint Targeting in Cancer Therapy: Toward Combination Strategies with Curative Potential", CELL, vol. 161, no. 2, 9 April 2015 (2015-04-09), pages 205 - 214, XP055427087, ISSN: 0092-8674, DOI: 10.1016/j.cell.2015.03.030**
- **TARHINI A A ET AL: "Safety and Efficacy of Combination Immunotherapy With Interferon Alfa-2b and Tremelimumab in Patients With Stage IV Melanoma", JOURNAL OF CLINICAL ONCOLOGY, vol. 30, no. 3, 20 January 2012 (2012-01-20), pages 322 - 328, XP055492880, ISSN: 0732-183X, DOI: 10.1200/JCO.2011.37.5394**


(52) Cooperative Patent Classification (CPC): (Cont.)
**A61P 35/02; C07K 16/2818; C12N 5/0645;**
A01K 2207/12; A01K 2217/05; A01K 2227/105; A01K 2267/0331; A61K 35/28; A61K 48/0058; A61K 2039/545; A61K 2039/55522; A61K 2239/31; A61K 2239/38; A61K 2239/48; A61K 2300/00; C07K 14/7051; C07K 2317/76; C07K 2319/03; C12N 2510/02; C12N 2740/16043; C12N 2840/007

C-Sets
**A61K 31/405, A61K 2300/00;**
**A61K 39/3955, A61K 2300/00**

## Description

### FIELD OF INVENTION

The present invention relates to the use of cytokine gene therapy, in particular interferon (e.g. interferon-alpha or -gamma) gene therapy, in combination with a tumor associated antigen (TAA)-specific T-cell for treating or preventing cancer.

### BACKGROUND OF THE INVENTION

Immunotherapy has recently gained renewed interest as anticancer treatment, due to its potential to induce long-lasting remission. An increased understanding of the mechanisms co-opted by cancer cells to evade immune response has led to the development of novel therapeutics targeting immune checkpoints, thereby unleashing the power of the immune system[1]. Clinical testing of these drugs has led to unprecedented rates of durable responses in tumors previously considered invariably lethal, such as metastatic melanoma[2,3]. However, despite these advances, a large fraction of patients do not respond to these therapies, either due to the failure to generate tumor-specific T cells or the existence of an immunosuppressive tumor microenvironment, which imparts resistance to blockade of the classical negative checkpoints, CTLA4 or PD1/PDL14.

Current efforts are thus aiming at identifying new immune checkpoint targets and combination therapies, which might extend the benefits of immunotherapy to a larger number of tumor patients. In this regard, there is renewed interest in the use of cytokines, such as interferons (IFNs) as anti-cancer agents[5]. In addition to the cytostatic and anti-angiogenic effects on tumor cells and blood vessels, IFNs, in particular type I interferons, increase the maturation and cross-priming capacity of dendritic cells (DCs), the proliferation and cytotoxicity of T cells, the killing capacity of natural killer (NK) cells, and immunoglobulin class switch of B cells[6,7]. We previously reported proof-of-principle that a cell and gene therapy strategy selectively expressing an interferon-alpha (IFN$\alpha$) transgene in the tumor infiltrating monocyte/macrophage progeny of transplanted, genetically engineered hematopoietic stem cells (HSC) can boost anti-tumor responses. This tumor-targeted IFN$\alpha$ delivery strategy showed no systemic toxicity in the mice and inhibited the growth of spontaneous mammary tumors as well as lung and liver metastases of breast and colorectal cancer cells, respectively[8-10]. EP 2 856 876 A1 teaches to combine tumour-antigen specific T cells with various cytokines, including interferons, GM-CSF or erythropoietin.

Another immunotherapy approach is adoptive transfer of genetically engineered T cells expressing a transgenic T cell receptor (TCR) or a chimeric antigen receptor (CAR) directed against a tumor-associated antigen (TAA)[11,12]. This approach is particularly suitable for malignancies with low mutation burden that fail to induce endogenous T cell responses against TAAs. CAR T cells recognizing the CD19 antigen have demonstrated remarkable efficacy in relapsed and refractory B cell malignancies. However, these studies also suggested that the therapeutic effect was less evident in nodal disease with respect to bone marrow (BM) disease or leukemia, suggesting that an immunosuppressive microenvironment represents a major impediment towards successful immunotherapy, especially in solid tumor masses. Moreover, in fast-growing tumors such as B cell acute lymphoblastic leukemia (B-ALL), antigen loss occurs in up to 20% of patients treated with CD19 CAR T cells, illustrating a limitation of immunotherapy directed against a single antigen[11,13]. However, there is little data available on whether leukemia induces TAA-specific T cell responses outside of allogeneic transplant setting, and whether such a response can be potentiated for therapeutic purposes.

There remains a need for improved cancer therapies. The present invention achieves this need by establishing that improved efficacy can be achieved when a cytokine such as IFN$\alpha$ delivered by gene therapy is used in combination with other immunotherapeutic strategies.

### SUMMARY OF THE INVENTION

The inventors have surprisingly found that a cytokine such as an interferon delivered by gene therapy can boost the induction, consolidation and maintenance of anti-tumor responses and synergize with other immunotherapeutic strategies, in particular treatment with immune checkpoint inhibitors and treatment with tumor associated antigen (TAA)-specific T-cells.

According to one aspect of the present invention, there is provided a hematopoietic stem cell (HSC), a hematopoietic progenitor cell (HPC), a myeloid/monocyte-committed progenitor cell, a macrophage or a monocyte comprising a vector wherein the vector comprises at least one mir-130a and/or mir-126 target sequence operably linked to a nucleotide sequence encoding a cytokine for use in treating or preventing a cancer in a patient, wherein the HSC, the HPC, the myeloid/monocyte-committed progenitor cell, the macrophage or the monocyte is used in combination with a tumor associated antigen (TAA)-specific T-cell.

In some embodiments, the HSC, the HPC, the myeloid/monocyte-committed progenitor cell, the macrophage

or the monocyte is used further in combination with an immune checkpoint inhibitor.

According to another aspect of the present invention there is provided a TAA-specific T-cell for use in treating or preventing a cancer in a patient wherein the patient has previously been administered with a HSC, a HPC, a myeloid/monocyte-committed progenitor cell, a macrophage or a monocyte comprising a vector wherein the vector comprises at least one mir-130a and/or mir-126 target sequence operably linked to a nucleotide sequence encoding a cytokine. In some embodiments, the TAA-specific T-cell expresses a CAR and/or a transgenic TCR.

According to another aspect of the present invention, there is provided a HSC, a HPC, a myeloid/monocyte-committed progenitor cell, a macrophage or a monocyte comprising a vector wherein the vector comprises at least one mir-130a and/or mir-126 target sequence operably linked to a nucleotide sequence encoding a type 1 interferon for use in treating or preventing a cancer in a patient, wherein the HSC, the HPC, the myeloid/monocyte-committed progenitor cell, the macrophage or the monocyte is used in combination with a tumor associated antigen (TAA)-specific T-cell.

In some embodiments, the HSC, the HPC, the myeloid/monocyte-committed progenitor cell, the macrophage or the monocyte is used further in combination with an immune checkpoint inhibitor.

According to another aspect of the present invention there is provided a TAA-specific T-cell for use in treating or preventing a cancer in a patient wherein the patient has previously been administered with a HSC, a HPC, a myeloid/monocyte-committed progenitor cell, a macrophage or a monocyte comprising a vector wherein the vector comprises at least one mir-130a and/or mir-126 target sequence operably linked to a nucleotide sequence encoding a type 1 interferon. In some embodiments, the TAA-specific T-cell expresses a CAR and/or a transgenic TCR.

In one embodiment, the TAA-specific T-cell expresses a chimeric antigen receptor (CAR) and/or a transgenic T-cell receptor (TCR). In one embodiment, the TAA-specific T-cell expresses a CAR. In one embodiment, the TAA-specific T-cell expresses a transgenic TCR.

Thus, the present invention may use a T-cell engineered to express a TAA-specific CAR or TCR.

In one embodiment, the cytokine is an interferon (IFN), IL-12 or granulocyte-macrophage colony-stimulating factor (GM-CSF).

In one embodiment, the cytokine is an interferon (IFN). Preferably, the cytokine is a type I interferon or a type II interferon.

In a preferred embodiment, the cytokine is IFN$\alpha$.

In another preferred embodiment, the cytokine is interferon-gamma (IFN$\gamma$).

In one embodiment, the type 1 interferon is interferon-alpha (IFN$\alpha$) or interferon-beta (IFN$\beta$).

In one embodiment, the type 1 interferon is IFN$\alpha$.

In one embodiment, the type 1 interferon is IFN$\beta$.

In one embodiment, the type II interferon is interferon-gamma (IFN$\gamma$).

In one embodiment, the HSC, the HPC, the myeloid/monocyte-committed progenitor cell, the macrophage, or the monocyte of the present invention is provided for use in preventing the recurrence of the cancer in the patient.

In one embodiment, the HSC, the HPC, the myeloid/monocyte-committed progenitor cell, the macrophage, or the monocyte of the present invention is provided for use in preventing the progression of the cancer in the patient.

In one embodiment, the TAA is selected from any one or more of carcinoembryonic antigen (CEA), estrogen receptor, progesterone receptor, ephrinB2, ROR1, mesothelin, c-Met, GD-2, and MAGE A3 TCR, 4-1BB, adenocarcinoma antigen, alpha-fetoprotein, BAFF, B-lymphoma cell, C242 antigen, carbonic anhydrase 9 (CA-IX), CCR4, CD152, CD200, CD22, CD19, CD22, CD123, CD221, CD23 (IgE receptor), CD28, CD4, CD40, CD44, CD44 v6, CD51, CD52, CD56, CD74, CD80, CS-1, CNT0888, CTLA-4, DR5, EpCAM, CD3, fibronectin extra domain-B, folate receptor 1, glycoprotein 75, GPNMB, HGF, human scatter factor receptor kinase, IGF-1 receptor, IGF-I, IgGI, L1-CAM, IL-13, IL-6, insulin-like growth factor I receptor, integrin $\alpha 5\beta 1$, integrin $\alpha v\beta 3$, MORAb-009, MS4A1, mucin CanAg, N-glycolylneuraminic acid, NPC-1C, PDGF-R$\alpha$, PDL192, phosphatidylserine, prostatic carcinoma cells, RANKL, RON, SCH 900105, SDC1, SLAMF7, tenascin C, TGF beta 2, TGF-$\beta$, TRAIL-R1, TRAIL-R2, tumor antigen CTAA16.88, Vascular endothelial growth, factor (VEGF), VEGF-A, VEGFR-1, VEGFR2, vimentin, 5T4, CD5, CD19, CD20, CD21, CD25, CD37, CD30, CD33, CD45, CAMPATH-1 (CDw52), HLA-DR, Anti-idiotype, TAG-72, Ep-CAM, MUC1, Folate-binding protein, A33, G250, Prostate-specific membrane antigen, (PSMA), Prostate specific antigen (PSA), Ferritin, Gangliosides (e.g. GD2, GD3, GM2), $Le^y$, CA-125, CA19-9, Epidermal growth factor receptor (EGFR), p185HER2, IL-2 receptor, de2-7 EGFR, Fibroblast activation protein (FAP), Tenascin, metalloproteinases, Endosialin, Carbonic anhydrase, Galectin 9, Aldolase A, elF$\gamma$4, Tyrosinase, Galectin 4, HERKV-K10, p53, NY-LU-12, Restin, NY-CO-38, MAGE-1, MAGE-4a, SSX2, NY-ESO-1, SCP-1, 707-AP, AFP, ART-4, BAGE, b-catenin/m, Bcr-abl, CAMEL, CAP-1, CASP-8, CDC27m, CDK4/m, CT, Cyp-B, DAM-6 (MAGE-B2) and DAM-10 (MAGE-B1), ELF2M, ETV6-AML1, G250, GAGE, GnT-V, Gp100, HAGE, HER-2/neu, HLA-A*0201-R170I, HPV-E7, HSP70-2M, HST-2, hTERT (hTRT), iCE, KIAA0205, LAGE, LDLR/FUT, MAGE, MART-1/Melan-A, MC1R, Myosin/m, MUC1, MUM-1, MUM-2, MUM-3, NA88-A, NY-ESO-1, P15, p190 minor bcr-abl, Pml/RARa, PRAME, RAGE, RU1, RU2, SAGE, SART-1, SART-3, TEL/AML1, TPI/m, TRP-1, protein 1, gp75, TRP-2, TRP-2/INT2 or WT1.

In one embodiment, the TAA is CD19.

In a preferred embodiment, the TAA-specific T-cell expresses a CD19-specific CAR.

In one embodiment, the TAA-specific T-cell is derived from a cell isolated from the patient.

In one embodiment, the TAA-specific T-cell has been engineered to disrupt at least one endogenous gene, preferably wherein the at least one endogenous gene is selected from an endogenous gene encoding a TCR $\alpha$ chain, a TCR $\beta$ chain and a major histocompatibility complex (MHC).

In one embodiment the immune checkpoint inhibitor is an antibody, preferably wherein the immune checkpoint inhibitor antibody is selected from the group consisting of an anti-CTLA4 antibody, an anti-PD1 antibody, an anti-PDL1 antibody, an anti-PDL2 antibody and an anti-LAG-3 antibody.

In one embodiment the vector comprises at least one mir-130a target sequence and at least one mir-126 target sequence, preferably the vector comprises two mir-130a target sequences and two mir-126 target sequences.

In one embodiment the HSC, the HPC, the myeloid/monocyte-committed progenitor cell, the macrophage or the monocyte used in the present invention comprises at least one mir-130a target sequence and at least one mir-126 target sequence, preferably the HSC, the HPC, the myeloid/monocyte-committed progenitor cell, the macrophage or the monocyte used in the present invention comprises two mir-130a target sequences and two mir-126 target sequences.

The vector may further comprise a tissue specific promoter operably linked to the nucleotide sequence encoding the cytokine, for example the type 1 interferon. A preferred tissue specific promoter is the TEK (Tie2) promoter.

In one embodiment the cancer is a hematological malignancy or a solid tumor, preferably wherein the hematological malignancy is selected from the group consisting of acute myeloid leukemia (AML), lymphoblastic leukemia, acute lymphoblastic leukemia (ALL), myelodysplastic syndromes (MDS), myeloproliferative neoplasms (MPN), primary myelofibrosis, essential thrombocythemia, polycythemia vera, atypical chronic myeloid leukemia, chronic myeloid leukemia (CML), lymphoma, multiple myeloma, non Hodgkin lymphoma, and Hodgkin lymphoma; or preferably wherein the solid tumor is selected from the group consisting of lung cancer, breast cancer, oesophageal cancer, gastric cancer, colon cancer, cholangiocarcinoma, pancreatic cancer, ovarian cancer, head and neck cancers, synovial sarcoma, angiosarcoma, osteosarcoma, thyroid cancer, endometrial cancer, neuroblastoma, rabdomyosarcoma, liver cancer, melanoma, prostate cancer, renal cancer, soft tissue sarcoma, urothelial cancer, biliary cancer, glioblastoma, cervical cancer and colorectal cancer.

In one embodiment, the cancer is a metastasis from a primary tumor.

In one embodiment, the cell used in the present invention is a HSC comprising a vector or a polynucleotide, wherein the vector or the polynucleotide comprises at least one mir-130a and/or mir-126 target sequence operably linked to a nucleotide sequence encoding a cytokine, for example a type 1 interferon.

In one embodiment, the cell used in the present invention is a HPC comprising a vector or a polynucleotide, wherein the vector or the polynucleotide comprises at least one mir-130a and/or mir-126 target sequence operably linked to a nucleotide sequence encoding a cytokine, for example a type 1 interferon.

In one embodiment, the cell used in the present invention is a myeloid/monocyte-committed progenitor cell comprising a vector or a polynucleotide, wherein the vector or the polynucleotide comprises at least one mir-130a and/or mir-126 target sequence operably linked to a nucleotide sequence encoding a cytokine, for example a type 1 interferon.

In one embodiment, the cell used in the present invention is a macrophage comprising a vector or a polynucleotide, wherein the vector or the polynucleotide comprises at least one mir-130a and/or mir-126 target sequence operably linked to a nucleotide sequence encoding a cytokine, for example a type 1 interferon.

In one embodiment, the cell used in the present invention is a monocyte comprising a vector or a polynucleotide, wherein the vector or the polynucleotide comprises at least one mir-130a and/or mir-126 target sequence operably linked to a nucleotide sequence encoding a cytokine, for example a type 1 interferon.

In one embodiment, the IFN is a type II IFN (preferably IFN$\gamma$) or a type I IFN (preferably IFN$\alpha$ or IFN$\beta$)

In one embodiment, the cytokine is IFN$\gamma$. In one embodiment, the cytokine is IFN$\alpha$. In one embodiment, the cytokine is IFN$\beta$.

## DESCRIPTION OF THE DRAWINGS

**Figure 1. Inhibition of OVA-ALL growth in IFN mice by induction of OVA-specific CD8+ T cells.**
**a,** Schematic representation of the lentiviral vectors (LV) used to engineer HSC and generate spontaneous B-ALL. b, Experimental design. **c**, Absolute numbers (mean ± SEM) of parental ALL in the peripheral blood (PB) over time of CTRL (n=10, treated with isotype control antibody), IFN ((n=10, treated with isotype control antibody), CTRL + aCTLA4 (n=14) and IFN + aCTLA4 (n=13) mice. Each dot represents a single mouse. *$p<0.05$, **$p<0.01$, ****$p<0.0001$, nonparametric rank-based method for longitudinal data in factorial experiments. **d**, **e, f** Absolute numbers (mean ± SEM) of OVA-ALL (engineered with the NGFR/OVA bidirectional LV shown on top) in the PB over time (d), and in BM and spleen (e, **f**; 14 days after tumor injection) of IFN (n=15) and CTRL (n=15) mice (one

out of 9 experiments shown). Each dot represents a single mouse. ****p<0.0001, nonparametric rank-based method for longitudinal data in factorial experiments (**d**); *p<0.05, ** p<0.01, Mann-Whitney (**e**, **f**). **g**, Splenic CD8+ T cells from IFN mice, 13 days upon tumor injection, or from non tumor-bearing CTRL (naive C57Bl/6, n=1) mice tested by IFNg-ELISPOT against the EL4 target cell line transduced with NGFR-OVA or NGFR-CD20 BdLV. Each dot represents a single mouse, mean ± SEM. Low tumor burden (n=5): 5.2 ± 4.9 (mean ± SEM) % OVA-ALL; mid tumor burden (n=1): 33% OVA-ALL; high tumor burden (n=6): 51 ± 4.9 %OVA-ALL. **h,** Percentage (mean ± SEM) of OVA-specific T cells in the PB of IFN (n=14-6) and CTRL (n=15-8) mice. **p<0.01, Mann Whitney performed at 9 days upon OVA-ALL injection. Each dot represents a single mouse. T cells from transgenic OT-I mice are used as positive control (not shown). **i**, **j**, Absolute numbers (mean ± SEM) of OVA-specific T cells in the BM (**i**) and spleen (**j**), 9 days upon tumor injection, in IFN (n= 23) and CTRL (n=26) mice from 3 independent experiments. **p<0.01, ***p< 0.001, Mann-Whitney. Each dot represents a single mouse. **k**, Splenic CD8+ T cells from IFN (n=7) and CTRL (n=8) mice 9 days upon tumor injection or from non tumor bearing mice tested as in (**g**). Concanavalin A (ConA) stimulation and T cells from OT-I mice (not shown) are used as positive controls. Each dot represents a single mouse. **l, m,** Percentage (**l**) and absolute numbers (**m**) (mean ± SEM) of OVA-ALL in the PB of IFN (n=9), CD8-depleted IFN (n=7) and CTRL (n=9) mice. **p<0.01, ****p<0.0001, nonparametric rank-based method for longitudinal data in factorial experiments (**l**); *p<0.05, ****p<0.0001, Kruskall-Wallis with adjusted p-value by Dunn's test (m).

**Figure 2. Adoptively transferred OT-I T cells expand and contain OVA-ALL in IFN mice.**
**a, b,** Experimental design (**a**) and OVA-ALL growth over time (b, percentage in PB; mean ± SEM) in IFN (n=7) or CTRL (n=7) mice. **c**, Absolute numbers (mean ± SEM) of OTI T cells in BM and spleen of IFN (n= 7), CTRL (n=7) and non tumor-bearing (C57Bl/6 no tumor, n=3) mice 3 days upon OT-I adoptive transfer. *p<0.05, **p<0.01, Mann-Whitney. Each dot represents a single mouse. **d**, Percentages (mean ± SEM) of naive (CD44-CD62L+), central memory (CD44+CD62L+) and effector memory (CD44+CD62L-) cells within OT-I T cells in the BM and spleen of mice from (c) 3 days upon adoptive transfer. **p<0.01, nonparametric combination test. **e**, **f**, Experimental design (e) and survival curve (**f**) of OVA-ALL-injected CTRL (n=12), IFN + OT-I (n=9) and CTRL + OT-I (n=10) mice. *p<0.05, ****p<0.0001 Mantel-Haenszel test, adjusted p-value by Bonferroni test. **g**, Absolute numbers (mean ± SEM) of adoptively transferred OT-I T cells in the PB over time of CTRL + OT-I and IFN + OT-I mice from (f), and non tumor-bearing C57Bl/6 mice (n=3). Long-term surviving mice are shown in green. Each line represents a single mouse. **h**, Percentage (mean ± SEM) of Lag3 expression on OT-I T cells in the PB of mice from (**f**, **g**). Each line represents a single mouse.

**Figure 3. ISG/Th1 primed gene signature and increased classical monocytes and M1-skewing in IFN mice.**
**a,** Volcano plot showing changes in the expression of a panel of cancer immune genes in the BM and spleen of IFN and CTRL mice before and after OVA-ALL challenge. Horizontal dashed lines indicate threshold of significance (FDR<0.05). Vertical dashed lines indicate genes with 2-fold increased/decreased expression with respect to CTRL mice. ISGs are shown in orange, Th1 and T cell activation genes in light blue, genes common to IFN and Th1 responses in green, macrophage and DC activation genes in red, NK cell activation genes in purple, antigen processing genes in blue, leukemia associated genes in grey. Gated insets shown magnified on the right. n=3 mice for each sample, except for the CTRL BM + ALL, n=2. **b**, Expression analysis by RT-qPCR of the indicated Th1 genes (mean fold-change over OVA-ALL-injected CTRL mice ± SEM) in splenic CD4+ T cells from OVA-ALL-injected IFN (n=6) and CTRL (n=5) mice 9 days upon tumor injection and from a non tumor-bearing C57Bl/6 mouse. Each dot represents a single mouse. *p<0.05, Mann-Whitney. **c**, Percentage (mean ± SEM) of the indicated myeloid populations in the PB of IFN (n=11) and CTRL (n=13) mice before and after (12 days) OVA-ALL injection. *p<0.05, **p<0.01, ***p<0.001, ****p<0.0001, Kruskall-Wallis, with adjusted p value by Dunn's test. Statistical analysis is performed within each individual myeloid cell population.

**Figure 4. IFN cell and gene therapy induces durable anti-cancer responses and when combined with immune checkpoint therapy further improves survival.**
**a, b, c, d** Survival curves of OVA-ALL-injected IFN and CTRL mice after the first (a; n=11,13; *p<0.05, Mantel-Haenszel test) and subsequent tumor challenges with the indicated ALL cells (**b**: n=3 long-term surviving IFN mice from (**a**) vs. 4 naive mice; **c**: same 3 surviving mice from (**a**, **b**) vs. 4 naive mice; **d**: 2 of the 3 surviving mice from (**a, b, c**) vs. 4 naive mice. **e**, Experimental design. **f, g,** Survival curves of OVA-ALL-injected (f) IFN (n=14), CTRL (n=14), IFN +aCTLA4 (n=14) and CTRL + aCTLA4 (n=15) mice, *p<0.05, ***p<0.001, Mantel-Haenszel test, adjusted p-value by Bonferroni method; and (**g**) IFN + aCTLA4 (n=10) and CTRL + aCTLA4 (n=12) mice, *p<0.05, Mantel-Haenszel test. **h,** PBMC from surviving mice from (**f**) (IFN n=3, CTRL n=1, IFN + aCTLA4 n=4, CTRL + aCTLA4 n=3) 51 days upon tumor injection and from non-tumor bearing (OT-I, n=1 and transplanted CTRL mice, n=2) mice tested by IFNg-ELISPOT against the EL4 target cell line transduced with NGFR-OVA or NGFR-CD20 BdLV or PGK-OFP or PGK-tTA LV. The dashed line indicates the higher median background level seen against the EL4 NGFR target cells. Each dot represents a single mouse tested against all four TAAs. **i,** Clonality and similarity of the TCR-beta CDR repertoire of surviving mice (each arrow represents a mouse) from (f) before OVA-ALL injection (start of the arrow, T0) and 4 days after second tumor re-challenge (tip of the arrow, T2). **j,** PBMC from mice from (g) (IFN + αCTLA-4 survivors, full blue symbols, n=3; CTRL + αCTLA-4 survivors, full red symbols, n=1; IFN + αCTLA-4 non responders, empty blue symbols, n=7; CTRL + αCTLA-4, empty red symbol, n=8 out of 12 non-responder controls) tested by IFNg-ELISPOT as in (**h**), 19 days upon tumor injection. Each dot represents a single mouse tested against all four TAAs. **k,** Survival curve of mice from (**g**) stratified based on their immune reactivity as assessed by IFNg-ELISPOT assay shown in (**j**). Mice shown in red react against 2 or more antigens; mice shown in black react against 1 or no antigens. Note that reactivity against OVA and NGFR count for 1 as their expression is co-regulated by a bidirectional promoter present within a BdLV.

**Figure 5. Engineered OVA-ALL showed increased immunogenicity as compared to parental ALL.**
**a,** Schematic representation of the bidirectional LV (BdLV) used to generate OVA-ALL with representative plot showing NGFR expression on purified OVA-ALL. a, Gating strategy used to identify B-ALL (CD19+OFP+) and NGFR expression on ALL in PB of tumor-injected mice. **c,** Top: Percentage (mean ± SEM) of OVA-ALL in the PB of immune-competent C57Bl/6 (n=5) and immune-deficient NSG (n=6) mice and of parental ALL in the PB of immune-competent C57Bl/6 (n=5) mice. Bottom: expression of the NGFR marker on ALL present in the PB of immune-competent C57Bl/6 (n=5) and immunedeficient NSG (n=6) mice. Note that expression of NGFR and OVA antigens is co-regulated by a bidirectional promoter present within a BdLV. **d, e,** Percentage (mean ± SEM) of OVA-ALL and expression of NGFR marker on leukemic cells present in the BM of immune competent C57Bl/6 (n=6) (d) and immune-deficient NSG (n=6) mice (e) at the time of sacrifice.

**Figure 6. Inhibition of OVA-ALL growth in IFN mice and induction of OVA-specific T cells.**
**a**, Gating strategy used to identify OVA-specific CD8+ T cells. **b, c, d,** Percentage (**b**) and absolute numbers (**c**, **d**) (mean ± SEM) of OVA-ALL in PB (b) and in BM and spleen (**c**, **d**) of IFN (n=14-6) and CTRL (n=15-8) mice, 9 days upon tumor injection. ***P<0.001, Mann-Whitney. Each dot represents a single mouse. **e**, Percentage of OVA-ALL in PB of IFN (n=9), CD8-depleted IFN (n=7) and CTRL (n=9) mice 9 days upon tumor challenge. Each dot represents a single mouse. *p<0.05, ***p<0.001, Kruskall-Wallis with adjusted p-value by Dunn's test.

**Figure 7. Gating strategy used to assess OVA-ALL apoptosis, cell cycle and proliferation rate.**
**a**, **b**, Gating strategy (**a**) and analysis (**b**) (percentage, mean ± SEM) of early apoptotic (Annexin+7-AAD-), late apoptotic (Annexin+7-ADD+), dead/necrotic (Annexin-7AAD+) and alive (Annexin-7-AAD-) OVA-ALL cells present in BM and spleen of IFN (n=11) and CTRL (n=15) mice, 9 days upon tumor injection. *p<0.05, Mann-Whitney. **c**, **d**, Gating strategy (c) and analysis (**d**: percentage, mean ± SEM) of OVAALL distribution in the G0 (Ki67-Hoechst$^{low}$), G1 (Ki67+Hoechst$^{low}$) and S-G2-M (Ki67+Hoechsthigh) phase of the cell cycle in BM and spleen of IFN (n=11) and CTRL (n=15) mice, 9 days upon tumor injection. **e**, **f**, Gating strategy (e) and analysis (f: percentage, mean ± SEM) of proliferating EdU+ ALL cells present in BM and spleen of IFN (n=11) and CTRL (n=15) mice, 9 days upon tumor injection. **p<0.01, Mann-Whitney.

**Figure 8. Adoptive transfer of OT-I T cells in OVA-ALL-injected CTRL, IFN mice and non-tumor bearing C57Bl/6 mice.**
**a**, Absolute numbers (mean ± SEM) of OVA-ALL in the PB of CTRL (n=7) and IFN (n=7) mice at the indicated time upon OT-I adoptive transfer. Each dot represents a single mouse. **b**, Lag3 expression on OT-I T cells in BM and spleen of IFN (n= 7), CTRL (n=7) and non tumor-bearing (C57Bl/6 no tumor, n=3) mice, 3 days upon adoptive transfer of OT-I T cells. Each dot represents a single mouse. **c**, Gating strategy used to identify adoptively transferred OT-I T cells (CD45.2+) and Lag3 expression on OT-I T cells present in BM and spleen of mice from (a, b). OVA-ALL cells are first excluded by gating on OFP-CD8+ T cells. CD45.1 and CD45.2 markers are used to distinguish among radio-resistant recipient-derived CD8+ T cells (CD45.1+), donor-derived CD8+ T cells (CD45.1.2+) and adoptively transferred OT-I T cells (CD45.2+). In non tumorbearing mice, adoptively transferred OT-I T cells are defined as CD8+ T cells and are distinguished from CD8+ recipient-derived T cells (CD45.1+) by gating on CD45.2+ CD8+ T cells. **d**, Gating strategy used to identify naive (CD62L+CD44-), central memory (CD62L+CD44+) and effector memory (CD62L-CD44+) adoptively transferred OT-I T cells. OT-I T cells are identified as described in (c, OVA-ALL are first excluded by gating on CD19-CD8+ T cells). **e**, **f**, Absolute numbers of OVA-ALL (e) (mean ± SEM) and NGFR marker expression (**f**) on ALL in BM and spleen from mice shown in (**a, b**) at 3 days upon adoptive transfer of OT-I T cells. Each dot represents a single mouse. **p<0.01, ***p<0.001, Mann- Whitney (e).

**Supplementary Figure 6. Gene expression analysis of prototypical Th2 and Treg genes in purified splenic**

**CD4+ T cells.**

**a**, Expression analysis by RT-qPCR of the indicated Th2 and Treg genes (mean fold-change over OVA-ALL-injected CTRL mice ± SEM) in splenic CD4+ T cells purified from OVA-ALL-injected IFN (n=6) and CTRL (n=5) mice, 9 days upon tumor injection and from a non tumor-bearing naive C57Bl/6 mouse. Each dot represents a single mouse.

**Figure 9. Adoptively transferred OT-I T cells expand and contain OVA-ALL in IFN mice.**

**a**, **b**, **c**, **d**, Percentage (mean ± SEM) of ALL cells and expression of NGFR marker on ALL cells in the PB (**a**, **b**) and BM (**c**, **d**) of IFN + OT-I (n=9, **a**, **c**) and CTRL + OT-I (n=10, **b**, **d**) mice. Long-term surviving mice are shown in green. Note that NGFR expression is not shown for surviving mice that eradicate leukemia. Each individual mouse is represented as single dot (**b**, **d**) or line (**a**, **b**). **e**, **f**, Percentage (mean ± SEM) of naive (CD62L+CD44-), central memory (CD62L+CD44+) and effector memory (CD62LCD44+) OT-I T cells in the BM, spleen and lymph nodes of euthanized tumor-bearing mice from (**a**, **b**, **c**, **d**, CTRL + OT-I, n=8; IFN + OT-I, n=3). **f**, Percentage (mean ± SEM) of PD1 expression on OT-I T cells present in the BM, spleen and lymph nodes of mice shown in (**e**). Each dot represents a single mouse.

**Figure 11. Flow cytometric analysis of myeloid cells in the PB and spleen of tumor-bearing and non tumor-injected IFN and CTRL mice.**

**a, b**, **c**, Gating strategy (a) and analysis (b, absolute numbers, mean ± SEM) of myeloid cell populations in the spleen of tumor-free and OVA-ALL-injected IFN and CTRL mice (CTRL: n=3, IFN n=2, CTRL + ALL: n=9, IFN + ALL: n=7), 10 days upon tumor injection. Macrophages (MF) are identified as CD19-CD11c-Ly6G-F4/80+ cells and further distinguished into MHC-II+ MF and MHC-II- Mf using the IAb (MHC-II) marker. We distinguished CD19-F4/80-CD11c+MHCII- myeloid cells from the CD19-F4/80-CD11c+MHCII+ dendritic cells. We defined granulocytes as CD19-F4/80-CD11c-CD11b+Ly6C+Ly6G+, classical monocytes as CD19-F4/80-CD11c-CD11b+Ly6ClowLy6G- and non classical monocytes as CD19-F4/80-CD11c-CD11b+Ly6C-Ly6G-. **p<0.01, Kruskall-Wallis with adjusted p-value by Dunn's test. Statistics is performed within each individual myeloid population. **d,** Percentage of MHC-II macrophages present in tumor-free or tumor-bearing IFN and CTRL mice from (c). **p<0.01, Kruskall-Wallis with adjusted p-value by Dunn's test.

**Figure 12. Flow cytometric analysis of myeloid cells in the BM and T regulatory cells in the BM and spleen of tumor-bearing and non tumor-injected IFN and CTRL mice.**

**a**, **b**, Gating strategy and analysis (absolute numbers, mean ± SEM) of myeloid cell populations in the BM of tumor-free and OVA-ALL-injected IFN and CTRL mice (CTRL: n=3, IFN: n=2, CTRL + ALL: n=9, IFN + ALL: n=7), 10 days upon tumor injection. Each dot represents a single mouse. Macrophages (MF) are identified as CD19-CD11c-Ly6G-F4/80+ cells, dendritic cells (DCs) as CD19-F4/80-CD11c+MHCII+, granulocytes as CD19-F4/80-CD11c-CD11b+Ly6C+Ly6G+, classical monocytes as CD19-F4/80-CD11c-CD11b+Ly6ClowLy6G- and non classical monocytes as CD19-F4/80-CD11c-CD11b+Ly6CLy6G-.*p<0.05, **p<0.01, Kruskall-Wallis with adjusted p-value by Dunn's test. Statistics is performed within each individual myeloid population. **c**, **d**, Gating strategy (**c**) and analysis (**d**, absolute numbers, mean ± SEM) of T regulatory cells in BM and spleen of tumor-free and OVA-ALL-injected IFN and CTRL mice from (b). Each dot represents a single mouse. T regulatory cells are defined as CD3+CD4+CD25+FoxP3+. **p<0.01, Kruskall-Wallis with adjusted p-value by Dunn's test. Statistics is performed within each individual tissue.

**Figure 13. Flow cytometric analysis of NK and NKT cells in the spleen of tumor-bearing and non tumor-injected IFN and CTRL mice.**

**a**, **b**, Gating strategy (a) and analysis (b, absolute numbers, mean ± SEM) of NK and NKT cells present in spleen and BM of tumor-free or OVA-ALL-injected IFN and CTRL mice (CTRL: n=3, IFN: n=2, CTRL + ALL: n=9, IFN + ALL: n=7), 10 days upon tumor injection. Each dot represents a single mouse. NK cells are identified as CD19-CD3-NK1.1+ and NKT cells as CD19-CD3+NK1.1+. Using the CD11b and CD27 markers we further distinguished among the most immature (CD11b-CD27+), the mature and most cytotoxic and cytokine producers (CD11b+CD27+) and the mature and less cytotoxic and cytokine producers (CD11b+CD27-) NK cells. **p<0.01, ***p<0.001, Kruskall-Wallis with adjusted p-value by Dunn's test. Statistics is performed within each individual population. **c**, Percentage (mean ± SEM) of the indicated sub-populations of NK cells in the spleen (top) and BM (bottom) of tumor-free or tumorbearing IFN and CTRL mice from (**b**). Each dot represents a single mouse. **d**, **e,** Percentage (mean ± SEM) of NKG2D and NKp46 expression on CD11b+CD27+ (**d**) and CD11b+CD27-(**d**, **e**) NK cells present in the spleen (d) and BM (e) of mice from (**b**). Each dot represents a single mouse. *p<0.05, **p>0.01, Kruskall-Wallis with adjusted p-value by Dunn's test. Statistics is performed within each individual population.

**Figure 14. Immune pressure exerted by OVA-specific T cells leads to immune-selection of OVA-negative**

**ALL cells in the mice.**

**a,** Percentage (mean ± SEM) of OVA-ALL in the PB of each individual IFN (n=11) and CTRL (n=13) mice. Each line represents a single mouse. **b,** NGFR expression on BM-infiltrating ALL (or PB circulating ALL for those mice for which BM analysis is not available) and absolute numbers (mean ± SEM) of OVA-specific T cells (each line represents a single mouse) in the PB of IFN (n=8) and CTRL (n=13) mice showing fast or delayed disease course (fast disease course: CTRL: 13-29 days (range), 20.4 ± 1.3 (mean ± SEM), n=11; IFN:16-27 days, 20.8 ± 1.8, n=5; delayed disease course: CTRL: 34-44 days, 39 ± 5, n=2; IFN: 34-100 days, 56 ± 22, n=3). **c**, absolute numbers (mean ± SEM) of OVA-specific T cells in the PB of long term survival IFN (n=3) mice. Each line represents a single mouse. **d**, Percentage (mean ± SEM) of OVA-specific T cells in surviving IFN mice (n=3) vs. 4 naive mice from Figure **4b**, 14 days upon second tumor challenge with OVA-ALL. Each dot represents a single mouse. **e**, Representative plot showing the OVA-ALL and parental ALL cells mixed at 1 to 1 ratio before injection in the mice.

**Figure 15. Leukemic growth kinetic in individual mice.**

**a, b,** Percentage (mean ± SEM) of OVA-ALL in the PB of each individual (a) IFN (n=14), CTRL (n=14), IFN + aCTLA4 (n=14) and CTRL + aCTLA4 (n=15) mice and (b) IFN + aCTLA4 (n=10) and CTRL + aCTLA4 (n=12) mice. Each line represents a single mouse.

**Figure 16. Immune-selection of OVA-negative ALL cells is enhanced in both CTLA4-treated IFN and CTRL mice.**

**a,** NGFR expression on BM-infiltrating ALL (or PB circulating ALL for those mice for which BM analysis is not available) and absolute numbers (mean ± SEM) of OVA-specific T cells (each line represents a single mouse) in the PB of IFN (n=14), CTRL (n=14), IFN+aCTLA4 (n=14) and CTRL+aCTLA4 (n=15) mice showing fast, delayed disease course or long-term survival. Fast disease course: CTRL: 14-21 days (range), 15.66 ± 0.8 (mean ± SEM), n=12; IFN: 17-25 days, 21 ± 1.3, n=8; CTRL + CTLA4: 14-21 days, 19 ± 1.2, n=6; IFN + CTLA4: 25 days, 25 ± 0, n=2; delayed disease course: CTRL: 36 days, n=1; IFN: 30-36 days, 34 ± 2, n=3; CTRL + CTLA4, 30-64 days, 42 ± 7.4, n=5; IFN + CTLA4: 25-64 days, 44 ± 4.9, n=7). **b**, Percentage of OVA-specific T cells within CD8+ T cells in the PB of mice from (**a**). *p<0.05, **p<0.01, ****p<0.0001, nonparametric rank-based method for longitudinal data in factorial experiments. **c**, Clonality and similarity of the TCR-beta CDR repertoire of tumor-free long-term surviving mice (each arrow represents a mouse) from Figure **4f** before OVA-ALL injection (start of the arrow, T0) and at 30 days upon OVA-ALL challenge (tip of the arrow, T1).

**Figure 17. IFN gene therapy inhibits ALL growth by activating adaptive immunity.**

Absolute numbers (mean ± SEM) of parental ALL in the peripheral blood (PB) over time of CTRL (n=10, treated with isotype control antibody), IFN (n=10, treated with isotype control antibody), CTRL + αCTLA4 (n=14) and IFN + αCTLA4 (n=13) mice. Each dot represents a single mouse. *p<0.05, **p<0.01, ****p<0.0001, nonparametric rank-based method for longitudinal data in factorial experiments.

**Figure 18. IFN gene therapy imposes an immune-stimulatory program in the TME.**

Percentage of DCs presenting the immune-dominant OVA (SINFEKL) peptide on MHC-I molecules in the BM of mice from CTRL + ALL (n=7) and IFN + ALL (n=8). *p<0.05 Mann-Whitney test.

**Figure 19. Transcriptional reprogramming of the leukemia TME by IFN gene therapy.**

**a**,**b**, Volcano plots (left panels) showing differentially expressed genes (abs. $\log_2$FC>1.5, FDR<0.05) up-regulated (green) or down-regulated (orange) in splenic macrophages from tumor-bearing (ALL) vs. control (CTRL) mice (a) or ALL mice treated (IFN+ALL) or not (ALL) with IFN gene therapy (b). Bar plots (right panel) show GO terms enriched in up-regulated (green) or down-regulated (orange) genes from the indicated comparisons. **c**,**d**, Selected RNA-Seq snapshots of deregulated genes in macrophages from ALL mice (c) or ISGs induced in IFN+ALL mice (**d**). **e**, **f**, tSNE plots incorporating scRNA-Seq data from 10,821 CD11b$^+$ cells sorted from the spleen of CTRL or ALL mice, treated or not with IFN gene therapy. Transcriptionally defined clusters and associated cell types (e) are indicated by numbers and colours in the legend. Single-cell RNA-Seq data in cells from cluster 1 (non-classical monocytes), coloured based on the experimental condition (**f**). **g**, GO terms enriched in the top 100 genes (ranked by $\log_2$FC) up-regulated (green) or down-regulated (orange) in scRNA-Seq data from cluster 1 (non-classical monocytes) in the indicated comparisons. **h**, Mean expression (log transformed TPM values, normalized for number of cells) in non-classical monocytes of selected genes for the indicated conditions. **i**, Minimum spanning tree (MST) analysis of scRNA-Seq data from cluster 1 (non-classical monocytes), after sub-clustering. Each pie represents a sub-cluster and shows its size (number of cells) and composition (experimental condition). Pies marked by asterisks represent minor sub-clusters with dispersed composition.

**Figure 20. IFN gene therapy boosts activation of adoptively transferred CAR19-transduced T cells and enhances survival.**

**a**, **b**, Experimental design (a) and ALL growth over time (b, absolute numbers in PB; mean ± SEM) in CTRL + CTRLT (n=5), IFN + CTRLT (n=5), CTRL + CART19 (n=7), IFN + CART19 (n=7). ****p<0.0001, nonparametric rank-based method for longitudinal data in factorial experiments. **c**, Percentage (mean ± SEM) of Lag3 expression on CD8+NGFR+ CART19 cells in the PB of mice from (**b**). Long-term surviving mice are shown in green. Each line represents a single mouse. **d**, **e**, Experimental design (**d**) and ALL growth over time (e, absolute numbers in PB; mean ± SEM) in CTRL + CTRLT (n=7), IFN + CTRLT (n=6), CTRL + CART19 (n=7), IFN + CART19 (n=6), CTRL + iCART19 (n=7), IFN + iCART19 (n=6), IFN + CTRLT late (n=6, late intervention trial), IFN + iCART19 late (n=6, late intervention trial). ****p<0.0001, nonparametric rank-based method for longitudinal data in factorial experiments. Statistical analyses is performed on selected groups (see Supplementary Online Table 9 and 10) **f**, Percentage overtime (mean ± SEM) of Lag3 expression on CD8+NGFR+ CART19 cells in the PB of mice from (**b**). CTRL + CART19 and CTRL + iCART19 mice are plotted together. Long-term surviving mice are shown in green. Each line represents a single mouse. **g**, Survival curve of mice treated with CART19 or iCART19 cells from (b and e). CTRL mice treated with CART19 and iCART19 from Figure 20. b, e are plotted together (CTRL + i/CART19, n=21), IFN + CART19 (n=13), IFN + iCART19 (n=6), IFN + iCART19 late (n=6). ****p≤0.0001, Mantel-Haenszel test, adjusted p-value by Bonferroni method.

**Figure 21. Engineered OVA-ALL showed increased immunogenicity as compared to parental ALL.**

Survival curve of ALL-injected CTRL (n=10, treated with isotype control antibody), IFN (n=10, treated with isotype control antibody), CTRL + αCTLA4 (n=14) and IFN + αCTLA4 (n=13) mice. **p<0.01, Mantel-Haenszel test.

**Figure 22. Bulk RNA-Seq analyses in splenic macrophages.**

**a**, Box plots showing expression levels in the indicated conditions of genes up-regulated (n=213) or down-regulated (n=258) in macrophages from ALL mice treated with IFN gene therapy (IFN+ALL) as compared to ALL controls. Numbers represent results of Wilcoxon signed-rank test in the indicated comparisons. b, Heatmap of correlation between RNA-Seq datasets in macrophages from the indicated conditions. Numbers indicate calculated coefficients of determination ($R^2$). Samples are ordered based on unsupervised hierarchical clustering.

**Figure 23. Cell type identification in scRNA-Seq data from splenic $CD11b^+$ cells.**

**a**, tSNE plots showing single-cell expression levels of the indicated gene signatures (GS). Non-classical monocytes: Cd300e, 1700011I03Rik, Tspan9, Dmpk, Fxyd2; classical monocytes: Ly6c2, Tarm, Tfec, Psrc1, Mmp8; neutrophils: Il1f9, Pglyrp4, Nlrp12, Mrgpra2b, Mmp8, Ltf, Amer2, Stfa2l1, Gm5483; dendritic cells: Adam23, Procr, Mab21l3, Sucnr1, Fndc5, Rnf186, Flt3, Cd207, Adam11, Apol7c, Htr7; macrophages: Vcam1, Mertk, Actn1, Fcna, Crip2, Spic, Kcna2, Gfra2, Stab2, Jup; NK cells: Khdc1c, Khdc1b, Phactr3, Col8a2, Klra4, Adamts14, Gzma, Cma1, Gzmb, Clip4; T cells: Cd3g, Cd3e, Bcl11b, Actn2, Camk4; B cells: Pax5, Cd79a, Cd19, Chst3, Scn4a, Cacna1i, Ly6d, Klhl14, Mzb1. Colour scale reflects average expression (log transformed TPM) across genes within each signature. **b**, Heatmap showing expression (scaled log transformed TPM values) of top 20 discriminative genes for each cluster. Selected representative genes for each cluster are shown on the right. Up to 200 single cells are shown for each cluster.

**Figure 24. Leukemia and IFN-induced changes in scRNA-Seq data on splenic $CD11b^+$ cells.**

tSNE plots showing scRNA-Seq data from clusters 2-11, coloured based on the experimental condition.

**Figure 25. Sub-clustering analysis of scRNA-Seq data from non-classical monocytes and gene expression analyses on CD4 T cells.**

**a**, Flow cytometric plots showing OVA-ALL cells (top) and OVA-specific CD8 T cells (bottom) in the spleen from 3 representative ALL-injected CTRL (CTRL + ALL), IFN non responder (IFN + ALL NR) and IFN responder (IFN + ALL R) mice. CD11b+ cells from these mice were analysed by scRNA-seq. **b**, tSNE plots showing sub-cluster of cells within the non-classical monocyte population, coloured based on clustering (top plot) or experimental condition (bottom plot). Circled sub-clusters represent minor sub-clusters with dispersed composition and correspond to those marked by asterisks in Figure 19i.

**Figure 26. IFN gene therapy boosts activation of CART19 cells.**

**a**, Schematic representation of the bidirectional LV (BdLV) used to transduced mouse T cells and percentage of NGFR expression in un-transduced (UT) and transduced (CART19) CD4 and CD8 T cells prior to infusion in the mice from Figure 20b. **b**, Percentage of naive (CD62L+CD44+), central memory (CD62L+CD44+) and effector memory (CD62L-CD44+) UT or CART19 T cells prior to infusion in the mice from Figure 20b. c, Representative flow

cytofluorimetric plot of the IFN + CART19 surviving mouse from Figure 20b showing B cell aplasia in the PB. **d**, Percentage overtime (mean ± SEM) of PD1 expression on CD8+NGFR+ CART19 cells from CTRL + CART19 (n=7) and IFN + CART19 (n=7) mice. Long-term surviving mice are shown in green. Each line represents a single mouse. **e**, **f**, Level (mean fluorescent intensity, MFI) of NGFR expression overtime on CD4+ (**e**) and CD8+ (**f**) CART19 cells of mice from (**d**). Long-term surviving mice are shown in green. Each line represents a single mouse.

**Figure 27. IFN gene therapy boosts activation of iCART19 cells.**
**a**, Percentage of NGFR expression in un-transduced (UT) and transduced (CART19) CD4 and CD8 T cells prior to infusion in the mice from Figure 20e. **b**, Percentage of naive (CD62L+CD44+), central memory (CD62L+CD44+) and effector memory (CD62L-CD44+) UT or CART19 T cells prior to infusion in the mice from Figure 20e. c, Absolute numbers (mean ± SEM) of CD4 (top) and CD8 (bottom) NGFR+ CART19 cells from CTRL + CART19 (n=7), IFN + CART19 (n=6), CTRL + iCART19 (n=7), IFN + iCART19 (n=6) and IFN + iCART19 late (n=6, late intervention trial) mice. Long-term surviving mice are shown in green. Each line represents a single mouse. **d**, Level (mean fluorescent intensity, MFI) of NGFR expression overtime on CD4+ (top) and CD8+ (bottom) CART19 cells of mice from (**c**). Long-term surviving mice are shown in green. Each line represents a single mouse.

**Figure 28**
Structure of constructs NGFR control (top), Tig126/130a (middle) and Tta126/130a (bottom)

**Figure 29**
Assessment of IFNγ delivery on leukaemia growth and the protumoral microenvironment induced by the leukaemia.

**Figure 30**
Assessment of contemporaneous delivery of several immunostimulatory cytokines into the leukaemia microenvironment using the Tie2 vector backbone.

**Figure 31**
Assessment of synergy between gene therapy-based delivery of IFNγ with other immunotherapeutic strategies, including the checkpoint inhibitors anti-CTLA-4 and anti-LAG-3, and the indoleamine-2,3-dioxygenase (IDO) inhibitor, 1-Methyl-Tryptophan (1-MT).

## DETAILED DESCRIPTION

### CELLS

A stem cell is able to differentiate into many cell types. A cell that is able to differentiate into all cell types is known as totipotent. In mammals, only the zygote and early embryonic cells are totipotent. Stem cells are found in most, if not all, multicellular organisms. They are characterised by the ability to renew themselves through mitotic cell division and differentiate into a diverse range of specialised cell types. The two broad types of mammalian stem cells are embryonic stem cells that are isolated from the inner cell mass of blastocysts, and adult stem cells that are found in adult tissues. In a developing embryo, stem cells can differentiate into all of the specialised embryonic tissues. In adult organisms, stem cells and progenitor cells act as a repair system for the body, replenishing specialised cells, but also maintaining the normal turnover of regenerative organs, such as blood, skin or intestinal tissues.

Haematopoietic stem cells (HSCs) are multipotent stem cells that may be found, for example, in peripheral blood, bone marrow and umbilical cord blood. HSCs are capable of self-renewal and differentiation into any blood cell lineage. They are capable of recolonising the entire immune system, and the erythroid and myeloid lineages in all the haematopoietic tissues (such as bone marrow, spleen and thymus). They provide for life-long production of all lineages of haematopoietic cells.

Haematopoietic progenitor cells (HPCs) have the capacity to differentiate into a specific type of cell. In contrast to stem cells however, they are already far more specific: they are pushed to differentiate into their "target" cell. A difference between HSCs and HPCs is that HSCs can replicate indefinitely, whereas HPCs can only divide a limited number of times.

In one embodiment, the present invention may use a mixed population of cells comprising HSCs and HPCs (e.g. a HSPC population).

A differentiated cell is a cell which has become more specialised in comparison to a stem cell or progenitor cell. Differentiation occurs during the development of a multicellular organism as the organism changes from a single zygote to a complex system of tissues and cell types. Differentiation is also a common process in adults: adult stem cells divide and create fully-differentiated daughter cells during tissue repair and normal cell turnover. Differentiation

dramatically changes a cell's size, shape, membrane potential, metabolic activity and responsiveness to signals. These changes are largely due to highly-controlled modifications in gene expression. In other words a differentiated cell is a cell which has specific structures and performs certain functions due to a developmental process which involves the activation and deactivation of specific genes. Here, a differentiated cell includes differentiated cells of the haematopoietic lineage such as monocytes, macrophages, neutrophils, basophils, eosinophils, erythrocytes, megakaryocytes/platelets, dendritic cells, T-cells, B-cells and NK-cells. For example, differentiated cells of the haematopoietic lineage can be distinguished from HSCs and HPCs by detection of cell surface molecules which are not expressed or are expressed to a lesser degree on undifferentiated cells (HSCs and HPCs). Examples of suitable human lineage markers include CD33, CD13, CD14, CD15 (myeloid), CD19, CD20, CD22, CD79a (B), CD36, CD71, CD235a (erythroid), CD2, CD3, CD4, CD8 (T), CD56 (NK).

*Cell source*

In one embodiment, the HSC, the HPC, the myeloid/monocyte-committed progenitor cell, the macrophage or the monocyte used in the present invention is obtained from a tissue sample.

For example, the HSC, the HPC, the myeloid/monocyte-committed progenitor cell, the macrophage or the monocyte can be obtained from adult and foetal peripheral blood, umbilical cord blood, bone marrow, liver or spleen. Preferably, these cells are obtained from peripheral blood or bone marrow. They may be obtained after mobilisation of the cells *in vivo* by means of growth factor treatment.

Mobilisation of HSC or HPC may be carried out using, for example, G-CSF, plerixaphor or combinations thereof. Other agents, such as NSAIDs, CXCR2 ligands (Grobeta) and dipeptidyl peptidase inhibitors may also be useful as mobilising agents.

With the availability of the stem cell growth factors GM-CSF and G-CSF, most haematopoietic stem cell transplantation procedures are now performed using stem cells collected from the peripheral blood, rather than from the bone marrow. Collecting peripheral blood stem cells provides a bigger graft, does not require that the donor be subjected to general anaesthesia to collect the graft, results in a shorter time to engraftment and may provide for a lower long-term relapse rate.

Bone marrow may be collected by standard aspiration methods (either steady-state or after mobilisation), or by using next-generation harvesting tools.

In addition, HSCs may also be derived from induced pluripotent stem cells.

*HSC characteristics*

HSCs are typically of low forward scatter and side scatter profile by flow cytometric procedures. Some are metabolically quiescent, as demonstrated by Rhodamine labelling which allows determination of mitochondrial activity. HSCs may comprise certain cell surface markers such as CD34, CD45, CD133, CD90 and CD49f. They may also be defined as cells lacking the expression of the CD38 and CD45RA cell surface markers. However, expression of some of these markers is dependent upon the developmental stage and tissue-specific context of the HSC. Some HSCs called "side population cells" exclude the Hoechst 33342 dye as detected by flow cytometry. Thus, HSCs have descriptive characteristics that allow for their identification and isolation.

*Negative markers*

CD38 is the most established and useful single negative marker for human HSCs.

Human HSCs may also be negative for lineage markers such as CD2, CD3, CD14, CD16, CD19, CD20, CD24, CD36, CD56, CD66b, CD271 and CD45RA. However, these markers may need to be used in combination for HSC enrichment.

By negative marker it is to be understood that human HSCs lack the expression of these markers.

*Positive markers*

CD34 and CD133 are the most useful positive markers for HSCs.

Some HSCs are also positive for lineage markers such as CD90, CD49f and CD93. However, these markers may need to be used in combination for HSC enrichment.

By positive marker it is to be understood that human HSCs express these markers.

Accordingly, the therapeutic population of cells may be $CD34^{+}CD38^{-}$. Further separations may be carried out to obtain, for example, $CD34^{+}CD38^{-}CD45RA^{-}CD90^{+}CD49f^{+}$ cells.

*miRNA expression*

The expression of miRNAs mir-126 and mir-130a in a cell indicates that the cell is a HSC or a HPC. More specifically, mir-126 expression indicates that the cell is a primitive HPC. Mir-130a expression indicates that the cell is a more primitive HPC.

CYTOKINES

Cytokines are a category of small proteins, typically approximately 5-20 kDa in size, that play an important role in cell signalling. Cytokines may include interferons, chemokines, interleukins, lymphokines and tumour necrosis factors, and are produced by a broad range of cells, including macrophages, B lymphocytes, T lymphocytes, mast cells, endothelial cells and fibroblasts.

Cytokines act through receptors, and have a particularly important role in the immune system, modulating the balance between humoral and cell-based immune responses.

Cytokines include interferons (IFNs), IL-12 and granulocyte-macrophage colony-stimulating factor (GM-CSF).

Interferons (IFNs) are a group of signalling proteins that are made by host cells in response to the presence of pathogens (e.g. viruses, bacteria and parasites) and tumour cells.

IFNs are typically divided into three classes: type I, type II, and type III.

TYPE I INTERFERONS (IFNs)

Type I interferons (IFNs) are a class of cytokines produced by immunes cells (leukocytes, e.g. NK cells, B-cells, T-cells, macrophages etc.). Type I IFNs are referred to as pleiotropic cytokines as they can have a specific IFN effect on multiple cell types.

In one embodiment, the type I IFN is interferon-alpha (IFNα). IFNα is also known as IFN-alpha, IFNa, INFα-1/13.

An example IFNα amino acid sequence is:

MALTFALLVALLVLSCKSSCSVGCDLPQTHSLGSRRTLMLLAQMRRISLFSCLKDRHDFGFP
QEEFGNQFQKAETIPVLHEMIQQIFNLFSTKDSSAAWDETLLDKFYTELYQQLNDLEACVIQ
GVGVTETPLMKEDSILAVRKYFQRITLYLKEKKYSPCAWEVVRAEIMRSFSLSTNLQESLRS
KE

(SEQ ID NO: 6)

In one embodiment, the nucleotide sequence encoding the type I IFN encodes an IFNα protein comprising the amino acid sequence of SEQ ID NO:6. In one embodiment, the nucleotide sequence encoding the type I IFN encodes an IFNα protein comprising an amino acid sequence having at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5% identity to SEQ ID NO:6, and wherein the functionality of a IFNα protein having the amino acid sequence of SEQ ID NO:6 is substantially maintained.

An example nucleotide sequence encoding an IFNα is:

ATGGCCTTGACCTTTGCTTTACTGGTGGCCCTCCTGGTGCTCAGCTGCAAGTCAAGCTGCTC
TGTGGGCTGTGATCTGCCTCAAACCCACAGCCTGGGTAGCAGGAGGACCTTGATGCTCCTGG
CACAGATGAGGAGAATCTCTCTTTTCTCCTGCTTGAAGGACAGACATGACTTTGGATTTCCC
CAGGAGGAGTTTGGCAACCAGTTCCAAAAGGCTGAAACCATCCCTGTCCTCCATGAGATGAT
CCAGCAGATCTTCAATCTCTTCAGCACAAAGGACTCATCTGCTGCTTGGGATGAGACCCTCC
TAGACAAATTCTACACTGAACTCTACCAGCAGCTGAATGACCTGGAAGCCTGTGTGATACAG
GGGGTGGGGGTGACAGAGACTCCCCTGATGAAGGAGGACTCCATTCTGGCTGTGAGGAAATA
CTTCCAAAGAATCACTCTCTATCTGAAAGAGAAGAAATACAGCCCTTGTGCCTGGGAGGTTG
TCAGAGCAGAAATCATGAGATCTTTTTCTTTGTCAACAAACTTGCAAGAAAGTTTAAGAAGT
AAGGAATGA

(SEQ ID NO: 7)

In one embodiment, the nucleotide sequence encoding IFNα comprises the nucleotide sequence of SEQ ID NO: 7. In one embodiment, the nucleotide sequence encoding IFNα comprises a nucleotide sequence having at least 40%, at least 50%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5% identity to SEQ ID NO:7, wherein said nucleotide sequence encodes a protein having substantially the same function as the protein of SEQ ID NO: 6.

In one embodiment, the type 1 IFN is interferon-beta (IFNβ; IFNb).

An example IFNβ amino acid sequence is:

MTNKCLLQIALLLCFSTTALSMSYNLLGFLQRSSNFQCQKLLWQLNGRLEYCLKDRMNFD
IPEEIKQLQQFQKEDAALTIYEMLQNIFAIFRQDSSSTGWNETIVENLLANVYHQINHLK
TVLEEKLEKEDFTRGKLMSSLHLKRYYGRILHYLKAKEYSHCAWTIVRVEILRNFYFINR
LTGYLRN

(SEQ ID NO: 8)

In one embodiment, the nucleotide sequence encoding the type I IFN encodes an IFNβ protein comprising the amino acid sequence of SEQ ID NO: 8. In one embodiment, the nucleotide sequence encoding the type I IFN encodes a IFNβ protein comprising an amino acid sequence having at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5% identity to SEQ ID NO: 8, and wherein the functionality of a IFNβ protein having the amino acid sequence of SEQ ID NO: 8 is substantially maintained.

An example nucleotide sequence encoding an IFNβ is:

gaattctcaggtcgtttgctttcctttgctttctcccaagtcttgttttacaatttgctt
tagtcattcactgaaactttaaaaaacattagaaaacctcacagtttgtaaatctttttc
cctattatatatatcataagataggagcttaaataaagagttttagaaactactaaaatg
taaatgacataggaaaactgaaagggagaagtgaaagtgggaaattcctctgaatagaga
gaggaccatctcatataaataggccatacccacggagaaaggacattctaactgcaacct
ttcgaagcctttgctctggcacaacaggtagtaggcgacactgttcgtgttgtcaacatg
accaacaagtgtctcctccaaattgctctcctgttgtgcttctccactacagctctttcc
atgagctacaacttgcttggattcctacaaagaagcagcaattttcagtgtcagaagctc
ctgtggcaattgaatgggaggcttgaatactgcctcaaggacaggatgaactttgacatc
cctgaggagattaagcagctgcagcagttccagaaggaggacgccgcattgaccatctat
gagatgctccagaacatctttgctattttcagacaagattcatctagcactggctggaat
gagactattgttgagaacctcctggctaatgtctatcatcagataaaccatctgaagaca
gtcctggaagaaaaactggagaaagaagatttcaccaggggaaaactcatgagcagtctg
cacctgaaaagatattatgggaggattctgcattacctgaaggccaaggagtacagtcac
tgtgcctggaccatagtcagagtggaaatcctaaggaacttttacttcattaacagactt
acaggttacctccgaaactgaagatctcctagcctgtgcctctgggactggacaattgct
tcaagcattcttcaaccagcagatgctgtttaagtgactgatggctaatgtactgcatat
gaaaggacactagaagattttgaaattttttattaaattatgagttatttttatttattta
aattttattttggaaaataaattatttttggtgcaaaagtcaacatggcagttttaattt
cgatttgatttatataaccatccatattataaaattgccaagtacctattagttgttctt

tttaaaatatacctgcaaagtagtatactttctggcccctgcctttaaggaatttaaaat
tcaagaaagccatgatggaatatataaggtaagagacaataaggggacctgaaccttatg
ggggaataaatatggcatgaactgctgtgggattaaaagagaaaaggaaagctggagggt
ctggaactaaacctggggttcccattcctcctactgtgtgttccagattctctcatcata
aagttagaattgagctggccatcaggaatagccagaggaatatgtcagcttttgtgttct
ccctaaccttccccagttatttggggggatcactttgctcctcgaaagattttaaataat
tatgtgccccccaccatccctgcaagcttaagggtgagaagtcccatttacttccatgac
actattaagcagcaatctctttattctgctcatcatgggacagccaagatgtgtgggtat
cttaggggagctgtgggtccctgtctgctggcatggcacaggcatcagaggaagaagaac
ctttttataccctagccatctggttagtttctccctagtttttcaaaaaactaagcctg
cttccagtccccactgccttgttcatacagaattc

(SEQ ID NO: 9)

In one embodiment, the nucleotide sequence encoding IFNβ comprises the nucleotide sequence of SEQ ID NO: 9. In one embodiment, the nucleotide sequence encoding IFNβ comprises a nucleotide sequence having at least 40%, at least 50%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5% identity to SEQ ID NO:9, wherein said nucleotide sequence encodes a protein having substantially the same function as the IFNβ protein of SEQ ID NO: 8.

In one embodiment, the nucleotide sequence encoding type I IFN is codon optimised.

TYPE II INTERFERON - INTERFERON-γ (IFNγ)

Interferon-γ (IFNγ), also known as immune interferon, is activated by Interleukin-12 and plays an important role in regulating and activating the immune response.

An example IFNγ amino acid sequence is:

MKYTSYILAFQLCIVLGSLGCYCQDPYVKEAENLKKYFNAGHSDVADNGTLFLGILKNWKEE
SDRKIMQSQIVSFYFKLFKNFKDDQSIQKSVETIKEDMNVKFFNSNKKKRDDFEKLTNYSVT
DLNVQRKAIHELIQVMAELSPAAKTGKRKRSQMLFRGRRASQ

(SEQ ID NO: 12)

Amino acids 1-23 of SEQ ID NO: 12 may act as a signal peptide and be cleaved to form a mature protein that is represented by amino acids 24-161 of SEQ ID NO: 12. An example mature amino acid sequence of IFNγ is:

QDPYVKEAENLKKYFNAGHSDVADNGTLFLGILKNWKEESDRKIMQSQIVSFYFKLFKNFKD
DQSIQKSVETIKEDMNVKFFNSNKKKRDDFEKLTNYSVTDLNVQRKAIHELIQVMAELSPAA
KTGKRKRSQMLFRGRRASQ

(SEQ ID NO: 13)

In one embodiment, the nucleotide sequence encoding the IFNγ encodes an IFNγ protein comprising the amino acid sequence of SEQ ID NO: 12 or 13. In one embodiment, the nucleotide sequence encoding the IFNγ encodes an IFNγ protein comprising an amino acid sequence having at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5% identity to SEQ ID NO: 12 or 13, and wherein the functionality of an IFNγ protein having the amino acid sequence of SEQ ID NO: 12 or 13 is substantially maintained.

A further example IFNγ amino acid sequence is:

MNATHCILALQLFLMAVSGCYCHGTVIESLESLNNYFNSSGIDVEEKSLFLDIWRNWQKDGD
MKILQSQIISFYLRLFEVLKDNQAISNNISVIESHLITTFFSNSKAKKDAFMSIAKFEVNNP
QVQRQAFNELIRVVHQLLPESSLRKRKRSRC

(SEQ ID NO: 17)

In one embodiment, the nucleotide sequence encoding the IFNγ encodes an IFNγ protein comprising the amino acid sequence of SEQ ID NO: 17. In one embodiment, the nucleotide sequence encoding the IFNγ encodes an IFNγ protein comprising an amino acid sequence having at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5% identity to SEQ ID NO: 17, and wherein the functionality of an IFNγ protein having the amino acid sequence of SEQ ID NO: 17 is substantially maintained.

An example nucleotide sequence encoding an IFNγ is:

ATGAAATATACAAGTTATATCTTGGCTTTTCAGCTCTGCATCGTTTTGGGTTCTCTTGGCTG
TTACTGCCAGGACCCATATGTAAAAGAAGCAGAAAACCTTAAGAAATATTTTAATGCAGGTC
ATTCAGATGTAGCGGATAATGGAACTCTTTTCTTAGGCATTTTGAAGAATTGGAAAGAGGAG
AGTGACAGAAAAATAATGCAGAGCCAAATTGTCTCCTTTTACTTCAAACTTTTTAAAAACTT
TAAAGATGACCAGAGCATCCAAAAGAGTGTGGAGACCATCAAGGAAGACATGAATGTCAAGT
TTTTCAATAGCAACAAAAAGAAACGAGATGACTTCGAAAAGCTGACTAATTATTCGGTAACT
GACTTGAATGTCCAACGCAAAGCAATACATGAACTCATCCAAGTGATGGCTGAACTGTCGCC
AGCAGCTAAAACAGGGAAGCGAAAAAGGAGTCAGATGCTGTTTCGAGGTCGAAGAGCATCCC

AGTAA

(SEQ ID NO: 14)

An example nucleotide sequence encoding a mature form of IFNγ is:

CAGGACCCATATGTAAAAGAAGCAGAAAACCTTAAGAAATATTTTAATGCAGGTCATTCAGA
TGTAGCGGATAATGGAACTCTTTTCTTAGGCATTTTGAAGAATTGGAAAGAGGAGAGTGACA
GAAAAATAATGCAGAGCCAAATTGTCTCCTTTTACTTCAAACTTTTTAAAAACTTTAAAGAT
GACCAGAGCATCCAAAAGAGTGTGGAGACCATCAAGGAAGACATGAATGTCAAGTTTTTCAA
TAGCAACAAAAAGAAACGAGATGACTTCGAAAAGCTGACTAATTATTCGGTAACTGACTTGA
ATGTCCAACGCAAAGCAATACATGAACTCATCCAAGTGATGGCTGAACTGTCGCCAGCAGCT
AAAACAGGGAAGCGAAAAAGGAGTCAGATGCTGTTTCGAGGT

(SEQ ID NO: 15)

A further example nucleotide sequence encoding an IFNγ is:

ATGAACGCTACACACTGCATCTTGGCTTTGCAGCTCTTCCTCATGGCTGTTTCTGGCTGTTA
CTGCCACGGCACAGTCATTGAAAGCCTAGAAAGTCTGAATAACTATTTTAACTCAAGTGGCA
TAGATGTGGAAGAAAAGAGTCTCTTCTTGGATATCTGGAGGAACTGGCAAAAGGATGGTGAC
ATGAAAATCCTGCAGAGCCAGATTATCTCTTTCTACCTCAGACTCTTTGAAGTCTTGAAAGA
CAATCAGGCCATCAGCAACAACATAAGCGTCATTGAATCACACCTGATTACTACCTTCTTCA
GCAACAGCAAGGCGAAAAAGGATGCATTCATGAGTATTGCCAAGTTTGAGGTCAACAACCCA
CAGGTCCAGCGCCAAGCATTCAATGAGCTCATCCGAGTGGTCCACCAGCTGTTGCCGGAATC
CAGCCTCAGGAAGCGGAAAAGGAGTCGCTGCTGA

(SEQ ID NO: 16)

In one embodiment, the nucleotide sequence encoding IFNγ comprises the nucleotide sequence of SEQ ID NO: 14, 15 or 16. In one embodiment, the nucleotide sequence encoding IFNγ comprises a nucleotide sequence having at least 40%, at least 50%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5% identity to SEQ ID NO: 14, 15 or 16, wherein said nucleotide sequence encodes a protein having substantially the same function as the protein of SEQ ID NO: 12 or 13.

A further example nucleotide sequence encoding an IFNγ is:

ATGAACGCTACACACTGCATCTTGGCTTTGCAGCTCTTCCTCATGGCTGTTTCTGGCTGTTA
CTGCCACGGCACAGTCATTGAAAGCCTAGAAAGTCTGAATAACTATTTTAACTCAAGTGGCA
TAGATGTGGAAGAAAAGAGTCTCTTCTTGGATATCTGGAGGAACTGGCAAAAGGATGGTGAC
ATGAAAATCCTGCAGAGCCAGATTATCTCTTTCTACCTCAGACTCTTTGAAGTCTTGAAAGA
CAATCAGGCCATCAGCAACAACATAAGCGTCATTGAATCACACCTGATTACTACCTTCTTCA

GCAACAGCAAGGCGAAAAAGGATGCATTCATGAGTATTGCCAAGTTTGAGGTCAACAACCCA
CAGGTCCAGCGCCAAGCATTCAATGAGCTCATCCGAGTGGTCCACCAGCTGTTGCCGGAATC
CAGCCTCAGGAAGCGGAAAAGGAGTCGCTGCTGA

(SEQ ID NO: 18)

In one embodiment, the nucleotide sequence encoding IFNγ comprises the nucleotide sequence of SEQ ID NO: 18. In one embodiment, the nucleotide sequence encoding IFNγ comprises a nucleotide sequence having at least 40%, at least 50%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5% identity to SEQ ID NO: 18, wherein said nucleotide sequence encodes a protein having substantially the same function as the protein of SEQ ID NO: 17.

In one embodiment, the nucleotide sequence encoding the IFNγ is codon optimised.

TUMOUR NECROSIS FACTOR α (TNFα)

Tumor necrosis factor α (TNFα) is a cytokine that is involved in systemic inflammation. Its primary role is in the regulation of immune cells.

TNFα is mainly produced by activated macrophages, but is also produced by other cell types such as CD4+ lymphocytes, NK cells, neutrophils, mast cells, eosinophils, and neurons.

An example TNFα amino acid sequence is:

MSTESMIRDVELAEEALPQKMGGFQNSRRCLCLSLFSFLLVAGATTLFCLLNFGVIGPQRDE
KFPNGLPLISSMAQTLTLRSSSQNSSDKPVAHVVANHQVEEQLEWLSQRANALLANGMDLKD
NQLVVPADGLYLVYSQVLFKGQGCPDYVLLTHTVSRFAISYQEKVNLLSAVKSPCPKDTPEG
AELKPWYEPIYLGGVFQLEKGDQLSAEVNLPKYLDFAESGQVYFGVIAL

(SEQ ID NO: 19)

In one embodiment, the nucleotide sequence encoding TNFα encodes a TNFα protein comprising the amino acid sequence of SEQ ID NO: 19. In one embodiment, the nucleotide sequence encoding the TNFα encodes a TNFα protein comprising an amino acid sequence having at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5% identity to SEQ ID NO: 19, and wherein the functionality of a TNFα protein having the amino acid sequence of SEQ ID NO: 19 is substantially maintained.

An example nucleotide sequence encoding a TNFα is:

ATGAGCACAGAAAGCATGATCCGCGACGTGGAACTGGCAGAAGAGGCACTCCCCCAAAAGAT
GGGGGGCTTCCAGAACTCCAGGCGGTGCCTATGTCTCAGCCTCTTCTCATTCCTGCTTGTGG
CAGGGGCCACCACGCTCTTCTGTCTACTGAACTTCGGGGTGATCGGTCCCCAAAGGGATGAG
AAGTTCCCAAATGGCCTCCCTCTCATCAGTTCTATGGCCCAGACCCTCACACTCAGATCATC
TTCTCAAAATTCGAGTGACAAGCCTGTAGCCCACGTCGTAGCAAACCACCAAGTGGAGGAGC
AGCTGGAGTGGCTGAGCCAGCGCGCCAACGCCCTCCTGGCCAACGGCATGGATCTCAAAGAC
AACCAACTAGTGGTGCCAGCCGATGGGTTGTACCTTGTCTACTCCCAGGTTCTCTTCAAGGG
ACAAGGCTGCCCCGACTACGTGCTCCTCACCCACACCGTCAGCCGATTTGCTATCTCATACC
AGGAGAAAGTCAACCTCCTCTCTGCCGTCAAGAGCCCCTGCCCCAAGGACACCCCTGAGGGG
GCTGAGCTCAAACCCTGGTATGAGCCCATATACCTGGGAGGAGTCTTCCAGCTGGAGAAGGG
GGACCAACTCAGCGCTGAGGTCAATCTGCCCAAGTACTTAGACTTTGCGGAGTCCGGGCAGG
TCTACTTTGGAGTCATTGCTCTG

(SEQ ID NO: 20)

In one embodiment, the nucleotide sequence encoding TNF$\alpha$ comprises the nucleotide sequence of SEQ ID NO: 20. In one embodiment, the nucleotide sequence encoding TNF$\alpha$ comprises a nucleotide sequence having at least 40%, at least 50%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5% identity to SEQ ID NO: 20, wherein said nucleotide sequence encodes a protein having substantially the same function as the protein of SEQ ID NO: 19.

1-METHYL-TRYPTOPHAN (1-MT)

1-Methyltryptophan (1-MT) is an inhibitor of the tryptophan catabolic enzyme indoleamine 2,3-dioxygenase (IDO) and has the structure:

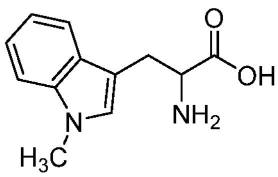

1-MT can be present as a salt or ester, in particular a pharmaceutically-acceptable salt or ester.

The 1-ML may be D-1-MT. the 1-ML may be L-1-MT. The 1-ML may be a racemic mixture.

Pharmaceutically-acceptable salts of the agents of the invention include suitable acid addition or base salts thereof. A review of suitable pharmaceutical salts may be found in Berge et al. (1977) J Pharm Sci 66: 1-19.

The invention also includes where appropriate all enantiomers and tautomers of the agents. The skilled person will recognise compounds that possess optical properties (e.g. one or more chiral carbon atoms) or tautomeric characteristics. The corresponding enantiomers and/or tautomers may be isolated/prepared by methods known in the art.

**MICRO RNA TARGET SEQUENCE**

MicroRNA genes are scattered across all human chromosomes, except for the Y chromosome. They can be either located in non-coding regions of the genome or within introns of protein-coding genes. Around 50% of miRNAs appear in clusters which are transcribed as polycistronic primary transcripts. Similar to protein-coding genes, miRNAs are usually transcribed from polymerase-II promoters, generating a so-called primary miRNA transcript (pri-miRNA). This pri-miRNA is then processed through a series of endonucleolytic cleavage steps, performed by two enzymes belonging to the RNAse Type III family, Drosha and Dicer. From the pri-miRNA, a stem loop of about 60 nucleotides in length, called mirna precursor (pre-mirna), is excised by a specific nuclear complex, composed of Drosha and DiGeorge

syndrome critical region gene (DGCR8), which crops both strands near the base of the primary stem loop and leaves a 5' phosphate and a 2 bp long, 3' overhang. The pre-mirna is then actively transported from the nucleus to the cytoplasm by RAN-GTP and Exportin. Then, Dicer performs a double strand cut at the end of the stem loop not defined by the Drosha cut, generating a 19-24 bp duplex, which is composed of the mature miRNA and the opposite strand of the duplex, called miRNA*. In agreement with the thermodynamic asymmetry rule, only one strand of the duplex is selectively loaded into the RNA-induced silencing complex (RISC), and accumulates as the mature microRNA. This strand is usually the one whose 5' end is less tightly paired to its complement, as was demonstrated by single-nucleotide mismatches introduced into the 5' end of each strand of siRNA duplexes. However, there are some miRNAs that support accumulation of both duplex strands to similar extent.

MicroRNAs trigger RNAi, very much like small interfering RNAs (siRNA) which are extensively being used for experimental gene knockdown. The main difference between miRNA and siRNA is their biogenesis. Once loaded into RISC, the guide strand of the small RNA molecule interacts with mRNA target sequences preferentially found in the 3' untranslated region (3'UTR) of protein-coding genes. It has been shown that nucleotides 2-8 counted from the 5' end of the miRNA, the so-called seed sequence, are essential for triggering RNAi. If the whole guide strand sequence is perfectly complementary to the mRNA target, as is usually the case for siRNAs and plant miRNAs, the mRNA is endonucleolytically cleaved by involvement of the Argonaute (Ago) protein, also called "slicer" of the small RNA duplex into the RNA-induced silencing complex (RISC). DGRC (DiGeorge syndrome critical region gene 8) and TRBP (TAR (HIV) RNA binding protein 2) are double-stranded RNA-binding proteins that facilitate mature miRNA biogenesis by Drosha and Dicer RNase III enzymes, respectively. The guide strand of the miRNA duplex gets incorporated into the effector complex RISC, which recognizes specific targets through imperfect base-pairing and induces post-transcriptional gene silencing. Several mechanisms have been proposed for this mode of regulation: miRNAs can induce the repression of translation initiation, mark target mRNAs for degradation by deadenylation, or sequester targets into the cytoplasmic P-body.

On the other hand, if only the seed is perfectly complementary to the target mRNA but the remaining bases show incomplete pairing, RNAi acts through multiple mechanisms leading to translational repression. Eukaryotic mRNA degradation mainly occurs through the shortening of the polyA tail at the 3' end of the mRNA, and de-capping at the 5' end, followed by 5'-3' exonuclease digestion and accumulation of the miRNA in discrete cytoplasmic areas, the so called P-bodies, enriched in components of the mRNA decay pathway.

According to the present invention, expression of the cytokine, such as the type 1 IFN is regulated by endogenous miRNAs using corresponding miRNA target sequences. Using this method, a miRNA endogenously expressed in a cell prevents or reduces transgene expression in that cell by binding to its corresponding miRNA target sequence positioned in the vector or polynucleotide. This has a number of advantages over relying on tissue specific promoters, not least the fact that tissue specific promoters are often associated with leaky expression in a fraction of non-target cells.

miRNA target sequences that are useful in the present invention are miRNA target sequences which are expressed in HSCs but which are not expressed extensively in differentiated cells e.g., myeloid cells (including tumor-infiltrating macrophages). This is particularly important since IFN$\alpha$ expression is known to be toxic to HSCs.

Preferred examples of miRNA target sequences for use in the invention are mir-130a and mir-126.

Binding of mir-126 miRNA to the mir-126 target sequence blocks expression most effectively in HSC and in cells of the erythroid lineage. Thus, the mir-126 target sequence is particularly suitable for gene therapy applications relying on robust transgene expression in the myeloid and lymphoid lineage.

The mir-126 miRNA may have the nucleotide sequence: UCGUACCGUGAGUAAUAAUGCG (SEQ ID NO: 1).

In one embodiment, the mir-126 target sequence comprises the nucleotide sequence of GCATTATTACTCACGGTACGA (SEQ ID NO:2).

In one embodiment, the vector or polynucleotide used in the present invention comprises at least one, at least two, or at least three, copies of the nucleotide sequence of SEQ ID NO:2. In a preferred embodiment, the mir-126 target sequence comprises two copies of the nucleotide sequence of SEQ ID NO: 2.

In one embodiment the HSC, the HPC, the myeloid/monocyte-committed progenitor cell, the macrophage or the monocyte used in the present invention comprise at least one, at least two, or at least three, copies of the nucleotide sequence of SEQ ID NO:2. In a preferred embodiment, the mir-126 target sequence comprises two copies of the nucleotide sequence of SEQ ID NO: 2.

The copies mir-126 target sequences may be separated by a spacer sequence. The spacer sequence may comprise at least one, at least two, at least three or at least four or at least five nucleotide bases.

Binding of mir-130a miRNA to the mir-130a target sequence blocks expression most effectively in HSC and in cells of the erythroid lineage (similar to miR-126). Thus, the miR-130a target sequence is particularly suitable for gene therapy applications relying on robust transgene expression in the myeloid and lymphoid lineage.

The mir-130a miRNA may have the nucleotide sequence of: CAGUGCAAUGUUAAAAGGGCAU (SEQ ID NO: 3).

In one embodiment, the mir-130a target sequence comprises the nucleotide sequence of ATGCCCTTTTAACATTGCACTG (SEQ ID NO: 4).

In one embodiment, the mir-130a target sequence comprises at least one, at least two or at least three copies of the nucleotide sequence of SEQ ID NO: 4. In a preferred embodiment, the mir-130a target sequence comprises two copies of the nucleotide sequence of SEQ ID NO: 4.

The copies mir-130a target sequences may be separated by a spacer sequence. The spacer sequence may comprise at least one, at least two, at least three, at least four, or at least five nucleotide bases.

In one embodiment, the vector comprises two copies of a mir-126 target sequence and two copies of a mir-130a target sequence.

In one embodiment, two copies of the mir-126 target sequence and two copies of the mir-130a target sequence are comprised in the nucleotide sequence of:

**GCATTATTACTCACGGTACGA**CGAT**GCATTATTACTCACGGTACGA**ACGCGTATGCCCTTTTAACATTGCACTGATGCATATGCCCTTTTAACATTGCACTG (SEQ ID NO: 5).

Combination target sequence, e.g. a target sequence comprising two copies of the mir-126 target sequence and two copies of the mir-130a target sequence are a particular preferred for use in the present invention since use of this combination maximizes repression of the vector in HSC and expression in the myeloid progeny.

Furthermore, when using the combination target, transgene downregulation in HSC is assured by two independent miRNAs, and the risk of interfering with endogenous miRNA regulation is reduced, thus increasing safety and efficacy of the target sequence.

VECTOR

A vector is a tool that allows or facilitates the transfer of an entity from one environment to another. In accordance with the present invention, and by way of example, some vectors used in recombinant nucleic acid techniques allow entities, such as a segment of nucleic acid (e.g. a heterologous DNA segment, such as a heterologous Cdna segment), to be transferred into a target cell. The vector may serve the purpose of maintaining the heterologous nucleic acid (DNA or RNA) within the cell, facilitating the replication of the vector comprising a segment of nucleic acid, or facilitating the expression of the protein encoded by a segment of nucleic acid. Vectors may be non-viral or viral. Examples of vectors used in recombinant nucleic acid techniques include, but are not limited to, plasmids, chromosomes, artificial chromosomes and viruses. The vector may also be, for example, a naked nucleic acid (e.g. DNA). In its simplest form, the vector may itself be a nucleotide of interest.

The vectors used in the invention may be, for example, plasmid or virus vectors and may include a promoter for the expression of a polynucleotide and optionally a regulator of the promoter. In a preferred embodiment the vector is a viral vector.

Vectors comprising polynucleotides used in the invention may be introduced into cells using a variety of techniques known in the art, such as transformation, transfection and transduction. Several techniques are known in the art, for example transduction with recombinant viral vectors, such as retroviral, lentiviral, adenoviral, adeno-associated viral, baculoviral and herpes simplex viral vectors, Sleeping Beauty vectors; direct injection of nucleic acids and biolistic transformation.

Non-viral delivery systems include but are not limited to DNA transfection methods. Here, transfection includes a process using a non-viral vector to deliver a gene to a target cell. Typical transfection methods include electroporation, DNA biolistics, lipid-mediated transfection, compacted DNA-mediated transfection, liposomes, immunoliposomes, lipofectin, cationic agent-mediated transfection, cationic facial amphiphiles (CFAs) (Nature Biotechnology 1996 14; 556) and combinations thereof.

The term "transfection" is to be understood as encompassing the delivery of polynucleotides to cells by both viral and non-viral delivery.

In addition, the invention may employ gene targeting protocols, for example the delivery of DNA-modifying agents.

Viral delivery systems include but are not limited to adenovirus vector, an adeno-associated viral (AAV) vector, a herpes viral vector, a retroviral vector, a lentiviral vector, and a baculoviral vector.

Retroviruses are RNA viruses with a life cycle different to that of lytic viruses. In this regard, a retrovirus is an infectious entity that replicates through a DNA intermediate. When a retrovirus infects a cell, its genome is converted to a DNA form by a reverse transcriptase enzyme. The DNA copy serves as a template for the production of new RNA genomes and virally encoded proteins necessary for the assembly of infectious viral particles.

There are many retroviruses, for example murine leukemia virus (MLV), human immunodeficiency virus (HIV), equine infectious anaemia virus (EIAV), mouse mammary tumour virus (MMTV), Rous sarcoma virus (RSV), Fujinami

sarcoma virus (FuSV), Moloney murine leukemia virus (Mo-MLV), FBR murine osteosarcoma virus (FBR MSV), Moloney murine sarcoma virus (Mo-MSV), Abelson murine leukemia virus (A-MLV), Avian myelocytomatosis virus-29 (MC29), and Avian erythroblastosis virus (AEV) and all other retroviridiae including lentiviruses.

A detailed list of retroviruses may be found in Coffin et al ("Retroviruses" 1997 Cold Spring Harbour Laboratory Press Eds: JM Coffin, SM Hughes, HE Varmus pp 758-763).

Lentiviruses also belong to the retrovirus family, but they can infect both dividing and non-dividing cells (Lewis et al (1992) EMBO J. 3053-3058).

In one embodiment, the vector is a lentiviral vector.

PROMOTER

In one embodiment the vector comprises a tissue specific promoter. Preferably, the tissue specific promoter drives specific expression to a subset of tumor infiltrating myeloid cells, such as Tie2 expressing monocytes (TEM) or M2-polarized macrophages.

In a preferred embodiment, the tissue specific promoter is the TEK (Tie2) promoter.

An example TEK promoter nucleotide sequence is:

```
gatcacgagactagcctcgagtcacacctgcaaactggaaacattaattggttcttaagatc
atcatcgacgtgataaaacctgggacagaaattagtcaagactagctgcatctgccttttcc
tctggtgggtaggaaaaggaggagtataatgatttcctcaggcatgaaggtcgatgatgagc
aaagtgtatactctctaatctaatgtcataattcatattgtggagtaattatctggataagt
gtagggtctctgacctcattctagatattgtacattccatggctattttcattttggtccat
gaactctctttgctctcatgagcaccattttatcccaatctaatcctgtatgtttgtgttt
ttacacagattagtttttaaatgttatatataatttgcttctgaaacaccattgctcaatga
ctaccaaatctttctcattaccaaaatccttctatgccaacttcttcaagaaatttgatcac
ctttagatgaattgttaatgaaaattaaagctatagccggcaacatgggtatctttgggcta
atggccaaccaacaggccatctgtgtgaaagaaaacaggctaacaattttggactctggtct
cttggggctacattgagcattgacctcaccggtgctcactgaaattaattgcttttcaggtt
gtattttctcatcacggaaaccttcttctcccaattcaaaccatgtgggttaaaatgagaaa
acaaaagccaaaacggcttcccacacccaaaagctccttctgtcagagatcccagtagcccc
gggagagctgttagaagtctgagaaggattggtcatcatcgcataccatacataggtggagg
gcttgttattctcagtttcccgcctatgagaggataccccctattgtttctgaaaatgctgac
cgggacccacacttccaacaaaaattcctctgcccctacagcagcagcaaaagcagcagcag
aagcaacagcaacagataagtgttttgatgaattgcgagatggatagggcttgagtgccccc
agccctgctgataccaaatgcctttaagatacagcctttcccatcctaatctacaaaggaaa
caggaaaaaggaacttaaaactccctgtgctcagacagaaatgagactgttacagcctgctt
ctgtgctgttccttcttgcctctaacttgtaaacaagacgtagtaggacgatgctaatggaa
agtcacaaaccgctgggtttttgaaaggatcctagactcgagcggccgcca
```

(SEQ ID NO: 10)

In one embodiment, the TEK promoter comprises the nucleotide sequence of SEQ ID NO: 10. In one embodiment, the TEK promoter comprises a nucleotide sequence having at least 50%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5% identity to SEQ ID NO:10.

The TEK (Tie2) promoter may be combined with the mir-126 target sequence and/or the mir-130a target sequence. This enables specific transgene expression in a subset of tumor-infiltrating myeloid cells, e.g. tumor infiltrating macrophages.

In one embodiment, the vector may further comprise a TEK (Tie2) enhancer sequence.

An example TEK (Tie2) enhancer sequence is:

cttcagacctggaggaggagatatgaggaccaattgtggccagacgattccttaactcgtg
ttacacctgcagaatgagttttagatctagctgtgacctcttcccccagccccacccccatt
gtccccttgtgtgccttcaggaatctgatcattcttctctcctgctccttcccaaaggctgc
aggagcaggtgtgaagacgtggatgtgccagatgcagagtcctgacacttttcaacacatct
gcatattagaggaagtacatacccattgcttggtggtttcatgtctaatgtggtatgagtgt
gacaaagagagggagaaaatttggactagccaaagaagccagtcaggcgtggggtttgaagg
gcatcgtggcggctgtcatttgctctctgcttgtcacagccccttgcccagggcttgacca
gtgaggtgtatgtgctggtcacacccatctcagcagatctgtcagctttcccgcttttgtta
aagggtgatatcatgcttcctgggggggagcactggaagacaatgctcggccactttcctcca
gatacaataggcggagtcaggaaggcagtattgacattgctggggctggggaggcactcact
gctctgcggccgtcagatggtgaaccagcttaaccttggcacacagggcctgggttgtgcaa
ggcgtctggctgcagagccaaaggggactccaccctggggacaggagtgctttagacatctg
ggaatctgggatgggcttcaaattctgatccctgtgtcagaaacaaccacaaaacaataaga
gtaccagtaataacaaaaatgactaccctaggttgaatgcctttatgtgccaagtgtcaatt
g

(SEQ ID NO: 11)

In one embodiment, the TEK enhancer sequence comprises the nucleotide sequence of SEQ ID NO: 11. In one embodiment, the TEK enhancer comprises a nucleotide sequence having at least 50%, at least 60%, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, at least 99.5% identity to SEQ ID NO: 11.

CODON OPTIMISATION

The polynucleotides used in the present invention may be codon-optimised. Codon optimisation has previously been described in WO 1999/41397 and WO 2001/79518.

Different cells differ in their usage of particular codons. This codon bias corresponds to a bias in the relative abundance of particular tRNAs in the cell type. By altering the codons in the sequence so that they are tailored to match with the relative abundance of corresponding tRNAs, it is possible to increase expression. By the same token, it is possible to decrease expression by deliberately choosing codons for which the corresponding tRNAs are known to be rare in the particular cell type. Thus, an additional degree of translational control is available.

Many viruses, including HIV and other lentiviruses, use a large number of rare codons and by changing these to correspond to commonly used mammalian codons, increased expression of the packaging components in mammalian producer cells can be achieved. Codon usage tables are known in the art for mammalian cells, as well as for a variety of other organisms.

Codon optimization may also involve the removal of mRNA instability motifs and cryptic splice sites.

TUMOR ASSOCIATED ANTIGEN (TAA)

As used herein the term tumor associated antigen (TAA; also known as a tumor antigen) refers to an antigenic molecule expressed by a cancer cell, e.g. a tumor cell. A TAA may be recognised by an immune cell which expresses an immune receptor (e.g. a TCR, a transgenic TCR or a CAR) that is able to specifically bind to a portion (e.g. an epitope) of the TAA molecule.

As used herein, the term TAA-specific T-cell refers to a T-cell which expresses a TCR or a CAR that is specific for a TAA.

A T-cell expressing a TAA-specific TCR or TAA-specific CAR may be able to specifically target and kill tumor cells expressing the TAA. The TAA-specific TCR may be a transgenic TCR

In one embodiment, the TAA-specific T-cell may not be genetically engineered. The T cells may be naturally induced tumor-specific T cells. For example, the T cells used in the present invention may be expanded from the tumor or lymph node.

A particular TAA may be expressed at a high relative abundance on a particular type of tumor cell compared to a non-tumor cell (e.g. a healthy cell).

Examples of TAAs include carcinoembryonic antigen (CEA), estrogen receptor, progesterone receptor, ephrinB2, ROR1, mesothelin, c-Met, GD-2, and MAGE A3 TCR, 4-1BB, adenocarcinoma antigen, alpha-fetoprotein,

BAFF, B-lymphoma cell, C242 antigen, carbonic anhydrase 9 (CA-IX), CCR4, CD152, CD200, CD22, CD19, CD22, CD123, CD221, CD23 (IgE receptor), CD28, CD4, CD40, CD44, CD44 v6, CD51, CD52, CD56, CD74, CD80, CS-1, CNT0888, CTLA-4, DR5, EpCAM, CD3, fibronectin extra domain-B, folate receptor 1, glycoprotein 75, GPNMB, HGF, human scatter factor receptor kinase, IGF-1 receptor, IGF-I, IgGI, L1-CAM, IL-13, IL-6, insulin-like growth factor I receptor, integrin $\alpha5\beta1$, integrin $\alpha v\beta3$, MORAb-009, MS4A1, mucin CanAg, N-glycolylneuraminic acid, NPC-1C, PDGF-R$\alpha$, PDL192, phosphatidylserine, prostatic carcinoma cells, RANKL, RON, SCH 900105, SDC1, SLAMF7, tenascin C, TGF beta 2, TGF-$\beta$, TRAIL-R1, TRAIL-R2, tumor antigen CTAA16.88, Vascular endothelial growth, factor (VEGF), VEGF-A, VEGFR-1, VEGFR2, vimentin, 5T4, CD5, CD19, CD20, CD21, CD25, CD37, CD30, CD33, CD45, CAMPATH-1 (CDw52), HLA-DR, Anti-idiotype, TAG-72, Ep-CAM, MUC1, Folate-binding protein, A33, G250, Prostate-specific membrane antigen, (PSMA), Prostate specific antigen (PSA), Ferritin, Gangliosides (e.g. GD2, GD3, GM2), $Le^y$, CA-125, CA19-9, Epidermal growth factor receptor (EGFR), p185HER2, IL-2 receptor, de2-7 EGFR, Fibroblast activation protein (FAP), Tenascin, metalloproteinases, Endosialin, Carbonic anhydrase, Galectin 9, Aldolase A, eIF$\gamma$4, Tyrosinase, Galectin 4, HERKV-K10, p53, NY-LU-12, Restin, NY-CO-38, MAGE-1, MAGE-4a, SSX2, NY-ESO-1, SCP-1, 707-AP, AFP, ART-4, BAGE, b-catenin/m, Bcr-abl, CAMEL, CAP-1, CASP-8, CDC27m, CDK4/m, CT, Cyp-B, DAM-6 (MAGE-B2) and DAM-10 (MAGE-B1), ELF2M, ETV6-AML1, G250, GAGE, GnT-V, Gp100, HAGE, HER-2/neu, HLA-A*0201-R170I, HPV-E7, HSP70-2M, HST-2, hTERT (hTRT), iCE, KIAA0205, LAGE, LDLR/FUT, MAGE, MART-1/Melan-A, MC1R, Myosin/m, MUC1, MUM-1, MUM-2, MUM-3, NA88-A, NY-ESO-1, P15, p190 minor bcr-abl, Pml/RARa, PRAME, RAGE, RU1, RU2, SAGE, SART-1, SART-3, TEL/AML1, TPI/m, TRP-1, protein 1, gp75, TRP-2, TRP-2/INT2 or WT1.

Additional TAAs may be identified using methods known in the art - see, e.g. the review article by Zilberberg et al. 2015 (Biology of Blood and Marrow Transplantation, Volume 21, Issue 6, June 2015, Pages 1000-1007) the content of which is incorporated herein by reference.

T-CELL

As used herein the term "T-cell" may refer to CD8+ T-cells, CD4+ T-cells, naive T-cells, memory stem T-cells, central memory T-cells, double negative T-cells, effector memory T-cells, effector T-cells, Th0 cells, Tc0 cells, Th1 cells, Tc1 cells, Th2 cells, Tc2 cells, Th17 cells, Th22 cells, gamma/delta T-cells.

The T-cell used in the present invention may be used for adoptive T-cell transfer. The present invention also encompasses adoptive transfer of tumor-infiltrating lymphocytes (TIL) and/or marrow infiltrating lymphocytes (MIL).

As used herein the term "adoptive T-cell transfer" refers to the administration of a T-cell population to a patient. A T-cell may be isolated from a subject and then genetically modified and cultured *in vitro* (*ex vivo*) in order to express a TAA-specific TCR or CAR before being administered to the patient.

Adoptive cell transfer may be allogenic or autologous.

By "autologous cell transfer" it is to be understood that the starting population of cells is obtained from the same subject as that to which the transduced T-cell population is administered. Autologous transfer is advantageous as it avoids problems associated with immunological incompatibility and is available to subjects irrespective of the availability of a genetically matched donor.

By "allogeneic cell transfer" it is to be understood that the starting population of cells is obtained from a different subject as that to which the transduced cell population is administered. Preferably, the donor will be genetically matched to the subject to which the cells are administered to minimise the risk of immunological incompatibility. Alternatively, the donor may be mismatched and unrelated to the patient.

Suitable doses of transduced cell populations are such as to be therapeutically and/or prophylactically effective. The dose to be administered may depend on the subject and condition to be treated, and may be readily determined by a skilled person.

The T-cell may be derived from a T-cell isolated from a patient. The T-cell may be part of a mixed cell population isolated from the subject, such as a population of peripheral blood lymphocytes (PBL). T-cells within the PBL population may be activated by methods known in the art, such as using anti-CD3 and/or anti-CD28 antibodies or cell sized beads conjugated with anti-CD3 and/or anti-CD28 antibodies.

The T-cell may be a $CD4^+$ helper T cell or a $CD8^+$ cytotoxic T cell. The T-cell may be in a mixed population of $CD4^+$ helper T cell/$CD8^+$ cytotoxic T-cells. Polyclonal activation, for example using anti-CD3 antibodies optionally in combination with anti-CD28 antibodies will trigger the proliferation of $CD4^+$ and $CD8^+$ T-cells.

The T-cell may be isolated from the subject to which the genetically modified cell is to be adoptively transferred. In this respect, the cell may be made by isolating a T-cell from a subject, optionally activating the T-cell, transferring the TCR gene to the cell *ex vivo.* Subsequent immunotherapy of the subject may then be carried out by adoptive transfer of the TCR-transduced cells. As used herein this process refers to autologous T-cell transfer - i.e. the TCR-transduced cells are administered to the same subject from which the T-cells were originally derived.

Alternatively the T-cell may be isolated from a different subject, such that it is allogeneic. The T-cell may be isolated from a donor subject. For example, if the subject is undergoing allogeneic haematopoietic stem cell transplantation (Allo-HSCT) or solid organ transplantation or cell transplantation or stem cell therapy, the cell may be derived from the donor, from which the organs, tissues or cells are derived. The donor and the subject undergoing treatment may be siblings.

Alternatively the T-cell may be derived from a stem cell, such as a haemopoietic stem cell (HSC). Gene transfer into HSCs does not lead to TCR expression at the cell surface as stem cells do not express CD3 molecules. However, when stem cells differentiate into lymphoid precursors that migrate to the thymus, the initiation of CD3 expression leads to the surface expression of the introduced TCR in thymocytes.

An advantage of this approach is that the mature T-cells, once produced, express only the introduced TCR and little or no endogenous TCR chains, because the expression of the introduced TCR chains suppresses rearrangement of endogenous TCR gene segments to form functional TCR alpha and beta genes. A further benefit is that the gene-modified stem cells are a continuous source of mature T-cells with the desired antigen specificity. Accordingly, the HSC, the HPC, or the vector as defined herein may be used in combination with a gene-modified stem cell, preferably a gene-modified hematopoietic stem cell, which, upon differentiation, produces a T-cell expressing a TAA-specific TCR.

Other approaches known in the art may be used to reduce, limit, prevent, silence, or abrogate expression of endogenous genes in the cells of the present invention or cells prepared by the methods of the present invention.

As used herein the term "disrupting" refers to reducing, limiting, preventing, silencing, or abrogating expression of a gene. The person skilled in the art is able to use any method known in the art to disrupt an endogenous gene, e.g., any suitable method for genome editing, gene silencing, gene knock-down or gene knock-out.

For example, an endogenous gene may be disrupted with an artificial nuclease. An artificial nuclease is, e.g., an artificial restriction enzyme engineered to selectively target a specific polynucleotide sequence (e.g. encoding a gene of interest) and induce a double strand break in said polynucleotide sequence. Typically, the double strand break (DSB) will be repaired by error-prone non-homologous end joining (NHEJ) thereby resulting in the formation of a non-functional polynucleotide sequence, which may be unable to express an endogenous gene.

In some embodiments, the artificial nuclease is selected from the group consisting of zinc finger nucleases (ZFN), transcription activator-like effector nucleases (TALEN) and CRISPR/Cas (e.g. CRISPR/Cas9).

T-CELL RECEPTOR (TCR)

During antigen processing, antigens are degraded inside cells and then carried to the cell surface by major histocompatibility complex (MHC) molecules. T-cells are able to recognise this peptide:MHC complex at the surface of the antigen presenting cell. There are two different classes of MHC molecules: MHC I and MHC II, each class delivers peptides from different cellular compartments to the cell surface.

A T cell receptor (TCR) is a molecule found on the surface of T-cells that is responsible for recognizing antigens bound to MHC molecules. The TCR heterodimer consists of an alpha ($\alpha$) and beta ($\beta$) chain in around 95% of T-cells, whereas around 5% of T-cells have TCRs consisting of gamma ($\gamma$) and delta ($\delta$) chains.

Engagement of the TCR with antigen and MHC results in activation of the T lymphocyte on which the TCR is expressed through a series of biochemical events mediated by associated enzymes, co-receptors, and specialized accessory molecules.

Each chain of the TCR is a member of the immunoglobulin superfamily and possesses one N-terminal immunoglobulin (Ig)-variable (V) domain, one Ig-constant (C) domain, a transmembrane/cell membrane-spanning region, and a short cytoplasmic tail at the C-terminal end.

The variable domain of both the TCR $\alpha$ chain and $\beta$ chain have three hypervariable or complementarity determining regions (CDRs). CDR3 is the main CDR responsible for recognizing processed antigen, although CDR1 of the alpha chain has also been shown to interact with the N-terminal part of the antigenic peptide, whereas CDR1 of the beta chain interacts with the C-terminal part of the peptide. CDR2 is thought to recognize the MHC molecule.

The constant domain of the TCR domain consists of short connecting sequences in which a cysteine residue forms a disulfide bond, making a link between the two chains.

The TCR used in the present invention may have one or more additional cysteine residues in each of the $\alpha$ and $\beta$ chains such that the TCR may comprise two or more disulphide bonds in the constant domains.

The structure allows the TCR to associate with other molecules like CD3 which possess three distinct chains ($\gamma$, $\delta$, and $\varepsilon$) in mammals and the $\zeta$-chain. These accessory molecules have negatively charged transmembrane regions and are vital to propagating the signal from the TCR into the cell. The CD3- and $\zeta$-chains, together with the TCR, form what is known as the T cell receptor complex.

The signal from the T cell complex is enhanced by simultaneous binding of the MHC molecules by a specific co-receptor. For helper T-cells, this co-receptor is CD4 (specific for class II MHC); whereas for cytotoxic T-cells, this co-receptor is CD8 (specific for class I MHC). The co-receptor allows prolonged engagement between the antigen presenting cell and the T cell and recruits essential molecules (e.g., LCK) inside the cell involved in the signalling of the activated T lymphocyte.

Accordingly, as used herein the term "T-cell receptor" (TCR) refers to a molecule capable of recognising a peptide when presented by an MHC molecule. The molecule may be a heterodimer of two chains $\alpha$ and $\beta$ (or optionally $\gamma$ and $\delta$) or it may be a single chain TCR construct.

The TCR used in the present invention may be a hybrid TCR comprising sequences derived from more than one species. For example, it has surprisingly been found that murine TCRs are more efficiently expressed in human T-cells than human TCRs. The TCR may therefore comprise human variable regions and murine constant regions.

A disadvantage of this approach is that the murine constant sequences may trigger an immune response, leading to rejection of the transferred T-cells. However, the conditioning regimens used to prepare patients for adoptive T-cell therapy may result in sufficient immunosuppression to allow the engraftment of T-cells expressing murine sequences.

The portion of the TCR that establishes the majority of the contacts with the antigenic peptide bound to the major histocompatibility complex (MHC) is the complementarity determining region 3 (CDR3), which is unique for each T cell clone. The CDR3 region is generated upon somatic rearrangement events occurring in the thymus and involving non-contiguous genes belonging to the variable (V), diversity (D, for $\beta$ and $\delta$ chains) and joining (J) genes. Furthermore, random nucleotides inserted/deleted at the rearranging loci of each TCR chain gene greatly increase diversity of the highly variable CDR3 sequence. Thus, the frequency of a specific CDR3 sequence in a biological sample indicates the abundance of a specific T cell population. The great diversity of the TCR repertoire in healthy human beings provides a wide range protection towards a variety of foreign antigens presented by MHC molecules on the surface of antigen presenting cells. In this regard, it is of note that theoretically up to $10^{15}$ different TCRs can be generated in the thymus.

T-cell receptor diversity is focused on CDR3 and this region is primarily responsible for antigen recognition.

The CDRs may, for example, comprise one, two, or three substitutions, additions or deletions from the given sequence, provided that the TCR retains the capacity to bind TAA derived peptide when presented by an MHC molecule.

TCRs specific for TAA may be generated easily by the person skilled in the art using any method known in the art.

For example, TAA-specific TCRs maybe identified by the TCR gene capture method of Linnemann et al (Nature Medicine 19, 1534-1541 (2013)). Briefly, this method uses a high-throughput DNA-based strategy to identify TCR sequences by the capture and sequencing of genomic DNA fragments encoding the TCR genes and may be used to identify TAA-specific TCRs.

IMPROVED TCR EXPRESSION AND REDUCED TCR MISPAIRING

Increasing the supply of CD3 molecules may increase TCR expression, for example, in a cell that has been modified to express the TCRs of the present invention. Accordingly, the T cell may be modified (e.g. using a vector) to comprise one or more genes encoding CD3-gamma, CD3-delta, CD3-epsilon and/or CD3-zeta. In one embodiment, the T cell comprises a gene encoding CD3-zeta. The T cell may comprise a gene encoding CD8. The vector encoding such genes may encode a selectable marker or a suicide gene, to increase the safety profile of the genetically engineered cell. The genes may be linked by self-cleaving sequences, such as the 2A self-cleaving sequence.

Alternatively one or more separate vectors encoding a CD3 gene may be provided for co-transfer to a T cell simultaneously, sequentially or separately with one or more vectors encoding TCRs.

The transgenic TCR may be expressed in a T-cell used in the present invention to alter the antigen specificity of the T-cell. TCR-transduced T-cells express at least two TCR alpha and two TCR beta chains. While the endogenous TCR alpha/beta chains form a receptor that is self-tolerant, the introduced TCR alpha/beta chains form a receptor with defined specificity for the given target antigen.

However, TCR gene therapy requires sufficient expression of transferred (i.e. transgenic) TCRs as the transferred TCR might be diluted by the presence of the endogenous TCR, resulting in suboptimal expression of the tumor specific TCR. Furthermore, mispairing between endogenous and introduced chains may occur to form novel receptors, which might display unexpected specificities for self-antigens and cause autoimmune damage when transferred into patients.

Hence, several strategies have been explored to reduce the risk of mispairing between endogenous and introduced TCR chains. Mutations of the TCR alpha/beta interface is one strategy currently employed to reduce unwanted mispairing. For example, the introduction of a cysteine in the constant domains of the alpha and beta chain allows the formation of a disulfide bond and enhances the pairing of the introduced chains while reducing mispairing with wild type chains.

Accordingly, the TCRs used in the present invention may comprise one or more mutations at the $\alpha$ chain/$\beta$ chain interface, such that when the $\alpha$ chain and the $\beta$ chain are expressed in a T-cell, the frequency of mispairing between said chains and endogenous TCR $\alpha$ and $\beta$ chains is reduced. In one embodiment, the one or more mutations introduce a cysteine residue into the constant region domain of each of the $\alpha$ chain and the $\beta$ chain, wherein the cysteine residues are capable of forming a disulphide bond between the $\alpha$ chain and the $\beta$ chain.

Another strategy to reduce mispairing relies on the introduction of polynucleotide sequences encoding siRNA,

added to the genes encoding for the tumor specific TCR α and or β chains, and designed to limit the expression of the endogenous TCR genes (Okamoto S. Cancer research 69, 9003-9011, 2009).

Accordingly, the vector or polynucleotide encoding the TCRs used in the present invention may comprise one or more siRNA or other agents aimed at limiting or abrogating the expression of the endogenous TCR genes.

It is also possible to combine artificial nucleases, such as zinc finger nucleases (ZFN), transcription activator-like effector nucleases (TALEN) or CRISPR/Cas systems, designed to target the constant regions of the endogenous TCR genes (TRAC and, or TRBC), to obtain the permanent disruption of the endogenous TCR alpha and/or beta chain genes, thus allowing full expression of the tumor specific TCR and thus reducing or abrogating the risk of TCR mispairing. This process, known as the TCR gene editing proved superior to TCR gene transfer *in vitro* and *in vivo* (Provasi E., Genovese P., Nature Medicine May; 18(5):807-15; 2012).

In addition, the genome editing technology allows targeted integration of a expression cassette, comprising a polynucleotide encoding a TCR used in the present invention, and optionally one or more promoter regions and/or other expression control sequences, into an endogenous gene disrupted by the artificial nucleases (Lombardo A., Nature biotechnology 25, 1298-1306; 2007).

Another strategy developed to increase expression of transgenic TCRs and to reduce TCR mispairing consists in "murinization," which replaces the human TCR α and TCR β constant regions (e.g. the TRAC, TRBC1 and TRBC2 regions) by their murine counterparts. Murizination of TCR constant regions is described in, for example, Sommermeyer and Uckert J Immunol; 2010 (184:6223-6231). Accordingly, the TCR used in the present invention may be murinized.

CHIMERIC ANTIGEN RECEPTOR (CAR)

CARs comprise an extracellular ligand binding domain, most commonly a single chain variable fragment of a monoclonal antibody (scFv) linked to intracellular signaling components, most commonly CD3ζ alone or combined with one or more costimulatory domains. A spacer is often added between the extracellular antigen-binding domain and the transmembrane moiety to optimize the interaction with the target.

A CAR for use in the present invention may comprise:

(i) an antigen-specific targeting domain;
(ii) a transmembrane domain;
(iii) optionally at least one costimulatory domain; and
(iv) an intracellular signaling domain.

Preferably the antigen-specific targeting domain comprises an antibody or fragment thereof, more preferably a single chain variable fragment.

Preferably the antigen-specific targeting domain targets a TAA.

In one embodiment, the antigen-specific targeting domain targets CD19.

Examples of transmembrane domains include a transmembrane domain of a zeta chain of a T cell receptor complex, CD28 and CD8a.

Examples of costimulatory domains include a costimulating domain from CD28, CD137 (4-1BB), CD134 (OX40), DaplO, CD27, CD2, CD5, ICAM-1, LFA-1, Lck, TNFR-I, TNFR-II, Fas, CD30 and CD40.

In one embodiment, the costimulatory domain is a costimulating domain from CD28.

Examples of intracellular signaling domains include human CD3 zeta chain, FcyRIII, FcsRI, a cytoplasmic tail of a Fc receptor and an immunoreceptor tyrosine-based activation motif (ITAM) bearing cytoplasmic receptors.

*Chimeric Antigen Receptors*

"Chimeric antigen receptor" or "CAR" or "CARs" as used herein refers to engineered receptors which can confer an antigen specificity onto cells (for example T cells such as naive T cells, central memory T cells, effector memory T cells or combinations thereof). CARs are also known as artificial T-cell receptors, chimeric T-cell receptors or chimeric immunoreceptors. Preferably the CARs of the invention comprise an antigen-specific targeting region, an extracellular domain, a transmembrane domain, optionally one or more co-stimulatory domains, and an intracellular signaling domain.

*Antigen-specific targeting domain*

The antigen-specific targeting domain provides the CAR with the ability to bind to the target antigen of interest. The antigen-specific targeting domain preferably targets an antigen of clinical interest against which it would be desirable to trigger an effector immune response that results in tumor killing.

The antigen-specific targeting domain may be any protein or peptide that possesses the ability to specifically

recognize and bind to a biological molecule (e.g., a cell surface receptor or tumor protein, or a component thereof). The antigen-specific targeting domain includes any naturally occurring, synthetic, semi-synthetic, or recombinantly produced binding partner for a biological molecule of interest.

Illustrative antigen-specific targeting domains include antibodies or antibody fragments or derivatives, extracellular domains of receptors, ligands for cell surface molecules/receptors, or receptor binding domains thereof, and tumor binding proteins.

In a preferred embodiment, the antigen-specific targeting domain is, or is derived from, an antibody. An antibody-derived targeting domain can be a fragment of an antibody or a genetically engineered product of one or more fragments of the antibody, which fragment is involved in binding with the antigen. Examples include a variable region (Fv), a complementarity determining region (CDR), a Fab, a single chain antibody (scFv), a heavy chain variable region (VH), a light chain variable region (VL) and a camelid antibody (VHH).

In a preferred embodiment, the binding domain is a single chain antibody (scFv). The scFv may be murine, human or humanized scFv.

"Complementarity determining region" or "CDR" with regard to an antibody or antigen-binding fragment thereof refers to a highly variable loop in the variable region of the heavy chain or the light chain of an antibody. CDRs can interact with the antigen conformation and largely determine binding to the antigen (although some framework regions are known to be involved in binding). The heavy chain variable region and the light chain variable region each contain 3 CDRs.

"Heavy chain variable region" or "VH" refers to the fragment of the heavy chain of an antibody that contains three CDRs interposed between flanking stretches known as framework regions, which are more highly conserved than the CDRs and form a scaffold to support the CDRs.

"Light chain variable region" or "VL" refers to the fragment of the light chain of an antibody that contains three CDRs interposed between framework regions.

"Fv" refers to the smallest fragment of an antibody to bear the complete antigen binding site. An Fv fragment consists of the variable region of a single light chain bound to the variable region of a single heavy chain.

"Single-chain Fv antibody" or "scFv" refers to an engineered antibody consisting of a light chain variable region and a heavy chain variable region connected to one another directly or via a peptide linker sequence.

Antibodies that specifically bind a tumor cell surface molecule can be prepared using methods well known in the art. Such methods include phage display, methods to generate human or humanized antibodies, or methods using a transgenic animal or plant engineered to produce human antibodies. Phage display libraries of partially or fully synthetic antibodies are available and can be screened for an antibody or fragment thereof that can bind to the target molecule. Phage display libraries of human antibodies are also available. Once identified, the amino acid sequence or polynucleotide sequence coding for the antibody can be isolated and/or determined.

With respect to targeting domains that target cancer antigens, the selection of the targeting domain will depend on the type of cancer to be treated, and may target tumor antigens. A tumor sample from a subject may be characterized for the presence of certain biomarkers or cell surface markers. For example, breast cancer cells from a subject may be positive or negative for each of Her2Neu, Estrogen receptor, and/or the Progesterone receptor. A tumor antigen or cell surface molecule is selected that is found on the individual subject's tumor cells. Preferably the antigen-specific targeting domain targets a cell surface molecule that is found on tumor cells and is not substantially found on normal tissues, or restricted in its expression to non-vital normal tissues.

TAA which may be targeted by the CAR used in the present invention include but are not limited to any one or more of carcinoembryonic antigen (CEA), estrogen receptor, progesterone receptor, ephrinB2, ROR1, mesothelin, c-Met, GD-2, and MAGE A3 TCR, 4-1BB, adenocarcinoma antigen, alpha-fetoprotein, BAFF, B-lymphoma cell, C242 antigen, carbonic anhydrase 9 (CA-IX), CCR4, CD152, CD200, CD22, CD19, CD22, CD123, CD221, CD23 (IgE receptor), CD28, CD4, CD40, CD44, CD44 v6, CD51, CD52, CD56, CD74, CD80, CS-1, CNT0888, CTLA-4, DR5, EpCAM, CD3, fibronectin extra domain-B, folate receptor 1, glycoprotein 75, GPNMB, HGF, human scatter factor receptor kinase, IGF-1 receptor, IGF-I, IgGI, L1-CAM, IL-13, IL-6, insulin-like growth factor I receptor, integrin $\alpha 5\beta 1$, integrin $\alpha v\beta 3$, MORAb-009, MS4A1, mucin CanAg, N-glycolylneuraminic acid, NPC-1C, PDGF-R$\alpha$, PDL192, phosphatidylserine, prostatic carcinoma cells, RANKL, RON, SCH 900105, SDC1, SLAMF7, tenascin C, TGF beta 2, TGF-$\beta$, TRAIL-R1, TRAIL-R2, tumor antigen CTAA16.88, Vascular endothelial growth, factor (VEGF), VEGF-A, VEGFR-1, VEGFR2, vimentin, 5T4, CD5, CD19, CD20, CD21, CD25, CD37, CD30, CD33, CD45, CAMPATH-1 (CDw52), HLA-DR, Anti-idiotype, TAG-72, Ep-CAM, MUC1, Folate-binding protein, A33, G250, Prostate-specific membrane antigen, (PSMA), Prostate specific antigen (PSA), Ferritin, Gangliosides (e.g. GD2, GD3, GM2), $Le^y$, CA-125, CA19-9, Epidermal growth factor receptor (EGFR), p185HER2, IL-2 receptor, de2-7 EGFR, Fibroblast activation protein (FAP), Tenascin, metalloproteinases, Endosialin, Carbonic anhydrase, Galectin 9, Aldolase A, elF$\gamma$4, Tyrosinase, Galectin 4, HERKV-K10, p53, NY-LU-12, Restin, NY-CO-38, MAGE-1, MAGE-4a, SSX2, NY-ESO-1, SCP-1, 707-AP, AFP, ART-4, BAGE, b-catenin/m, Bcr-abl, CAMEL, CAP-1, CASP-8, CDC27m, CDK4/m, CT, Cyp-B, DAM-6 (MAGE-B2) and DAM-10 (MAGE-B1), ELF2M, ETV6-AML1, G250, GAGE, GnT-V, Gp100, HAGE, HER-2/neu, HLA-A*0201-R170I, HPV-E7, HSP70-2M, HST-2, hTERT

(hTRT), iCE, KIAA0205, LAGE, LDLR/FUT, MAGE, MART-1/Melan-A, MC1R, Myosin/m, MUC1, MUM-1, MUM-2, MUM-3, NA88-A, NY-ESO-1, P15, p190 minor bcr-abl, Pml/RARa, PRAME, RAGE, RU1, RU2, SAGE, SART-1, SART-3, TEL/AML1, TPI/m, TRP-1, protein 1, gp75, TRP-2, TRP-2/INT2 or WT1.

*Co-stimulatory domain*

The CAR used in the present invention may also comprise one or more co-stimulatory domains. This domain may enhance cell proliferation, cell survival and development of memory cells.

Each co-stimulatory domain comprises the co-stimulatory domain of any one or more of, for example, members of the TNFR super family, CD28, CD137 (4-1BB), CD134 (OX40), DaplO, CD27, CD2, CD5, ICAM-1, LFA-1, Lck, TNFR-1, TNFR-II, Fas, CD30, CD40 or combinations thereof. Co-stimulatory domains from other proteins may also be used with the CAR used in the present invention. Additional co-stimulatory domains will be apparent to those of skill in the art.

*Intracellular signaling domain*

The CAR used in the present invention may also comprise an intracellular signaling domain. This domain may be cytoplasmic and may transduce the effector function signal and direct the cell to perform its specialized function. Examples of intracellular signaling domains include, but are not limited to, ζ chain of the T-cell receptor or any of its homologs (e.g., η chain, FcεR1γ and β chains, MB1 (Igα) chain, B29 (Igβ) chain, etc.), CD3 polypeptides (Δ, δ and ε), syk family tyrosine kinases (Syk, ZAP 70, etc.), src family tyrosine kinases (Lck, Fyn, Lyn, etc.) and other molecules involved in T-cell transduction, such as CD2, CD5 and CD28. The intracellular signaling domain may be human CD3 zeta chain, FcyRIII, FcsRI, cytoplasmic tails of Fc receptors, immunoreceptor tyrosine-based activation motif (ITAM) bearing cytoplasmic receptors or combinations thereof.

*Transmembrane domain*

The CAR used in the present invention may also comprise a transmembrane domain. The transmembrane domain may comprise the transmembrane sequence from any protein which has a transmembrane domain, including any of the type I, type II or type III transmembrane proteins. The transmembrane domain of the CAR used in the present invention may also comprise an artificial hydrophobic sequence. The transmembrane domains of the CARs used in the present invention may be selected so as not to dimerize. Additional transmembrane domains will be apparent to those of skill in the art. Examples of transmembrane (TM) regions used in CAR constructs are: 1) The CD28 TM region (Pule et al, Mol Ther, 2005, Nov;12(5):933-41; Brentjens et al, CCR, 2007, Sep 15;13(18 Pt 1):5426-35; Casucci et al, Blood, 2013, Nov 14;122(20):3461-72.); 2) The OX40 TM region (Pule et al, Mol Ther, 2005, Nov;12(5):933-41); 3) The 41BB TM region (Brentjens et al, CCR, 2007, Sep 15;13(18 Pt 1):5426-35); 4) The CD3 zeta TM region (Pule et al, Mol Ther, 2005, Nov;12(5):933-41; Savoldo B, Blood, 2009, Jun 18;113(25):6392-402.); 5) The CD8a TM region (Maher et al, Nat Biotechnol, 2002, Jan;20(1):70-5.; Imai C, Leukemia, 2004, Apr;18(4):676-84; Brentjens et al, CCR, 2007, Sep 15;13(18 Pt 1):5426-35; Milone et al, Mol Ther, 2009, Aug;17(8):1453-64.).

IMMUNE CHECKPOINT INHIBITOR

As used herein the term immune checkpoint inhibitor refers to a molecule, compound, antibody or drug that inhibits, blocks, prevents, reduces or downregulates the expression of, or is otherwise antagonistic to, an inhibitory checkpoint molecule. When expressed on the cell surface, an inhibitory checkpoint molecule inhibits or dampens the T-cell-mediated immune response to said cell. For example, expression of inhibitory checkpoint molecules may prevent a cell from being killed by a T-cell response. This mechanism is particularly deleterious where a cancer cell expresses inhibitory checkpoint molecules as this may allow the cancer cell to evade the host T-cell response. Accordingly, when inhibitory checkpoint molecules on tumor cells are inhibited by an immune checkpoint inhibitor, an enhanced host T-cell response against the tumor cell should occur.

In one embodiment, the immune checkpoint inhibitor inhibits an inhibitory checkpoint molecule selected from the group consisting of A2AR (Adenosine A2A receptor), B7-H3 (CD276), B7-H4 (VTCN1), BTLA (B and T Lymphocyte Attenuator; CD272), HVEM (Herpesvirus Entry Mediator), CTLA-4 (Cytotoxic T-Lymphocyte-Associated protein 4; CD152), IDO (Indoleamine 2,3-dioxygenase), TDO (tryptophan 2,3-dioxygenase), KIR (Killer-cell Immunoglobulin-like Receptor), LAG3 (Lymphocyte Activation Gene-3), PD-1 (Programmed Death 1 receptor), PD-L1 (PD-1 ligand 1), PD-L2 (PD-1 ligand 2), TIM-3 (T-cell Immunoglobulin domain and Mucin domain 3), VISTA (V-domain Ig Suppressor of T cell Activation), B7-1 (CD80), B7-2 (CD86). Combinations of check point inhibitors may also be used.

In one embodiment, the immune checkpoint inhibitor is a PD-1 inhibitor; preferably the PD-1 inhibitor is an anti-PD-1 antibody.

In another embodiment, the immune checkpoint inhibitor is a PD-L1 inhibitor; preferably the PD-L1 inhibitor is an anti-PD-L1 antibody.

In another embodiment, the immune checkpoint inhibitor is a PD-L2 inhibitor, preferably the PD-L2 inhibitor is an anti-PD-L2 antibody.

In another embodiment, the immune checkpoint inhibitor is a CTLA4 inhibitor, preferably the CTLA4 inhibitor is an anti-CTLA4 antibody.

In another embodiment, the immune checkpoint inhibitor is a LAG-3 inhibitor; preferably the LAG-3 inhibitor is an anti-LAG-3 antibody.

As used herein, the term "antibody" is understood as a polypeptide substantially encoded by an immunoglobulin gene or immunoglobulin genes, or fragments thereof, which specifically bind and recognize an antigen (e.g. a cell surface marker). As used herein, the term "antibody" refers to a whole or intact antibody molecule (e.g., IgM, IgG (including IgG1, IgG2, IgG3, and IgG4), IgA, IgD, or IgE) or any antigen-binding fragment thereof.

An antibody may be a polyclonal antibody or a monoclonal antibody. Monoclonal antibodies are produced by identical immune cells (e.g. hybridomas that may be generated from the fusion of an antibody producing B-cell line and a cancerous B-cell line). A monoclonal antibody directed to a particular antigen will recognise a single specific epitope on said antigen. In contrast, polyclonal antibodies are produced from multiple non-identical cell lines and therefore recognise several different epitopes on a particular antigen.

Antigen-binding fragments of an antibody include, e.g., a single chain antibody, a single chain Fv fragment (scFv), an Fd fragment, an Fab fragment, an Fab' fragment, or an $F(ab')_2$ fragment. An scFv fragment is a single polypeptide chain that includes both the heavy and light chain variable regions of the antibody from which the scFv is derived. In addition, intrabodies, minibodies, triabodies, and diabodies (see, e.g., Todorovska et al. (2001) J Immunol Methods 248(1):47-66; Hudson and Kortt (1999) J Immunol Methods 231(1):177-189; Poljak 25 (1994) Structure 2(12): 1121-1123; Rondon and Marasco (1997) Annual Review of Microbiology 21:257-283, are also included in the definition of antibody and are compatible for use in the methods described herein. The term "antibody," as used herein, also includes antibody fragments either produced by the modification of whole antibodies or those synthesized *de novo* using recombinant methods.

Suitable methods for producing an antibody or antigen binding fragments thereof directed to a particular antigen are known in the art (see, e.g., Greenfield (2014) Antibodies: A Laboratory Manual, Second Edition 201-221).

VARIANTS, DERIVATIVES, ANALOGUES, HOMOLOGUES AND FRAGMENTS

In addition to the specific proteins and polynucleotides mentioned herein, the present invention also encompasses the use of variants, derivatives, analogues, homologues and fragments thereof.

In the context of the present invention, a variant of any given sequence is a sequence in which the specific sequence of residues (whether amino acid or nucleic acid residues) has been modified in such a manner that the polypeptide or polynucleotide in question substantially retains at least one of its endogenous functions. A variant sequence can be obtained by addition, deletion, substitution, modification, replacement and/or variation of at least one residue present in the naturally-occurring protein.

The term "derivative" as used herein, in relation to proteins or polypeptides of the present invention includes any substitution of, variation of, modification of, replacement of, deletion of and/or addition of one (or more) amino acid residues from or to the sequence providing that the resultant protein or polypeptide substantially retains at least one of its endogenous functions.

The term "analogue" as used herein, in relation to polypeptides or polynucleotides includes any mimetic, that is, a chemical compound that possesses at least one of the endogenous functions of the polypeptides or polynucleotides which it mimics.

Proteins used in the present invention may also have deletions, insertions or substitutions of amino acid residues which produce a silent change and result in a functionally equivalent protein. Deliberate amino acid substitutions may be made on the basis of similarity in polarity, charge, solubility, hydrophobicity, hydrophilicity and/or the amphipathic nature of the residues as long as the endogenous function is retained. For example, negatively charged amino acids include aspartic acid and glutamic acid; positively charged amino acids include lysine and arginine; and amino acids with uncharged polar head groups having similar hydrophilicity values include asparagine, glutamine, serine, threonine and tyrosine.

A substitution may involve replacement of an amino acid for a similar amino acid (a conservative substitution). A similar amino acid is one which has a side chain moiety with related properties as grouped together, for example as shown below:

(i) basic side chains: lysine (K), arginine (R), histidine (H);

(ii) acidic side chains: aspartic acid (D) and glutamic acid (E);

(iii) uncharged polar side chains: asparagine (N), glutamine (Q), serine (S), threonine (T) and tyrosine (Y); or

(iv) non-polar side chains: glycine (G), alanine (A), valine (V), leucine (L), isoleucine (I), proline (P), phenylalanine (F), methionine (M), tryptophan (W) and cysteine (C).

Variant sequences may comprise amino acid substitutions, additions, deletions and/or insertions.

Conservative substitutions, additions or deletions may be made, for example according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar - charged | D E |
| | | K R |
| AROMATIC | | H F W Y |

The present invention also encompasses homologous substitution (substitution and replacement are both used herein to mean the interchange of an existing amino acid residue, with an alternative residue), e.g. like-for-like substitution such as basic for basic, acidic for acidic, polar for polar etc. Non-homologous substitution may also occur e.g. from one class of residue to another or alternatively involving the inclusion of unnatural amino acids, such as ornithine.

The term "variant" as used herein may mean an entity having a certain homology with the wild type amino acid sequence or the wild type nucleotide sequence. The term "homology" can be equated with "identity".

A variant sequence may include an amino acid sequence which may be at least 50%, 55%, 65%, 75%, 85% or 90% identical, preferably at least 95%, at least 97%, or at least 99% identical to the subject sequence. Typically, the variants will comprise the same active sites etc. as the subject amino acid sequence. Although homology can also be considered in terms of similarity (i.e. amino acid residues having similar chemical properties/functions), in the context of the present invention it is preferred to express homology in terms of sequence identity.

A variant sequence may include a nucleotide sequence which may be at least 40%, 45%, 50%, 55%, 65%, 75%, 85% or 90% identical, preferably at least 95%, at least 97%, or at least 99% identical to the subject sequence. Although homology can also be considered in terms of similarity, in the context of the present invention it is preferred to express homology in terms of sequence identity.

Preferably, reference to a sequence which has a percent identity to any one of the SEQ ID NOs detailed herein refers to a sequence which has the stated percent identity over the entire length of the SEQ ID NO referred to.

Identity comparisons can be conducted by eye or, more usually, with the aid of readily available sequence comparison programs. These commercially available computer programs can calculate percentage homology or identity between two or more sequences.

Percentage homology may be calculated over contiguous sequences, i.e. one sequence is aligned with the other sequence and each amino acid in one sequence is directly compared with the corresponding amino acid in the other sequence, one residue at a time. This is called an "ungapped" alignment. Typically, such ungapped alignments are performed only over a relatively short number of residues.

Although this is a very simple and consistent method, it fails to take into consideration that, for example, in an otherwise identical pair of sequences, one insertion or deletion in the nucleotide sequence may cause the following codons to be put out of alignment, thus potentially resulting in a large reduction in percent homology when a global alignment is performed. Consequently, most sequence comparison methods are designed to produce optimal alignments that take into consideration possible insertions and deletions without penalising unduly the overall homology score. This is achieved by inserting "gaps" in the sequence alignment to try to maximise local homology.

However, these more complex methods assign "gap penalties" to each gap that occurs in the alignment so that, for the same number of identical amino acids, a sequence alignment with as few gaps as possible, reflecting higher relatedness between the two compared sequences, will achieve a higher score than one with many gaps. "Affine gap costs" are typically used that charge a relatively high cost for the existence of a gap and a smaller penalty for each

subsequent residue in the gap. This is the most commonly used gap scoring system. High gap penalties will of course produce optimised alignments with fewer gaps. Most alignment programs allow the gap penalties to be modified. However, it is preferred to use the default values when using such software for sequence comparisons. For example when using the GCG Wisconsin Bestfit package the default gap penalty for amino acid sequences is -12 for a gap and -4 for each extension.

Calculation of maximum percentage homology therefore firstly requires the production of an optimal alignment, taking into consideration gap penalties. A suitable computer program for carrying out such an alignment is the GCG Wisconsin Bestfit package (University of Wisconsin, U.S.A.; Devereux et al. (1984) Nucleic Acids Res. 12: 387). Examples of other software that can perform sequence comparisons include, but are not limited to, the BLAST package (see Ausubel et al. (1999) ibid - Ch. 18), FASTA (Atschul et al. (1990) J. Mol. Biol. 403-410) and the GENEWORKS suite of comparison tools. Both BLAST and FASTA are available for offline and online searching (see Ausubel et al. (1999) ibid, pages 7-58 to 7-60). However, for some applications, it is preferred to use the GCG Bestfit program. Another tool, called BLAST 2 Sequences is also available for comparing protein and nucleotide sequences (see FEMS Microbiol. Lett. (1999) 174: 247-50; FEMS Microbiol. Lett. (1999) 177: 187-8).

Although the final percentage homology can be measured in terms of identity, the alignment process itself is typically not based on an all-or-nothing pair comparison. Instead, a scaled similarity score matrix is generally used that assigns scores to each pairwise comparison based on chemical similarity or evolutionary distance. An example of such a matrix commonly used is the BLOSUM62 matrix - the default matrix for the BLAST suite of programs. GCG Wisconsin programs generally use either the public default values or a custom symbol comparison table if supplied (see the user manual for further details). For some applications, it is preferred to use the public default values for the GCG package, or in the case of other software, the default matrix, such as BLOSUM62.

Once the software has produced an optimal alignment, it is possible to calculate percentage homology, preferably percentage sequence identity. The software typically does this as part of the sequence comparison and generates a numerical result.

"Fragments" are also variants and the term typically refers to a selected region of the polypeptide or polynucleotide that is of interest either functionally or, for example, in an assay. "Fragment" thus refers to an amino acid or nucleic acid sequence that is a portion of a full-length polypeptide or polynucleotide.

Such variants may be prepared using standard recombinant DNA techniques such as site-directed mutagenesis. Where insertions are to be made, synthetic DNA encoding the insertion together with 5' and 3' flanking regions corresponding to the naturally-occurring sequence either side of the insertion site may be made. The flanking regions will contain convenient restriction sites corresponding to sites in the naturally-occurring sequence so that the sequence may be cut with the appropriate enzyme(s) and the synthetic DNA ligated into the cut. The DNA is then expressed in accordance with the invention to make the encoded protein. These methods are only illustrative of the numerous standard techniques known in the art for manipulation of DNA sequences and other known techniques may also be used.

COMBINATION

The HSCs, HPCs, myeloid/monocyte-committed progenitor cells, macrophages, or monocytes of the present invention may be used in combination with a TAA-specific T cell. In some embodiments, the HSCs, HPCs, myeloid/monocyte-committed progenitor cells, macrophages, or monocytes are used further in combination with an immune checkpoint inhibitor.

Alternatively, the HSCs, HPCs, myeloid/monocyte-committed progenitor cells, macrophages, or monocytes of the present invention may be used in combination with a TAA-specific T cell expressing a CAR. In some embodiments, the HSCs, HPCs, myeloid/monocyte-committed progenitor cells, macrophages, or monocytes are used further in combination with an immune checkpoint inhibitor.

Alternatively, the HSCs, HPCs, myeloid/monocyte-committed progenitor cells, macrophages, or monocytes of the present invention may be used in combination with a TAA-specific T cell expressing a transgenic TCR. In some embodiments, the HSCs, HPCs, myeloid/monocyte-committed progenitor cells, macrophages, or monocytes are used further in combination with an immune checkpoint inhibitor.

As used herein, the phrase "used in combination with" encompasses administration of the HSCs, HPCs, myeloid/monocyte-committed progenitor cells, macrophages, or monocytes of the present invention sequentially, separately or simultaneously with an immune checkpoint inhibitor and/or a TAA-specific T cell.

The HSC, HPC, myeloid/monocyte-committed progenitor cells, macrophages, or monocytes of the present invention may be administered at least 5 minutes, at least 10 minutes, at least 15 minutes, at least 30 minutes, at least 45 minutes, at least 60 minutes prior a TAA-specific T-cell and/or an immune checkpoint inhibitor.

The HSC, HPC, myeloid/monocyte-committed progenitor cells, macrophages, or monocytes of the present invention may be administered at least 1 hour, at least 2 hours, at least 3 hours, at least 4 hours, at least 5 hours, at least 6 hours, at least 12 hours, at least 24 hours, at least 48 hours, at least 72 hours prior to a TAA-specific T-cell and/or

an immune checkpoint inhibitor.

The HSC, HPC, myeloid/monocyte-committed progenitor cells, macrophages, or monocytes of the present invention may be administered at least one day, at least two days, at least three days, at least four days, at least five days, at least six days, at least seven days, or at least 14 days prior to a TAA-specific T-cell and/or an immune checkpoint inhibitor.

The HSC, the HPC, myeloid/monocyte-committed progenitor cells, macrophages, or monocytes of the present invention may be administered at least one week, at least two weeks, at least three weeks, at least four weeks, at least five weeks, at least six weeks, at least seven weeks, at least eight weeks, at least nine weeks, at least 10 weeks, at least 11 weeks, or at least 12 weeks prior to a TAA-specific T-cell and/or an immune checkpoint inhibitor.

The HSC, the HPC, myeloid/monocyte-committed progenitor cells, macrophages, or monocytes of the present invention may be administered at least one month, at least two months, at least three months, at least four months, at least five months, at least six months, at least seven months, at least eight months, at least nine months, at least 10 months, at least 11 months, or at least 12 months prior to a TAA-specific T-cell and/or an immune checkpoint inhibitor.

The HSC, the HPC, myeloid/monocyte-committed progenitor cells, macrophages, or monocytes of the present invention may be administered at least one month, at least two months, at least three months, at least four months, at least five months, at least six months, at least seven months, at least eight months, at least nine months, at least 10 months, at least 11 months, or at least 12 months prior to a TAA-specific T-cell and/or an immune checkpoint inhibitor.

As used herein, the phrase "used in combination with" encompasses administration of the HSCs, HPCs, myeloid/monocyte-committed progenitor cells, macrophages, or monocytes of the present invention sequentially, separately or simultaneously with 1-methyl-tryptophan (1-MT).

The HSC, HPC, myeloid/monocyte-committed progenitor cells, macrophages, or monocytes of the present invention may be administered at least 5 minutes, at least 10 minutes, at least 15 minutes, at least 30 minutes, at least 45 minutes, at least 60 minutes prior to 1-methyl-tryptophan (1-MT).

The HSC, HPC, myeloid/monocyte-committed progenitor cells, macrophages, or monocytes of the present invention may be administered at least 1 hour, at least 2 hours, at least 3 hours, at least 4 hours, at least 5 hours, at least 6 hours, at least 12 hours, at least 24 hours, at least 48 hours, at least 72 hours prior to 1-methyl-tryptophan (1-MT).

The HSC, HPC, myeloid/monocyte-committed progenitor cells, macrophages, or monocytes of the present invention may be administered at least one day, at least two days, at least three days, at least four days, at least five days, at least six days, at least seven days, or at least 14 days prior to 1-methyl-tryptophan (1-MT).

The HSC, the HPC, myeloid/monocyte-committed progenitor cells, macrophages, or monocytes of the present invention may be administered at least one week, at least two weeks, at least three weeks, at least four weeks, at least five weeks, at least six weeks, at least seven weeks, at least eight weeks, at least nine weeks, at least 10 weeks, at least 11 weeks, or at least 12 weeks prior to 1-methyl-tryptophan (1-MT).

The HSC, the HPC, myeloid/monocyte-committed progenitor cells, macrophages, or monocytes of the present invention may be administered at least one month, at least two months, at least three months, at least four months, at least five months, at least six months, at least seven months, at least eight months, at least nine months, at least 10 months, at least 11 months, or at least 12 months prior to 1-methyl-tryptophan (1-MT).

The HSC, the HPC, myeloid/monocyte-committed progenitor cells, macrophages, or monocytes of the present invention may be administered at least one month, at least two months, at least three months, at least four months, at least five months, at least six months, at least seven months, at least eight months, at least nine months, at least 10 months, at least 11 months, or at least 12 months prior to 1-methyl-tryptophan (1-MT).

## PHARMACEUTICAL COMPOSITION

The HSCs, HPCs, myeloid/monocyte-committed progenitor cells, macrophages, monocytes immune checkpoint inhibitors, TAA-specific T-cells, vectors, 1-methyl-tryptophan (1-MT) and combinations thereof for use according to the present invention may be formulated with a pharmaceutically acceptable carrier, diluent or excipient.

## PATIENT

The patient may be a human patient. The patient may be a non-human animal.

The patient may be afflicted with a cancer. Alternatively, the patient may be at risk of developing a cancer.

The patient may have been previously determined to be at risk of developing a cancer. The increased risk may have been determined by genetic screening and/or by reviewing the patient's family history. The patient may have been determined to express one or more genetic markers indicative of an increased risk of developing a cancer.

Suitably, a person skilled in the art will be aware of genetic risk factors (e.g. genetic markers) associated with increased risk of developing a cancer. The skilled person may use any suitable method or technique known in the art to determine whether the subject has an increased risk of developing a cancer.

The subject may have previously received treatment for the cancer. The subject may be in remission from the cancer. The subject may be resistant to chemotherapy.

In some aspects of the present invention, the patient has been previously administered a hematopoietic stem cell (HSC), a hematopoietic progenitor cell (HPC), a myeloid/monocyte-committed progenitor cell, a macrophage or a monocyte of the invention prior to administration of a TAA-specific T-cell and/or an immune checkpoint inhibitor.

In one embodiment, the HSC, the HPC, the myeloid/monocyte-committed progenitor cell, the macrophage or the monocyte of the present invention is administered to the patient at least 6 hours, at least 12 hours, at least 24 hours, at least 48 hours, at least 72 hours prior to administration with a TAA-specific T-cell and/or an immune checkpoint inhibitor.

In one embodiment, the HSC, the HPC, the myeloid/monocyte-committed progenitor cell, the macrophage or the monocyte of the present invention is administered to the patient at least one day, at least two days, at least three days, at least four days, at least five days, at least six days, at least seven days, or at least 14 days prior to administration with a TAA-specific T-cell and/or an immune checkpoint inhibitor.

In one embodiment, the HSC, the HPC, the myeloid/monocyte-committed progenitor cell, the macrophage or the monocyte of the present invention is administered to the patient at least one week, at least two weeks, at least three weeks, at least four weeks, at least five weeks, at least six weeks, at least seven weeks, at least eight weeks, at least nine weeks, at least 10 weeks, at least 11 weeks, or at least 12 weeks prior to administration with a TAA-specific T-cell and/or an immune checkpoint inhibitor.

In one embodiment, the HSC, the HPC, the myeloid/monocyte-committed progenitor cell, the macrophage or the monocyte of the present invention is administered to the patient at least one month, at least two months, at least three months, at least four months, at least five months, at least six months, at least seven months, at least eight months, at least nine months, at least 10 months, at least 11 months, or at least 12 months prior to administration with a TAA-specific T-cell and/or an immune checkpoint inhibitor.

The patient may have been previously administered a hematopoietic stem cell (HSC), a hematopoietic progenitor cell (HPC), a myeloid/monocyte-committed progenitor cell, a macrophage or a monocyte of the invention prior to administration of 1-methyl-tryptophan (1-MT). The HSC, the HPC, the myeloid/monocyte-committed progenitor cell, the macrophage or the monocyte of the present invention may be administered to the patient at least 6 hours, at least 12 hours, at least 24 hours, at least 48 hours, at least 72 hours prior to administration with 1-methyl-tryptophan (1-MT).The HSC, the HPC, the myeloid/monocyte-committed progenitor cell, the macrophage or the monocyte of the present invention may be administered to the patient at least one day, at least two days, at least three days, at least four days, at least five days, at least six days, at least seven days, or at least 14 days prior to administration 1-methyl-tryptophan (1-MT).The HSC, the HPC, the myeloid/monocyte-committed progenitor cell, the macrophage or the monocyte of the present invention may be administered to the patient at least one week, at least two weeks, at least three weeks, at least four weeks, at least five weeks, at least six weeks, at least seven weeks, at least eight weeks, at least nine weeks, at least 10 weeks, at least 11 weeks, or at least 12 weeks prior to administration with 1-methyl-tryptophan (1-MT).The HSC, the HPC, the myeloid/monocyte-committed progenitor cell, the macrophage or the monocyte of the present invention may be administered to the patient at least one month, at least two months, at least three months, at least four months, at least five months, at least six months, at least seven months, at least eight months, at least nine months, at least 10 months, at least 11 months, or at least 12 months prior to administration with 1-methyl-tryptophan (1-MT).Both human and veterinary treatments are within the scope of the present invention.

The terms "comprising", "comprises" and "comprised of" as used herein are synonymous with "including" or "includes"; or "containing" or "contains", and are inclusive or open-ended and do not exclude additional, non-recited members, elements or steps. The terms "comprising", "comprises" and "comprised of' also include the term "consisting of".

The practice of the present invention will employ, unless otherwise indicated, conventional techniques of cell biology, molecular biology, histology, immunology, oncology, which are within the capabilities of a person of ordinary skill in the art. Such techniques are explained in the literature.

See, for example, Sambrook, J., Fritsch, E.F. and Maniatis, T. (1989) Molecular Cloning: A Laboratory Manual, 2nd Edition, Cold Spring Harbor Laboratory Press; Ausubel, F.M. et al. (1995 and periodic supplements) Current Protocols in Molecular Biology, Ch. 9, 13 and 16, John Wiley & Sons; Roe, B., Crabtree, J. and Kahn, A. (1996) DNA Isolation and Sequencing: Essential Techniques, John Wiley & Sons; Polak, J.M. and McGee, J.O'D. (1990) In Situ Hybridization: Principles and Practice, Oxford University Press; Gait, M.J. (1984) Oligonucleotide Synthesis: A Practical Approach, IRL Press; and Lilley, D.M. and Dahlberg, J.E. (1992) Methods in Enzymology: DNA Structures Part A: Synthesis and Physical Analysis of DNA, Academic Press.

Various preferred features and embodiments of the present invention will now be described by way of non-limiting examples.

## EXAMPLES

### Example 1 - Tumor-targeted IFNα boosts T cell-mediated anti-tumor responses in a ALL model

We transplanted C57Bl/6 mice with HSC transduced with either *mTie2*-IFN-mirT LV (IFN mice) or *mTie2*-GFP-mirT LV or Mock-transduced (both used as control mice), to target IFN/GFP expression to the differentiated TIE2+ monocyte progeny, which is highly enriched in tumors[15,16]. As shown previously, reconstitution with *mTie2*-IFN-mirT LV transduced cells results in a functional multi-lineage graft, with no overt side effects[8-10]. We then challenged reconstituted mice with our previously described B-ALL model (Fig. 1a) and found inhibition of leukemia growth in IFN vs. control mice (Fig. 1b,c). Administration of anti-CTLA4 blocking antibody (aCTLA4) had no effects in control mice but further and significantly improved ALL inhibition in IFN mice, suggesting an immune contribution to the observed response in IFN mice. The combination of IFN gene therapy and αCTLA4 significantly improved the survival of the mice (Figure 21). In order to investigate the induction of anti-tumor immunity, we engineered ALL cells with a lentiviral vector (LV) allowing for coordinate expression of the Ovalbumin (OVA) model antigen and the truncated form of the nerve growth factor receptor (NGFR) cell surface marker from a bidirectional promoter (OVA-ALL, Fig. 5a,b).

When injected into immunocompetent C57Bl/6 mice, OVA-ALL showed slower growth kinetics and delayed onset in a fraction of the mice as compared to parental ALL. At necropsy, all mice showed massive BM infiltration by ALL cells, with outgrowth of NGFR negative blasts in the mice with delayed disease (Fig. 5c,d). When OVA ALL cells were injected in immunodeficient NOD-SCID-IL2Rg-/- (NSG) mice we observed comparable tumor growth as the parental cells and no loss of NGFR expression (Fig. 5c,e). Altogether, these results indicate increased immunogenicity of the OVA-ALL variant leading to immune editing and selection of rare un-transduced or silenced (OVA/NGFR negative) ALL clones likely present in the infusion product. No mice, however, survived either challenge. We thus tested the ability of tumor-targeted IFN cell and gene based delivery to boost the anti-tumor immune response against OVA-ALL. Whereas

ALL rapidly expanded in control mice (Fig. 1d-f), there were delayed appearance and accumulation of blasts in the blood, and reduced infiltration in the BM and spleen, of IFN mice. Of note, a fraction of IFN mice showed absence of leukemia in all organs analyzed.

In vitro stimulated purified splenic CD8+ T cells from IFN mice showed induction of specific response against OVA by g-IFN-ELISPOT, with increasing number of responder cells in the mice showing the lowest tumor burden (Fig. 1g). OVA-specific CD8+ T cells were detected by OVA257-264-H-2Kb -pentamer staining in both IFN and control mice, with higher percentages and numbers in the blood and BM of the former group (Fig. 1h-j and Fig. 6a).

Moreover, the CD8+ T cells of control mice did not release IFN-g upon ex-vivo re-stimulation with OVA, as did T cells of IFN mice (Fig. 1k), suggesting dysfunction, consistently with the lack of tumor inhibition in control vs. IFN mice (Fig. 6b-d). Strikingly, depletion of CD8+ T cells in IFN mice abrogated the anti-tumor response (Fig. 1l,m). Overall, these results indicate induction of CTLs able to mount an effective response against a tumor neo-antigen in IFN mice.

We also noted an initial lower tumor burden in CD8-depleted IFN mice as compared to control, suggesting that additional mechanisms beside CD8-mediated control may contribute, at least initially and temporarily, to tumor inhibition (Fig. 6e). Whereas there was no difference in the apoptotic cell fraction and cell cycle distribution of BM and spleen ALL cells between IFN and control mice, (Fig. 7a-d), the proliferation rate, as measured by EdU incorporation, was lower in the BM, but not the spleen, of IFN mice (Fig. 7e,f). A delay in proliferation, possibly triggered directly by IFN, may favor the buildup of tumor specific CTL at effective effector to target ratio to suppress tumor cell growth.

### Example 2 - Adoptively transferred transgenic OT-I T cells only expand and contain leukemia in IFN mice

To investigate the effect of type-I IFN on T cell recruitment and activation, we adoptively transferred naive transgenic OVA-specific T cells (OT-I) in IFN and control mice (Fig. 2a). We adjusted the time of infusion between the 2 groups to infuse OT-I cells at comparable leukemia burden (Fig. 2b and Fig. 8a) and analyzed mice 3 days after. We observed substantially higher numbers of OT-I cells in the spleen and BM of leukemia injected IFN than control mice, in which OT-I cells were retrieved in low numbers similar to non-tumor bearing controls (Fig. 2c). Cytofluorimetric analysis showed that a majority of OT-I cells had up-regulated the activating receptor LAG3, and acquired central memory or effector memory phenotype, in the leukemia-bearing IFN and control mice, with increased activation in the former group.

These findings were in sharp contrast to tumor-free control mice, in which OT-I cells maintained the naive phenotype of the harvest (Fig. 2d and Fig. 8b-d). Leukemia burden was significantly reduced in IFN vs. control mice already at early times after adoptive T cell transfer (Fig. 8e). Whereas nearly all leukemic cells of control mice expressed the NGFR marker, there was an increasing fraction of NGFR negative cells in IFN mice, suggesting selective pressure against OVA-ALL (Fig. 8f).

We then explored the synergy of IFN cell therapy and the adoptive transfer of OT-I T cells in promoting survival (Fig. 2e). Whereas infusion of OT-I T cells resulted in 20% mice survival vs. none in the control group, the combined treatment significantly improved survival to 67% (Fig. 2f). Longitudinal analysis of IFN + OT-I mice revealed OT-I expansion, peaking at 6 days upon infusion, accompanied by transient LAG3 up-regulation and OVA-ALL clearance (Fig. 2g,h and Fig. 9a). Of note, those IFN + OT-I mice that succumbed to the disease (shown in black in Fig. 2g,h and Fig. 9a) showed the outgrowth of NGFR negative ALL cells in the blood and BM, thus confirming that OVA-expressing leukemic cells had been eradicated by the infused OT-I cells (Fig. 9a,c). Conversely, OT-I cells in most control mice failed to expand and showed constitutive high levels of LAG3 expression, a sign of T cell exhaustion Fig. 2g,h). Accordingly, infused OT-I cells failed to eradicate OVA-expressing B-ALL cells in these mice, as shown by an initial but transient decrease in circulating NGFR-expressing leukemic cells followed by their outgrowth in the blood and BM (Fig. 9b,d).

At necropsy, whereas a good fraction of OT-I T cells in the BM, spleen and lymph nodes of IFN + OT-I mice remained as memory pool and had down-regulated the PD1 inhibitory marker, the majority of OT-I T cells in CTRL + OT-I mice had features of exhausted effectors, as they all expressed the PD1 marker (Fig. 9e,f). As expected, OT-I T cells infused in non-tumor injected control mice did not expand and did not up-regulate Lag3 expression (Fig. 2g,h). Overall, these data show that, while OT-I cells underwent robust activation and expansion in IFN mice, leading to tumor clearance, these cells were hypo-functional in control mice, failed to expand and showed phenotypic evidence of exhaustion.

**Example 3 - Primed immune gene signature towards M1 and Th1 responses in IFN mice; and IFN gene therapy offsets leukemia-induced changes in the tumor microenvironment (TME) and imposes an immunostimulatory program**

To study whether our IFN gene delivery strategy alters the leukemia microenvironment favoring the induction of effective immune responses, we performed multiplexed measurement of gene expression on a 750 gene panel used for cancer immune profiling on the spleen and BM of control and IFN mice, before and after leukemia challenge. Nearly all significantly up-regulated genes in both tissues of IFN vs. control mice were IFN stimulated genes (ISG) and, in the spleen, T-cell activation, antigen processing, macrophage/DC activation, NK activation and innate immunity genes confirming that our treatment imposed an IFN / Th1 response signature in these tissues. These changes remained evident after tumor challenge and were accompanied by down-regulation of leukemia-associated genes (Fig. 3a). We then purified splenic CD4+ T cells to interrogate selected genes, and showed significant up-regulation of Tbx21, encoding the Th1 transcription factor TBET, and no changes in prototypical Th2 and Treg genes in IFN vs. control tumor-bearing mice. We also observed up-regulation of the Il17 gene in IFN mice but this change was not accompanied with up-regulation of the other prototypical Il22 and Rorc Th17 genes (Fig. 3b and Fig. 10a).

We then characterized the phenotype of myeloid cells in the blood, spleen and BM. We found increased percentage of classical (Ly6C+Ly6Glow) and reduced percentage of non-classical (Ly6C-Ly6G-) monocytes in the blood of IFN vs. control mice, and such differences were further enhanced upon leukemia challenge (Fig. 3c and Fig. 11a). We also found increased circulating granulocytes (Ly6Cint Ly6G+) in IFN mice. Leukemia growth in the spleen of control mice was accompanied by expansion of immature myeloid cells (CD11cint MHCII-F4/80-) and increased percentage of MHC-II negative M2 macrophages, whereas none of these changes were seen in IFN mice (Fig. 11b-d). Leukemia-associated changes were also absent in the BM myeloid cells of IFN mice, which showed instead an increase in DCs and in the fraction of DC presenting the immune-dominant $OVA_{257-264}$ peptide on MHC-I (Fig. 12a,b and Figure 18). Similarly, the changes in NK and NKT cells seen in the spleen and BM of leukemic mice - including decreased expression of the activating NKG2D receptor - were absent in IFN mice (Fig. 13a-e).

RNA-sequencing (RNA-Seq) analyses revealed leukemia-induced transcriptional changes in macrophages, which were substantially counteracted by IFN gene therapy (Figure 22a,b). Spleen macrophages from ALL vs. control mice up-regulated genes encoding for the immunosuppressive cytokine IL-10, the inhibitory immune checkpoint PD-1 as well as genes linked to cell division and response to immune stimuli gene ontology (GO) terms. Down-regulated genes were enriched in GO terms related to fatty acid metabolism, leukocyte activation and antigen presentation (Figure 19a,c). IFN gene therapy in ALL mice elicited an immunostimulatory program characterized by up-regulation of IFN-Stimulated Genes (ISGs) enriched in defense response, leukocyte migration and response to interferon GO terms, and abrogated leukemia-induced up-regulation of Il10 and down-regulation of MHC II genes (Figure 19b-d). IFN gene therapy in ALL mice induced ISGs at levels higher than those triggered in controls, (Figure 19d and Figure 22a), and the transcriptomes of macrophages from control and IFN tumor-free mice showed high correlation, while they were clearly distinct from the ALL and IFN+ALL groups (Figure 22b). These data confirm and extend previous reports that our monocyte-mediated gene therapy preferentially targets IFN to the TME (De Palma, M. et al. (2008) Cancer Cell 14: 299-311; Escobar, G. et al. (2014) Sci. Transl. Med. 6: 217ra3; Catarinella, M. et al. (2016) EMBO Mol. Med. 8: 155-170).

To dissect the impact of the leukemia and IFN gene therapy on the TME in a more unbiased manner, we performed single-cell (sc)RNA-Seq on CD11b+ cells isolated from the spleen of control and tumor-bearing mice, treated or not with IFN gene therapy. Using a droplet-based approach (Zheng, G.X. et al. (2017) Nat. Commun. 8: 14049), we generated scRNA-Seq data from 10,821 cells, detecting a mean of 1,338 genes/cell. Graph-based clustering and gene signature analyses using published datasets (Lavin, Y. et al. (2014) Cell 159: 1312-1326; Varol, D. et al. (2017) Immunity

46: 1030-1044; ImmGen) identified 11 clusters corresponding to monocytes (cl. 1-3), neutrophils (cl. 4-6), dendritic cells (cl. 7), macrophages (cl. 8), natural killer and T cells (cl. 9), mast cells (cl. 10) and B cells (cl. 11). Heterogeneity was observed within the monocyte and neutrophil populations, encompassing non-classical (cl. 1) and classical (cl. 2) monocytes, a cluster co-expressing monocyte and neutrophil genes (cl. 3) (Yáñez, A. et al. (2017) Immunity 47: 890-902) and neutrophil maturation intermediates (Figure 19e and Figure 23a,b). Leukemia had a major impact on the transcriptional landscape of non-classical monocytes (Figure 19f), which were expanded in the spleen of tumor-bearing mice (see Figure 11c). The other myeloid cell populations, including macrophages and DCs, showed comparatively less leukemia-induced alterations (Figure 24). Tumor-associated non-classical monocytes up-regulated genes enriched in GO terms such as complement activation and negative regulation of inflammation, while they down-regulated genes linked to antigen processing and presentation (Figure 19g). IFN gene therapy imposed an ISG-driven immunostimulatory program to non-classical monocytes from ALL mice, as evidenced by up-regulation of genes enriched in GO terms related to defense and innate immune response, as well as MHC II genes (Figure 19g,h). Transcriptional reprogramming of the TME by IFN gene therapy was less effective in non-classical monocytes from mice that did not respond to IFN gene therapy (Figure 25a), as revealed by graph-based clustering and differential gene expression (Figure 19f,h). Subclustering of scRNA-Seq data from non-classical monocytes identified four major subclusters (1A to 1D; Figure 25b). Subcluster 1A was comprised of cells from disease-free mice from both control and IFN mice, whereas the other three subclusters largely overlapped with cells from IFN+ALL responder (1B), IFN+ALL non responder (1C), and ALL (1D). Minimum-spanning tree (MST) analyses revealed a trajectory from 1A to 1D, confirming partial vs. effective reprogramming in cells from non responder vs. responder IFN mice (Figure 19i).

Unexpectedly, we also found increased numbers of FOXP3+CD25+CD4+ T cells in the BM of tumor-injected IFN mice (Fig. 12c,d). Overall, these changes are consistent with priming towards activation of M1 and Th1 responses in IFN mice, which may favor, upon leukemia challenge, the induction of protective immune response.

**Example 4 - IFN treatment induces durable anti-tumor responses targeting multiple tumor antigens**

We then investigated whether tumor-targeted IFNα treatment could also promote long-term durable responses in the mice. Strikingly, an average of 24% (average of 5 different experiments, n=11, 14, 8, 16, 12) of IFN mice survived long-term and were effectively cured of the disease, whereas only 2% of control mice (n=13, 14, 8, 16, 13) survived to it (one representative experiment shown in Fig. 4a and Fig. 14a). Of note, by stratifying the mice based on survival time, we found that mice euthanized early after tumor injection showed low percentage of circulating OVA-specific T cells and no appearance of NGFR negative ALL (Fig. 14b). Conversely, mice euthanized at later time points showed induction of variable percentages of circulating OVA-specific T cells and the appearance of NGFR-negative ALL clones (Fig. 14b). These results suggest that mice succumbing to the disease either failed to mount an immune response and died very early, or mounted anti-OVA responses but ultimately died due to the failure of these cells to protect the mice, either because of exhaustion or the emergence of immune-selected OVA negative ALL clones. Long-term survivors showed stable circulating OVA-specific T cells and, when re-challenged with OVA-ALL, remained disease-free (Fig. 4b and Fig. 14c). Tumor clearance was associated with expansion of circulating OVA-specific T cells, suggesting that development of memory T cell responses plays a key role in protecting mice from subsequent tumor challenge (Fig. 14d). Strikingly, long-term surviving IFN mice efficiently cleared both OVA-expressing and the parental, OVA-negative ALL cells, when re-challenged with a 1 to 1 ratio of these cells or parental cells alone, suggesting that spreading of the response towards additional tumor-associated antigens (TAA) had occurred, and might be required to achieve long-term durable protection (Fig. 4c,d and Fig. 14e).

**Example 5 - Combination of IFN treatment and CTLA-4 blockade enhances survival to leukemia**

We then investigated the combination of CTLA-4 blockade therapy with tumor-targeted IFNα treatment in our experimental OVA-ALL model (Fig. 4e). Whereas all treatment groups - IFN gene therapy or treatment with aCTLA4 alone or their combination - significantly increased mouse survival vs. control, the combination therapy was more effective (IFN 21%; aCTLA4 20%; CTRL+isotype control antibody 7%; IFN+aCTLA4 36%; Fig. 4f and Fig. 15a), as confirmed in a second experiment (IFN+αCTLA4 30% vs αCTLA4 8%; Fig. 4g and Fig. 15b). IFN or aCTLA-4 treatment increased the percentage of OVA207 specific T cells in PB, which was further increased in the combination group (Fig. 16b). Accordingly, immune selection of NGFR-negative ALL was more evident in the IFN+aCTLA4 and aCTLA4 groups, although it was not consistently accompanied by delayed disease course (Fig. 16a), suggesting that immune response to a single neo-antigen may not afford tumor protection. Beside the dominant OVA antigen, our ALL model also express OFP (which is co-expressed with miR-126) and the prokaryotic trans-activator tTA (which up-regulates miR-126 expression) as potential neo-antigens that help driving the transformed phenotype (see Fig. 1a). We stimulated peripheral blood mononuclear cells (PBMC) of long-term surviving mice (from experiment shown in Fig. 4f) with target cells transduced with LV expressing tTA, OFP, NGFR or OVA, and measured γ-IFN production by ELISPOT. The majority of these

mice showed reactivity against one or more neo-antigens in addition to OVA, with the strongest response observed against tTA and the weakest against NGFR (Fig. 4h).

These findings indicate that spreading of the immune response towards additional neo-antigens occurred in the majority of long term surviving mice. We then re-challenged these mice with a 1:1 ratio of OVA-positive and negative ALL and found that the majority of them survived the re-challenge. Intriguingly, analysis of individual mice indicated that those failing to survive had failed to generate a broad response against tumor antigens and/or succumbed to the selection of ALL clones lacking more than one neo-antigens (Supplementary Table 1). As a further indication of an adaptive immune response underlying the survival of the mice, we compared the TCR-β complementary determining region (CDR) repertoire of PBMCs before and after the leukemia challenges. Both the productive clonality (a measure of diversity ranging from 0=polyclonal to 1=monoclonal within each mouse) and the similarity of the repertoire among different mice increased upon leukemia re-challenge, indicating the expansion of tumor-reactive T cell clones against a common set of TAAs among the surviving mice (Fig. 4i and Fig. 16c).

To further assess whether generation of responses towards multiple neo-antigens is a predictor of long-term survival, we performed a new experiment and collected PBMCs early after OVA-ALL challenge to investigate T cell reactivity against all known TAAs by IFNg-ELISPOT assay. Mice showing an anti-tumor repertoire encompassing multiple TAAs had a much higher likelihood of long-term survival than mice responding to a single or no TAA, which all died of the disease (Fig. 4j,k).

Altogether, these data show that both IFN gene therapy and CTLA4 blockade increase T-cell priming and broaden the repertoire of response against tumor neo-antigens, and that these responses can be additive and protect long-term from the tumor.

**Example 6 - IFN gene therapy boosts activation and expansion of adoptively transferred CAR19-transduced T cells and enhances survival**

To further explore the synergy between IFN gene therapy and adoptive T-cell transfer in a clinically relevant model, we generated T cells expressing a previously described second generation (2G) CAR targeting the mouse CD19 (CART19 cells) and incorporating the CD28 endocostimulatory domain (Kochenderfer, J.N. et al. (2010) Blood 116: 3875-3886), and treated mice injected with the parental (OVA-negative) ALL. For CAR gene transfer we exploited LV that coordinately express the CAR and the NGFR marker from a bidirectional promoter (Casucci, M. et al. (2013) Blood 122: 3461-3472) (Figure 26a,b). To allow CART19 cells engraftment (Davila, M.L. et al. (2013) PLoS One 8: 1-14), mice were conditioned with cyclophosphamide prior to infusion (Figure 20a). Whereas CART19 cells alone had hardly any impact on the rapidly growing ALL in control mice in our experimental conditions, they significantly inhibited leukemia burden and depleted normal B cells in IFN mice (Figure 20b and Figure 26c). Reminiscent of the finding with OT-I T cells, CART19 cells revealed early and transient LAG3 and PD1 up-regulation in IFN but not control mice, reaching the highest peak in a long-term IFN survivor (Figure 20c and Figure 26d, IFN long-term survivor shown in green). Intriguingly, NGFR expression was also upregulated on CART19 cells of IFN mice, likely reflecting increased activity of the phosphoglycerate kinase promoter (PGK) in response to higher metabolic activation of CART cells in IFN mice (Figure 26e,f). Since in our LV NGFR and CAR19 expression are co-regulated by the PGK promoter, higher expression of the latter may have also favored more efficient CD19+ ALL killing in IFN mice. We also tested an improved CD19 CAR version (iCAR19), which contains inactivating mutations in the first and third CD3zeta ITAM domains and was reported to improve killing efficiency and increase T cell viability as compared to the standard CAR19 (Kochenderfer, J.N. et al. (2010) Blood 116: 3875-3886). We treated control or IFN mice with T-cells expressing either CAR19 or control T-cells at low or high leukemia burden (Figure 20d and Figure 27a,b). Whereas CART19 and iCART19 cells had detectable but not significant effect on tumor burden in control mice, they significantly inhibited ALL in IFN mice in either early and late intervention trials (Figure 20e). We also confirmed early LAG3 up-regulation in both CART19 cells from IFN mice (Figure 20f). Longitudinal analyses revealed peaks of iCART19 expansion in IFN mice concomitant to ALL growth inhibition or clearance, and early up-regulation of NGFR/CAR19 expression in IFN mice (Figure 27c,d). Overall, a significant fraction of IFN mice treated with CART19 cells were still alive at the latest follow-up (Figure 20g).

## MATERIALS AND METHODS

### Experimental design

Sample size was chosen according to previous experience with experimental models and assays. No sample or animal was excluded from the analyses. Mice were randomly assigned to each experimental group. Investigators were not blinded.

**Plasmid construction and LV production**

The mTie2-IFN-mirT, mTie2-GFP-mirT, PGK-OFP, PGK-tTA LV were previously described[9,10,14]. The NGFR-OVA transfer BdLV was generated by cloning the OVA cDNA (Agel - Sall) amplified from the PGK-OVA LV37 by PCR in place of the GFP cDNA (Agel - Sall) in the NGFR-GFP BdLV38 . The primers used were the followings: primer Fw: 5'-CGACCGGTCCACAAAGACAGCACCATGACA; primer Rv: 5'-ATTGTCGACTTAAGGGGAAACACATCTGCCAAAGA The NGFR-CD20 transfer BdLV was generated by cloning the codon optimized human CD20 cDNA from a synthetized plasmid (Kpnl blunt - Sall) in place of the GFP cDNA (Agel blunt - Sall) in the NGFR-GFP BdLV. The NGFR-CAR19 and NGFR-iCAR19 transfer BdLVs were generated by cloning the CAR19 and inactive CAR19 sequences previously described (Kochenderfer, J.N. et al. (2010) Blood 116: 3875-3886) by PCR in place of the GFP cDNA (Agel - Sall) in the NGFR-GFP BdLV using the following primers: primer Rv (inactive CAR19): 5'-AAACAGCTCCTCGAGTTATCTAGGGGCCA; primer Rv (CAR19): 5'-AAACAGCTCCCTCGAGTCATCTAGGGGCCAGT; primer Fw (CAR19 and inactive CAR19): 5'-AACACCGGTGTACCGAATTCATGGCGTG. Concentrated VSV-G-pseudotyped LVs were produced and titered as previously described (Follenzi, A. et al. (2000) Nat. Genet. 25: 217-222).

Mice

C57Bl/6 Ly45.2 and Ly45.1 mice were purchased from Charles River Laboratory. C57Bl/6 Ly45.1/Ly45.2 were obtained by crossing C57Bl/6 Ly45.2 and C57Bl/6 Ly45.1 mice in the San Raffaele Scientific Institute animal research facility and used as donors for HPC transplant. Transgenic OT-I C57Bl/6 Ly45.2 mice were maintained as colony at the San Raffaele Scientific Institute animal research facility. All animal procedures were performed according to protocols approved by the Animal Care and Use Committee of the San Raffaele Scientific Institute (IACUC 600) and communicated to the Ministry of Health and local authorities according to the Italian law.

**Hematopoietic stem progenitor cell (HSPC) transplantation**

*HSPC transplantation in C57Bl/6 mice*

Six-week-old C57Bl/6 Ly45.2/Ly45.1 mice were euthanized with CO2 and BM was harvested by flushing the femurs and tibias. Lineage-negative cells enriched in HS/PCs were isolated from total BM using an immuno-magnetic column-based cell purification system (lineage cell depletion kit mouse, Miltenyi, #130-090-858). $10^6$ lineage negative cells/ml were then pre-stimulated for 3-4 hr in a serum-free StemSpan medium (StemCell Technologies) containing a cocktail of cytokines (20 ng/ml IL-3, 100 ng/ml SCF, 100 ng/ml FLT-3L, 50 ng/ml TPO all from Peprotech) and then transduced with the indicated lentiviral vectors at a dose of $10^8$ transducing units/ml for 12 hr in the same cytokine-containing medium. After transduction, cells were washed and $10^6$ cells were infused into the tail vein of lethally irradiated 6-week-old C57Bl/6 Ly45.1 females. Radiation doses were 935 cGy split in two doses. Transduced cells were also cultured in vitro for 15 days in IMDM supplemented with 10% FBS, SCF (100 ng/ml), FLT3L (100ng/ml), penicillin (100 IU/ml), streptomycin (100ug/ml) and 2% glutamine and then tested together with the PBMC cells isolated from transplanted mice at stable hematopoietic reconstitution (6 weeks) to quantify the VCN by qPCR as previously described (De Palma et al., Blood 2005).

**Tumor studies**

*Mouse OVA-ALL*

The OVA-ALL sub-clone was generated by transducing the parental ALL clone #1114 with the NGFR-OVA BdLV. Briefly, ALL were kept in culture at a concentration of $2\times10^6$ cell/ml in Stem Span supplemented with 10% FBS, penicillin (100 IU/ml), streptomycin (100ug/ml), 2% glutamine, IL3 (20ng/ml), SCF (100ng/ml), FLT3L (100ng/ml), TPO (50ng/ml) and transduced at 2x107 TU/ml with the NGFR-OVA BdLV. After 6 hours of LV exposure, transduced ALL were washed and intravenously injected in sub-lethally irradiated (450 cGy) recipient C57Bl/6 Ly45.2 mice. Leukemic mice were sacrificed 14 days later, when ALL reached 90% of total PB cells, and BM was harvested by flushing the femurs and tibias.

NGFR+ transduced cells were isolated by immune-magnetic cell labeling (CD271 MicroBead Kit, Miltenyi, #130-099-023). Purity of selected ALL was determined by flow cytometer analysis by labeling cells with an anti NGFR antibody and was estimated to be 98% (NGFR+ ALL; see Fig. 5a). The OVA-ALL batch was then frozen in multiple aliquots, stored in liquid nitrogen and used for all experiments. For tumor challenge mice were intravenously injected with a dose of $10^5$ OVA-ALL or $3\times10^4$ parental ALL resuspended in 200$\mu$l of phosphate buffer saline (PBS). For re-challenge experiments we mixed OVA-ALL and parental (OVA-negative) ALL at a ratio of 1:1 (105 total ALL). The

composition of the infusion product was verified by flow cytometer analysis prior to in vivo injection by labeling mixed cells with an anti NGFR antibody. Tumor growth kinetic was determined by cytofluorimetric analysis on periodic PB sampling. For survival studies mice were daily inspected and when showing signs of suffering were evaluated for disease status and subsequently euthanized and BM and spleen were collected in sterile conditions for further analysis. For CTLA-4 blocking experiments, a mouse anti-mouse CTLA4 blocking monoclonal antibody (mAb clone 9D9 BioXCell, #BE0164) or the mouse isotype control antibody (mAb clone MCP-11 BioXCell, #BE0086) were administered intra-peritoneally from day 3 upon parental ALL or OVA-ALL injection at an initial dose of 200 μg/mouse and followed by doses of 100 μg/mouse every 3-4 days for a total of five antibody infusions.

### *In vivo* CD8 T cell depletion

For *in vivo* T cell depletion, an anti CD8 depleting monoclonal antibody (mAb 53-6.72 BioXCell, #BE0004-1) was administered intra-peritoneally to the mice at a dose of 200ug/mouse one day before OVA-ALL injection and then every 3 days for the entire duration of the experiment.

### Adoptive OT-I T cell transfer

For adoptive T cell experiments, OT-I T cells were purified from the spleen of 8 week-old transgenic female OT-I C57Bl/6 Ly45.2 mice by immune-magnetic selection (CD8+ T cell isolation kit, Miltenyi, #130-104-075). Purity of selected OT-I T cells was evaluated by cytofluorimetric analysis using a panel of anti CD8, CD4 and CD3 antibodies. $1 \times 10^6$ naïve OT-I T cells were intravenously injected in transplanted IFN or CTRL mice at the indicated time upon OVA-ALL injection.

### Generation of CART19 cells

T cells were first purified from the spleen of 8 weeks-old female C57Bl/6 CD45.2+ mice by immune-magnetic selection (Pan T cell isolation kit, Miltenyi, # 130-095-130) and subsequently activated with anti-CD3/CD28 Dyna beads (ThermoFisher # 11452D), according to manufacturer's instructions. T cells were cultured in RPMI supplemented with 10%FBS, penicillin (100U/ml), streptomycin (100ug/ml), 1% glutamine, IL2 (30u/ml), IL7 (5ng/ml), IL15 (5ng/ml), Na Pyruvate (1mM), Hepes (20mM), NEAA (1uM) and Beta-Mercaptoethanol (0.05mM). One day after activation, T cells were transduced at a concentration of $1 \times 10^6$ cells/ml with $10^8$TU/ml of the NGFR-CAR19 or NGFR-iCAR19 BdLVs. 12 hours after LV exposure T cells were washed and expanded in culture for 8 days prior to infusion in mice at a dose of $7 \times 10^6$ (experiment from Figure 20b) and 107 (experiment from Figure 20e) NGFR+ T cells.

### *In vivo* Edu proliferation assay

In vivo proliferation assays were performed using 5-ethynyl-2'deoxyuridine (EdU, Invitrogen), a thymidine analogue used in alternative to BrdU. EdU was dissolved in sterile 1x PBS at a concentration of 10 mg/ml. Mice were injected i.p. with 100μg of EdU 24h before analysis. BM and spleen were harvested and processed according to manufacturer's instructions (Click-iT® EdU Flow Cytometry Assay Kit, Invitrogen, #C10636), and percentages of EdU incorporation into leukemic cell were measured by flow cytometric analysis.

Cell culture and transduction

The mouse EL4 C57Bl/6-derived lymphoma cell line was maintained in IMDM supplemented with 10% FBS, penicillin (100 IU/ml), streptomycin (100 μg/ml) and 2% glutamine. Transduction was performed at a concentration of 106 cell/ml with a dose of $10^8$ TU/ml of the indicated vector stock. Cells were incubated for 12 hours and then washed to remove the vector. OFP on transduced cells was assessed upon 7 days of culture by flow cytometric analysis. NGFR and CD20 expression were evaluated by staining the cells with an anti-NGFR or anti-CD20 antibody at 7 days upon transduction (see flow cytometry section). The VCN of transduced cells was determined by real-time qPCR upon 15 days of culture as previously described (De Palma et al., Blood 2005).

### Flow cytometry

All cytometric analyses were performed using the FACSCanto II and LSRFortessa instruments (BD Bioscience) and analysed with the FlowJo software (v. 9.3, Tree Star Inc.).

*Cultured cells*

EL4 cells were washed and resuspended in PBS containing 2% FBS. For immunostaining, cells were incubated with anti-mouse Fc receptor blocking antibodies for 15 min at 4 °C and then stained for 20 min at 4 °C with the anti NGFR or anti CD20 antibodies (for antibodies see Supplementary Table 1). To exclude dead cells from the analysis, cells were washed and resuspended in PBS containing 10 ng/ml 7-aminoactinomycin D (7-AAD).

*Peripheral blood*

For each mouse, 250 μl of peripheral blood were added to 10 μL of PBS containing 45 mg/mL EDTA. For immunostaining a known volume of whole blood (100 μl) was first incubated with anti-mouse FcγIII/II receptor (Cd16/Cd32) blocking antibodies for 10 min at room temperature and then incubated in the presence of monoclonal antibodies (for antibodies see Supplementary Table 1) for 15 min at room temperature. Erythrocytes were removed by lyses with the TQ-Prep workstation (Beckman-Coulter) in the presence of an equal volume of FBS (100 μl) to protect white blood cells. For quantitative flow cytometry we first stained and lysed a known volume of blood (100 μl), as described above, and we then added a fixed volume (50 μl) of Flow-count Fluorospheres, with a known concentration Beckman Coulter, #7547053). To determine the absolute number of cells we used theformula: Absolute Count (cell/μl) = [Total number of cells counted/(Total number of Fluorospheres counted × 2)] × Concentration of the Flow-count Fluorospheres. For OVA- specific pentamer staining, whole blood was first lysed with $H_2O$. $1\times10^6$ PBMC were then resuspended in 50ul of PBS containing 2mM EDTA and 0.5% bovine serum albumine (BSA) and incubated with anti-mouse FcyIII/II receptor (Cd16/Cd32) blocking antibodies for 10 min at room temperature and then with OVA-specific pentamer reagent (0.5μg/$1\times10^6$ cells) and monoclonal antibodies (for antibodies see Supplementary Table 1) for other 10 min at room temperature. To exclude dead cells from the analysis, cells were washed and resuspended in PBS containing 2% FBS and 10 ng/ml of 7-aminoactinomycin D (7-AAD). For Treg cells staining in PB we first performed surface staining on 50μl of whole blood (for antibodies see Supplementary Table 1), as described above. Stained whole blood was then washed and resuspended in 100ul of the Fixation/Permeabiliation solution (eBioscience, #00-5523-00) and incubated for 20 min at room temperature, washed and resuspended in 50μl of Permeabilization solution containing the anti-mouse FoxP3 or isotype control antibodies and incubated for 20 min at room temperature.

*Bone marrow*

BM cells were obtained by flushing the femurs in PBS 2% FBS solution and by passing cell suspension through a 40 μm nylon filter. Cells (1x106 - 3x106 cells) were washed, resuspended in 100 μl of PBS containing 2mM EDTA and 0.5% bovine BSA, and incubated with anti-mouse FcyIII/II receptor (Cd16/Cd32) blocking antibodies for 15 min at 4°C. Staining was then performed with monoclonal antibodies (for antibodies see Supplementary Table 1) for 20 min at 4°C. For OVA-specific pentamer staining, $1\times10^6$ BM cells were stained as described above for PB. For all intracellular staining and for some surface staining the LIVE/DEAD Fixable Dead Cell Staining (ThermoFisher, #L34959) was used to discriminate alive and dead cells according to manufacturer's instructions. For Treg staining in BM, we first performed surface staining on $1\times10^6$ BM cells, as described above for PB.

*Spleen*

Spleens were first smashed and the resulting cell suspension was passed through 40 μm nylon filter. Erythrocytes were lysed by H20 and the obtained cells suspension was further passed through 40 μm nylon filter and washed in cold PBS containing 2mM EDTA and 0.5% BSA. Cells were processed and immunostained as described above for BM.

*Cell cycle staining*

BM cells and splenocytes from OVA-ALL injected mice were harvested as described above. Cells were then stained for surface markers, washed and fixed using BD Cytofix buffer (BD #554655), washed and permeabilized with BD Perm 2 (BD #347692), washed and stained with PerCP-Cy5.5- or APC-conjugated Ki67 antibody and finally resuspended in BD Cytofix buffer with Hoechst at 1 μg/mL. Cells were then analyzed on a BD LSRFortessa machine with UV laser.

*Cell apoptosis staining*

BM cells and splenocytes from OVA-ALL injected mice were harvested as descrived above. Cells were then stained with surface markers as described above. Stained cells were then washed twice with the cell staining buffer

(BioLegend, #420201) and resuspended in the Annexin V Binding Buffer (Biolegend #422201) and incubated with 5ul of Pacific blue conjugated Annexin V and 10ul of 7-AAD (Biolegend, #420403/420404) for 15 minutes at room temperature. 400ul of Annexin V Binding Buffer is then added to each sample and percentages of apoptotic/dead cells in OVA-ALL were measured by flow cytometric analysis within 30 min from staining.

**ELISPOT assay**

The g-IFN ELISPOT was performed in 96-well flat-bottomed plate (Millipore) coated with 5μg/ml of anti mouse IFN-g primary antibody (BD, #554430). Splenic CD8+ effector T cells were first purified by immune-magnetic cell labeling (CD8 MicroBead Kit, Miltenyi, #130-049-401) and purity was evaluated by cytofluorimetric analysis using a panel of anti CD8, CD4 and CD3 antibodies. Effector T cells were subsequently plated at a final concentration of 105 or $2\times10^5$ cells/well in presence of irradiated (6000 cGy) $10^5$ EL4 target cells. For some experiments, we also stimulated effector T cells with 2.5 μg/ml of Concanavalin A, as control. After 46 hours of incubation at 37 °C / 5%CO2, cells were washed and incubated with the biotinylated anti-mouse IFN-g antibody (clone XMG 1.2, BD #554410, 1μg/ml) for two hours. Plates were then washed four times with PBS-Tween followed by 3 washes with PBS and then the avidin-POD (Roche #11089153001) was added (1:5000 diluted in PBS) for 1 hour. Plates were then washed four times with PBS-Tween followed by 3 washes with PBS and then 50μl of the 3-amino-9-ethylcarbazole substrate (AEC, SIGMA #A6926/50tab) was added to each well for 15-20 minutes. The reaction was stopped by the addition of tap water, and the spots were quantified with ELI-Expert.Elispot-Reader and analyzed by Eli.Analyze Version 5.1 (A.EL.VIS). When using total PBMC as effector cells, total blood was first lysed with H2O to eliminate erythrocytes, recovered PBMC were counted and plated at a final concentration of $10^5$ cells/well.

**RNA extraction, qPCR and gene expression analysis**

RNA extraction from BM cells, splenocytes and purified splenic CD4+ T cells was performed using the RNeasy Plus mini Kit (Qiagen). Cells were lysed in Buffer RLT plus, supplemented with beta-mercaptoethanol. The lysate was then passed through a 20-gauge needle. RNA was then extracted according to manufacturer's instructions. The extracted mRNAs were retro-transcribed using the SuperScript Vilo kit (11754250; Invitrogen). All Q-PCR analyses on single genes were done using TaqMan probes from Applied Biosystems (see below). QPCR was run for 40 cycles using the Viia 7 instrument while the Viia 7 software was then used to extract the raw data (Ct). To determine gene expression, the difference (ΔCt) between the threshold cycle (Ct) of each gene and that of the reference gene was calculated. The lower the ΔCt, the higher the gene expression level. To obtain relative quantification values, we then calculated the fold-change expression of the gene of interest over its expression in the reference sample, by the formula $2^{-\Delta\Delta Ct}$. The p values are calculated based on Mann-Whitney of the mean $2^{-\Delta\Delta Ct}$ values for each gene between the two groups.

The following Taqman probes were used on purified splenic mouse CD4+ T cells: *Il10* Mm00439616_m1), *Il17* (Mm00439618_m1), *Ifn-g* (Mm01168134_m1), *Il2* Mm00434256_m1), *Tbx21* (TBET) (Mm00450960_m1), *Rorc* (Mm01261022_m1), *Foxp3* Mm00475156_m1), Gata3 (Mm00484683_m1), *Il22* (Mm00444241_m1), *Il4* Mm00445259_m1).

**TCR sequencing**

Input DNA was obtained from PBMC of IFN and CTRL mice before tumor injection (T0), 30 days upon OVA-ALL injection in tumor-free long-term surviving mice (T1) and 4 days upon re-challenge with OVA-ALL and parental ALL mixed at 1:1 ratio (T2). TCRβ chain sequencing was performed at Adaptive Biotechnologies using the ImmunoSEQ platform with primers specific for all 54 known expressed Vβ and all 13 Jβ regions. Each unique CDR template at the aminoacid level was quantified in counts per million (cpm). Clonality was evaluated as 1 - Pielou's evenness[39] :

$$C = 1 - \frac{H'}{H}$$

Where *H'* is the entropy of a sample, calculated on template counts higher than 0 (*i.e.* the set of all observable template in a mouse) and *H* is the maximal theoretical entropy for a mouse, defined by

$$H = \ln(S)$$

Where *S* is the number of distinct templates in a mouse. Similarity was evaluated as the 1 - Bray-Curtis distance

between a sample s and the mean of template counts at time t.

$$S_t = 1 - \frac{\sum_i |s_{i,t} - \overline{s_{i,t}}|}{\sum_i |s_{i,t} + \overline{s_{i,t}}|}$$

This measure is equivalent to Sorensen-Dice similarity index and it was evaluated including all *i* observed templates at time *t.*

**Bulk RNA-Sequencing**

RNA was extracted from 10,000-50,000 sorted cells using the ReliaPrep RNA MiniPrep System (Promega) and RNA-Seq libraries were prepared with the SMART-Seq2 protocol (Picelli, S. et al. (2014) Nat. Protoc. 9: 171-181), with minor modification. Briefly, RNA (1-5 ng) was reverse transcribed using custom oligodT and template-switching LNA oligos (sequences), followed by PCR amplification and clean-up (Ampure XP beads, Beckman Coulter). The resulting cDNA (0,5-1 ng) was tagmented at 55°C for 30 minutes and final RNA-Seq libraries generated using reagents from the Nextera XT DNA Library Prep Kit (Illumina). Sequencing was performed on a NextSeq 500 machine (Illumina, San Diego, CA) using the NextSeq 500/550 High Output v2 kit (75 cycles).

**Single-cell RNA-Sequencing**

Droplet-based digital 3' end scRNA-Seq was performed on a Chromium Single-Cell Controller (10X Genomics, Pleasanton, CA) using the Chromium Single Cell 3' Reagent Kit v2 according to the manufacturer's instructions. Briefly, suspended single cells were partitioned in Gel Beads in Emulsion (GEMs) and lysed, followed by RNA barcoding, reverse transcription and PCR amplification (12-14 cycles). Sequencing-ready scRNA-Seq were prepared according to the manufacturer's instructions, checked and quantified on 2100 Bioanalyzer (Agilent Genomics, Santa Clara, CA) and Qubit 3.0 (Invitrogen, Carlsbad, CA) instruments. Sequencing was performed on a NextSeq 500 machine (Illumina, San Diego, CA) using the NextSeq 500/550 High Output v2 kit (75 cycles).

**Computational Methods**

Raw reads were processed and aligned to the ENSEMBL mm10 transcriptome using Cell Ranger version 1.3 (https://support.10xgenomics.com/single-cell-gene-expression/software/pipelines/latest/what-is-cell-ranger) with default parameters. Only confidently mapped reads, non-PCR duplicates, with valid barcodes and UMIs (Unique Molecular Identifiers) were retained. We filtered out low quality cells. A minimum of 300 unique genes detected for cell was required, additionally cells with a ratio of mitochondrial versus endogenous gene expression exceeding 0.1 were discarded. Resulting 10,821 cells were retained for further analysis. Gene expression values were quantified in log transformed transcript per million [log(TPM + 1)]. Downstream analyses were performed using the R software package Seurat version 2.1 (https://github.com/satijalab/seurat/). Cell clustering and tSNE analysis were performed on 1,031 most variable genes, selected with mean expression higher than 0.01 and log transformed variance to mean ratio higher than 0.5.

**Nanostring data analysis**

RNA expression was evaluated on an nCounter SPRINT instrument using the nCounter PanCancer Immune Profiler for Mouse panel (Nanostring Technologies) following manufacturer's instructions. Transcript counts obtained with NanoString® were processed as follows. Raw counts in RCC files were normalized using R/Bioconductor library NanoStringNorm (v 1.1.21)[40]; technical assay variation was normalized using geometric mean of internal controls, background was estimated calculating the mean of negative controls, RNA counts were normalized using the geometric mean of housekeeping genes expression, additional variance stabilization was applied. Differential expression of normalized data was evaluated by means of linear models implemented in the R/Bioconductor limma library[41] . Transcripts were considered differentially expressed with a threshold of adjusted p-value <0.05 (Benjamini-Hochberg correction).

**Statistical analysis**

Values are expressed as mean ± standard error of the mean (SEM) as indicated. Statistical analyses were performed by Mann-Whitney test or Kruskall-Wallis test followed by Dunn post-test correction, unless otherwise indicated. Differences were considered statistically significant at p<0.05. Analyses were performed with R 3.2.2[42], NPC Test R10[43] and Prism (GraphPad Prism version 7.0). Analysis of cell populations in Fig. 2 was performed by nonparametric com-

bination test[43] , which is a permutation-based alternative test to the parametric Hotelling T-square two-sample test. This methodology allows performing multivariate comparison of the means in groups with small number of observations which do not satisfy the assumption for multivariate normality. Fig. 1a, I, Fig. 16b, Figure 17 and Figure 20b,e were modeled within a nonparametric framework which allows accounting for small sample size, the presence of outliers, non-Gaussian and heavily skewed data distribution, for which parametric procedures are not appropriate. A robust and flexible rank-based method suitable for the longitudinal analysis in factorial design was applied[44,45] . In this context hypothesis testing is focused on detecting differences in the distributions of collected variables rather than on difference between means[46,47]. This analysis was implemented by using nparLD package developed in R46 . Survival curves were estimated by means of Kaplan-Meier (KM). The event variable considered here was time to death, no informative censoring occurs. Nonparametric log-rank (Mantel-Haenszel) test statistics were used to compare the k survival curves related to the k-samples[48]. In the presence of more than 2 groups, multiple comparison issue was addressed by adjusting p-value by the Bonferroni method.

**Example 7**

We developed a novel lentiviral vector construct for tumour-specific expression of interferon-gamma (IFNγ), a type-II interferon. Tumour-specificity is provided by transcriptional control through enhancer/promoter sequences from the Tie2 (Tek) locus driving transgene expression selectively in a subset of tumour-infiltrating myeloid cells, while preventing expression in hematopoietic stem and progenitor cells with the help of microRNA target sequences recognized by miR-126-3p and miR-130a, as described previously (Escobar, G. et al. (2014) Sci Transl Med 6: 217ra3). This construct was termed "Tig126/130a".

An analogous construct for expression of interferon-alpha under the control of Tie2 and a miR-126 target sequence (i.e. Tie2.Ifna.126T) was termed "Tia126".

In addition to the Tig126/130a and Tia126 vectors, we developed a lentiviral vector carrying the cDNA of the tumor necrosis factor alpha (Tnfa) gene, which was termed "Tta126/130a". The structure of the constructs (e.g. Tig126/130a and Tta126/130a) is shown in Figure 28.

The therapy is delivered by transducing ex vivo hematopoietic stem and progenitor cells (HSPCs) with Tig126/130a, followed by transplantation of the engineered cells into a conditioned recipient. After engraftment, the HSPC progeny will expand and differentiate, and some of them will be recruited into the tumour microenvironment. Promoter activity is specifically upregulated in a subset of tumour-infiltrating monocytes/macrophages, in particular in Tie2-expressing monocytes/macrophages (TEMs) that localise to perivascular areas and have proangiogenic and immunosuppressive functions (De Palma, M. et al. (2005) Cancer Cell 8: 211-226; De Palma, M. et al. (2008) Cancer Cell 14: 299-311), delivering the cargo (e.g. IFNγ) in this strategic position.

We employed a transplantable model of B-acute lymphoblastic leukaemia (B-ALL) closely mimicking human Philadelphia-like B-ALL (Nucera, S. et al. (2016) Cancer Cell 29: 905-921), which readily induces a protumoral, immunosuppressive microenvironment characterised, for example, by the appearance of myeloid-derived suppressor cells, downregulation of MHC class II genes and upregulation of IL10 and PD1.

We first assessed the consequences of Tig126/130a-based IFNγ delivery on leukaemia growth and the protumoral microenvironment induced by the leukaemia (Figure 29).

6- to 8-week old C57/BL6 mice were reconstituted with Lineage-negative HSPCs transduced with either Tig126/130a, Tta126/130a or a control lentiviral vector expressing the biologically inactive, truncated nerve growth factor receptor (NGFR) gene. Tig126/130a and Tta126/130a dosage was quantified as a vector copy number of 1,39 and 2,01, respectively in vitro, and 0,44 and 0,83, respectively in vivo. Eight weeks post transplant, upon reconstitution of the peripheral blood, animals received the B-ALL that is then followed by frequent blood collection. Compared to control animals, mice engineered to express IFNγ showed significantly slower disease progression (Figure 29A). Moreover, at sacrifice we observed up-regulation of MHC class II expression on splenic macrophages (Figure 29B) as well as a reduction of myeloid-derived suppressor cells in the bone marrow (Figure 29C) pointing towards a conversion of the tumour microenvironment to a more anti-tumoral, "M1-like" polarisation state. The degree of leukaemia inhibition by Tig126/130a gene therapy was comparable to what we have previously observed with a type-I interferon cargo (namely, interferon-alpha) deployed using a similar vector backbone (Tia126), the same delivery platform and the same leukaemia model. On the contrary, Tta126/130a vector had no measurable effect on leukaemia growth if deployed as a monotherapy.

We then combined the contemporaneous delivery of several immunostimulatory cytokines into the leukaemia microenvironment using the Tie2 vector backbone equipped with different transgene cargo. We co-transduced lineage-negative HSPCs with doublet combinations of the Tia126, Tig126/130a and Tta126/130a vectors as compared to Tia126 monotherapy or a control group transduced with the biologically inactive Tie2.NGFR vector, and transplanted the cells into lethally irradiated recipient mice. Mice reconstituted as expected, with no additive toxicity due to combined cytokine expression. Vector copy number of the single vectors measured by specific quantitative PCR assays was rather low ranging from 0.1 to 0.3 in the reconstituted mice. Mice were challenged with B-ALL, as described above. Tia126 alone

showed a reduction in leukaemia growth compared to control, similarly to combinations with either Tig126/130a or Tta126/130a (Figure 30A).

Unexpectedly, the combination of Tig126/130a and Tta126/130a resulted in a dramatic reduction in leukaemia progression, associated with a significant increase in MHC class II expression on splenic macrophages (Figure 30B), reduction in MDSC (Figure 30C) and an increase in cytotoxic CD8+ T cells in the spleen (Figure 30D). These data suggest a synergism between IFNγ and TNFa, locally expressed in the tumour microenvironment through leukaemia infiltrating myeloid cells under control of the Tie2 enhancer/promoter.

Furthermore, we evaluated the potential synergy of our gene therapy-based delivery of IFNγ with other immunotherapeutic strategies including checkpoint inhibitors anti-CTLA-4 and anti-LAG-3 and the inhibitor of the IDO enzyme, 1-Methyl-Tryptophan (1-MT). We transduced lineage-negative HSPCs with Tig126/130a or a control biologically inactive Tie2.NGFR vector, and transplanted the cells into lethally irradiated recipient mice. Mice reconstituted as expected, with no additive toxicity due to combined cytokine expression. Vector copy number of the single vectors measured by specific quantitative PCR assays was 0,72 for the Tig126/130a and 0,84 for the controls in the reconstituted mice. Mice were challenged with B-ALL, as described above.

We observed that while anti-CTLA-4 (αCTLA4) and anti-LAG-3 (αLAG3) showed some efficacy already in monotherapy in reducing the B-ALL progression, the 1-MT treatment had no beneficial effect (Figure 31A). Moreover, combination of gene therapy-based delivery of IFNγ with both 1-MT and checkpoint inhibitors increased the efficacy of the treatments leading to a reduction of the disease progression (Figure 31A) associated with a significant increase in MHC class II expression on splenic and bone marrow macrophages (Figure 31A and 31B) and an increase in cytotoxic CD8+ T cells in the spleen (Figure 31D).

**REFERENCES**

1. Khalil, D. N., Smith, E. L., Brentjens, R. J. & Wolchok, J. D. The future of cancer treatment: immunomodulation, CARs and combination immunotherapy. Nat. Rev. Clin. Oncol. 13, 273-290 (2016).

2. Topalian, S. L., Drake, C. G. & Pardoll, D. M. Immune Checkpoint Blockade : A Common Denominator Approach to Cancer Therapy. Cancer Cell 27, 450-461 (2015).

3. Schadendorf, D. et al. Pooled Analysis of Long-Term Survival Data From Phase II and Phase III Trials of Ipilimumab in Unresectable or Metastatic Melanoma. J. Clin. Oncol. 33, 1889-94 (2015).

4. Pitt, J. M. et al. Resistance Mechanisms to Immune-Checkpoint Blockade in Cancer: Tumor-Intrinsic and -Extrinsic Factors. Immunity 44, 1255-1269 (2016).

5. Parker, B. S., Rautela, J. & Hertzog, P. J. Antitumour actions of interferons: implications for cancer therapy. Nat. Rev. Cancer 16, 131-44 (2016).

6. Hervas-Stubbs, S. et al. Direct effects of type I interferons on cells of the immune system. Clin. Cancer Res. 17, 2619-27 (2011).

7. Fuertes, M. B., Woo, S. R., Burnett, B., Fu, Y. X. & Gajewski, T. F. Type I interferon response and innate immune sensing of cancer. Trends Immunol. 34, 67-73 (2013).

8. De Palma, M. et al. Tumor-Targeted Interferon-Alpha Delivery by Tie2-Expressing Monocytes Inhibits Tumor Growth and Metastasis. Cancer Cell 14, 299-311 (2008).

9. Escobar, G. et al. Genetic engineering of hematopoiesis for targeted IFN-α delivery inhibits breast cancer progression. Sci. Transl. Med. 6, 217ra3 (2014).

10. Catarinella, M. et al. IFNα gene/cell therapy curbs colorectal cancer colonization of the liver by acting on the hepatic microenvironment. EMBO Mol. Med. 8, 155-70 (2016).

11. Fesnak, A. D., June, C. H. & Levine, B. L. Engineered T cells: the promise and challenges of cancer immunotherapy. Nat. Rev. Cancer 16, 566-81 (2016).

12. Rosenberg, S. A. & Restifo, N. P. Adoptive cell transfer as personalized immunotherapy for human cancer. Science (80-. ). 348, 62-68 (2015).

13. Sotillo, E. et al. Convergence of acquired mutations and alternative splicing of CD19 enables resistance to CART-19 immunotherapy. Cancer Discov. 5, 1282-1295 (2015).

14. Nucera, S. et al. miRNA-126 Orchestrates an Oncogenic Program in B Cell Precursor Acute Lymphoblastic Leukemia. Cancer Cell 29, 905-921 (2016).

15. De Palma, M., Venneri, M. A., Roca, C. & Naldini, L. Targeting exogenous genes to tumor angiogenesis by transplantation of genetically modified hematopoietic stem cells. Nat. Med. 9, 789-95 (2003).

16. De Palma, M. et al. Tie2 identifies a hematopoietic lineage of proangiogenic monocytes required for tumor vessel formation and a mesenchymal population of pericyte progenitors. Cancer Cell 8, 211-26 (2005).

17. Mellman, I., Coukos, G. & Dranoff, G. Cancer immunotherapy comes of age. Nature 480, 480-9 (2011).

18. Kumar, V., Patel, S., Tcyganov, E. & Gabrilovich, D. I. The Nature of Myeloid-Derived Suppressor Cells in the Tumor Microenvironment. Trends Immunol. 37, 208-220 (2016).

19. Ruffell, B. & Coussens, L. M. Macrophages and therapeutic resistance in cancer. Cancer Cell 27, 462-472 (2015).

20. Joyce Johanna A. and Fearon Douglas T. T cell exclusion, immune privilege, and the tumor microenvironment. Science (80-. ). 348, (2015).

21. Verdegaal, E. M. E. et al. Neoantigen landscape dynamics during human melanoma-T cell interactions. Nature 536, 91-5 (2016).

22. Matsushita, H. et al. Cancer exome analysis reveals a T-cell-dependent mechanism of cancer immunoediting. Nature 482, 400-4 (2012).

23. Linnemann, C. et al. High-throughput epitope discovery reveals frequent recognition of neo-antigens by CD4+ T cells in human melanoma. Nat Med 21, 81-85 (2015).

24. Lu, Y. C. et al. Efficient identification of mutated cancer antigens recognized by T cells associated with durable tumor regressions. Clin. Cancer Res. 20, 3401-3410 (2014).

25. McGranahan, N. et al. Clonal neoantigens elicit T cell immunoreactivity and sensitivity to immune checkpoint blockade. Science (80-. ). 351, 1463-1469 (2016).

26. Schumacher, T. N. & Schreiber, R. D. Neoantigens in cancer immunotherapy. Science (80-. ). 348, 69-74 (2015).

27. Sharma, P. & Allison, J. P. Immune Checkpoint Targeting in Cancer Therapy : Toward Combination Strategies with Curative Potential. Cell 161, 205-214 (2015).

28. Schildberg, F. A., Klein, S. R., Freeman, G. J. & Sharpe, A. H. Coinhibitory Pathways in the B7-CD28 Ligand-Receptor Family. Immunity 44, 955-972 (2016).

29. Yang, X. et al. Targeting the Tumor Microenvironment with Interferon-β Bridges Innate and Adaptive Immune Responses. Cancer Cell 25, 37-48 (2014).

30. Huang, T.-H., Chintalacharuvu, K. R. & Morrison, S. L. Targeting IFN-alpha to B Cell Lymphoma by a Tumor-Specific Antibody Elicits Potent Antitumor Activities. J. Immunol. 179, 6881-6888 (2007).

31. Wilson, E. B. et al. Blockade of chronic type I interferon signaling to control persistent LCMV infection. Science 340, 202-7 (2013).

32. Sandler, N. G. et al. Type I interferon responses in rhesus macaques prevent SIV infection and slow disease

progression. Nature 511, 601-605 (2014).

33. Joseph Benci, A. L. et al. Tumor Interferon Signaling Regulates a Multigenic Resistance Program to Immune Checkpoint Blockade. Cell 167, 1540-1554 (2016).

34. Gentles, A. J. et al. The prognostic landscape of genes and infiltrating immune cells across human cancers. Nat. Med. 21, 938-945 (2015).

35. Mlecnik, B. et al. Integrative Analyses of Colorectal Cancer Show Immunoscore Is a Stronger Predictor of Patient Survival Than Microsatellite Instability. Immunity 44, 698-711 (2016).

36. Bachireddy, P., Burkhardt, U. E., Rajasagi, M. & Wu, C. J. Haematological malignancies: at the forefront of immunotherapeutic innovation. Nat. Rev. Cancer 15, 201-215 (2015).

37. Mátrai, J. et al. Hepatocyte-targeted expression by integrase-defective lentiviral vectors induces antigen-specific tolerance in mice with low genotoxic risk. Hepatology 53, 1696-1707 (2011).

38. Amendola, M., Venneri, M. A., Biffi, A., Vigna, E. & Naldini, L. Coordinate dual-gene transgenesis by lentiviral vectors carrying synthetic bidirectional promoters. Nat. Biotechnol. 23, 108-116 (2005).

39. Tumeh, P., Harview, C., Yearley, J. & Al, E. PD-1 blockade induces responses by inhibiting adaptive immune resistance. Nature 515, 568-571 (2014).

40. Waggott, D. et al. NanoStringNorm: An extensible R package for the pre-processing of nanostring mRNA and miRNA data. Bioinformatics 28, 1546-1548 (2012).

41. Ritchie, M. E. et al. limma powers differential expression analyses for RNA742 sequencing and microarray studies. Nucleic Acids Res. 43, e47 (2015).

42. R Development Core Team. R: A Language and Environment for Statistical Computing. R Foundation for Statistical Computing Vienna Austria 0, {ISBN} 3-900051-07-0 (2016).

43. Pesarin, F. & Salmaso, L. Permutation Tests for Complex Data. Permutation Tests for Complex Data: Theory, Applications and Software (2010). doi:10.1002/9780470689516

44. Brunner, E. & Puri, M. L. Nonparametric methods in factorial designs. Stat. Pap. 42, 1-52 (2001).

45. Brunner, E., Domhof, S. & Langer, F. Nonparametric analysis of longitudinal data in factorial experiments. Wiley Ser. Probab. Stat. xvii, 261 (2002).

46. Noguchi, K., Gel, Y. R., Brunner, E. & Konietschke, F. nparLD: An R software package for the nonparametric analysis of longitudinal data in factorial experiments. J. Stat. Softw. 50, 1-23 (2012).

47. Feys, J. New Nonparametric Rank Tests for Interactions in Factorial Designs with Repeated Measures New Nonparametric Rank Tests for. 15, (2016).

48. Therneau, T. M. A Package for Survival Analysis in S. Survival (2015).

## Claims

1. A hematopoietic stem cell (HSC), a hematopoietic progenitor cell (HPC), a myeloid/monocyte-committed progenitor cell, a macrophage or a monocyte comprising a vector wherein the vector comprises at least one mir-130a and/or mir-126 target sequence operably linked to a nucleotide sequence encoding a cytokine for use in treating or preventing a cancer in a patient, wherein the HSC, the HPC, the myeloid/monocyte-committed progenitor cell, the macrophage or the monocyte is used in combination with a tumor associated antigen (TAA)-specific T-cell,

   preferably wherein the cytokine is an interferon (IFN), IL-12 or granulocyte-macrophage colony-stimulating

factor (GM-CSF),
preferably wherein the IFN is a type I IFN (preferably IFNα or IFNβ), or a type II IFN (preferably IFNγ),
more preferably wherein the type I IFN is IFNα.

**2.** A HSC, a HPC, a myeloid/monocyte-committed progenitor cell, a macrophage or a monocyte for use according to claim 1, wherein the HSC, the HPC, the myeloid/monocyte-committed progenitor cell, the macrophage or the monocyte is used further in combination with an immune checkpoint inhibitor.

**3.** A HSC, a HPC, a myeloid/monocyte-committed progenitor cell, a macrophage or a monocyte for use according to claim 1 or 2, wherein the TAA-specific T-cell expresses a chimeric antigen receptor (CAR) and/or a transgenic T cell receptor (TCR)

**4.** A HSC, a HPC, a myeloid/monocyte-committed progenitor cell, a macrophage or a monocyte for use according any preceding claim, wherein the TAA is selected from the group consisting of carcinoembryonic antigen (CEA), estrogen receptor, progesterone receptor, ephrinB2, ROR1, mesothelin, c-Met, GD-2, and MAGE A3 TCR, 4-1BB, adenocarcinoma antigen, alpha-fetoprotein, BAFF, B-lymphoma cell, C242 antigen, carbonic anhydrase 9 (CA-IX), CCR4, CD152, CD200, CD22, CD19, CD22, CD123, CD221, CD23 (IgE receptor), CD28, CD4, CD40, CD44, CD44 v6, CD51, CD52, CD56, CD74, CD80, CS-1, CNT0888, CTLA-4, DR5, EpCAM, CD3, fibronectin extra domain-B, folate receptor 1, glycoprotein 75, GPNMB, HGF, human scatter factor receptor kinase, IGF-1 receptor, IGF-I, IgGI, L1-CAM, IL-13, IL-6, insulin-like growth factor I receptor, integrin α5β1, integrin αvβ3, MORAb-009, MS4A1, mucin CanAg, N-glycolylneuraminic acid, NPC-1C, PDGF-Rα, PDL192, phosphatidylserine, prostatic carcinoma cells, RANKL, RON, SCH 900105, SDC1, SLAMF7, tenascin C, TGF beta 2, TGF-β, TRAIL-R1, TRAIL-R2, tumor antigen CTAA16.88, Vascular endothelial growth, factor (VEGF), VEGF-A, VEGFR-1, VEGFR2, vimentin, 5T4, CD5, CD19, CD20, CD21, CD25, CD37, CD30, CD33, CD45, CAMPATH-1 (CDw52), HLA-DR, Anti-idiotype, TAG-72, Ep-CAM, MUC1, Folate-binding protein, A33, G250, Prostate-specific membrane antigen, (PSMA), Prostate specific antigen (PSA), Ferritin, Gangliosides (e.g. GD2, GD3, GM2), $Le^y$, CA-125, CA19-9, Epidermal growth factor receptor (EGFR), p185HER2, IL-2 receptor, de2-7 EGFR, Fibroblast activation protein (FAP), Tenascin, metalloproteinases, Endosialin, Carbonic anhydrase, Galectin 9, Aldolase A, elFγ4, Tyrosinase, Galectin 4, HERKV-K10, p53, NY-LU-12, Restin, NY-CO-38, MAGE-1, MAGE-4a, SSX2, NY-ESO-1, SCP-1, 707-AP, AFP, ART-4, BAGE, b-catenin/m, Bcr-abl, CAMEL, CAP-1, CASP-8, CDC27m, CDK4/m, CT, Cyp-B, DAM-6 (MAGE-B2) and DAM-10 (MAGE-B1), ELF2M, ETV6-AML1, G250, GAGE, GnT-V, Gp100, HAGE, HER-2/neu, HLA-A*0201-R170I, HPV-E7, HSP70-2M, HST-2, hTERT (hTRT), iCE, KIAA0205, LAGE, LDLR/FUT, MAGE, MART-1/Melan-A, MC1R, Myosin/m, MUC1, MUM-1, MUM-2, MUM-3, NA88-A, NY-ESO-1, P15, p190 minor bcr-abl, Pml/RARa, PRAME, RAGE, RU1, RU2, SAGE, SART-1, SART-3, TEL/AML1, TPI/m, TRP-1, protein 1, gp75, TRP-2, TRP-2/INT2 and WT1,
preferably, wherein the TAA-specific T-cell expresses a CD19-specific CAR.

**5.** A HSC, a HPC, a myeloid/monocyte-committed progenitor cell, a macrophage or a monocyte for use according to any preceding claim, wherein the TAA-specific T-cell is derived from a cell isolated from the patient.

**6.** A HSC, a HPC, a myeloid/monocyte-committed progenitor cell, a macrophage or a monocyte for use according to any preceding claim, wherein the TAA-specific T-cell has been engineered to disrupt at least one endogenous gene, preferably wherein the at least one endogenous gene is selected from an endogenous gene encoding a TCR α chain, a TCR β chain and a major histocompatibility complex (MHC).

**7.** A HSC, a HPC, a myeloid/monocyte-committed progenitor cell, a macrophage or a monocyte for use according to any one of claims 2-6, wherein the immune checkpoint inhibitor:

(i) is an antibody, preferably wherein the immune checkpoint inhibitor antibody is selected from the group consisting of an anti-CTLA4 antibody, an anti-PD1 antibody, an anti-PDL1 antibody, an anti-PDL2 antibody and an anti-LAG-3 antibody; and/or
(ii) inhibits an inhibitory checkpoint molecule selected from the group consisting of A2AR (Adenosine A2A receptor), B7-H3 (CD276), B7-H4 (VTCN1), BTLA (B and T Lymphocyte Attenuator; CD272), HVEM (Herpesvirus Entry Mediator), CTLA-4 (Cytotoxic T-Lymphocyte-Associated protein 4; CD152), IDO (Indoleamine 2,3-dioxygenase), TDO (tryptophan 2,3-dioxygenase), KIR (Killer-cell Immunoglobulin-like Receptor), LAG3 (Lymphocyte Activation Gene-3), PD-1 (Programmed Death 1 receptor), PD-L1 (PD-1 ligand 1), PD-L2 (PD-1 ligand 2), TIM-3 (T-cell Immunoglobulin domain and Mucin domain 3), VISTA (V-domain Ig Suppressor of T cell Activation), B7-1 (CD80), and B7-2 (CD86).

**8.** A HSC, a HPC, a myeloid/monocyte-committed progenitor cell, a macrophage or a monocyte for use according to any preceding claim, wherein the vector comprises:

(i) at least one mir-130a target sequence and at least one mir-126 target sequence, preferably wherein the vector comprises two mir-130a target sequences and two mir-126 target sequences; and/or
(ii) a tissue specific promoter operably linked to the nucleotide sequence encoding the cytokine, preferably wherein the tissue specific promoter is a TEK (Tie2) promoter.

**9.** A HSC, a HPC, a myeloid/monocyte-committed progenitor cell, a macrophage or a monocyte for use according to any preceding claim, wherein the cancer is a hematological malignancy or a solid tumor, preferably wherein the hematological malignancy is selected from the group consisting of acute myeloid leukemia (AML), lymphoblastic leukemia, acute lymphoblastic leukemia (ALL), myelodysplastic syndromes (MDS), myeloproliferative neoplasms (MPN), primary myelofibrosis, essential thrombocythemia, polycythemia vera, atypical chronic myeloid leukemia, chronic myeloid leukemia (CML), lymphoma, multiple myeloma, non Hodgkin lymphoma and Hodgkin lymphoma; or preferably wherein the solid tumor is selected from the group consisting of lung cancer, breast cancer, oesophageal cancer, gastric cancer, colon cancer, cholangiocarcinoma, pancreatic cancer, ovarian cancer, head and neck cancers, synovial sarcoma, angiosarcoma, osteosarcoma, thyroid cancer, endometrial cancer, neuroblastoma, rabdomyosarcoma, liver cancer, melanoma, prostate cancer, renal cancer, soft tissue sarcoma, urothelial cancer, biliary cancer, glioblastoma, cervical cancer and colorectal cancer.

**10.** A tumor associated antigen (TAA)-specific T-cell for use in treating or preventing a cancer in a patient wherein the patient has previously been administered with a hematopoietic stem cell (HSC) a hematopoietic progenitor cell (HPC), a myeloid/monocyte-committed progenitor cell, a macrophage or a monocyte comprising a vector wherein the vector comprises at least one mir-130a and/or mir-126 target sequence operably linked to a nucleotide sequence encoding a cytokine, preferably wherein the TAA is selected from the group consisting of carcinoembryonic antigen (CEA), estrogen receptor, progesterone receptor, ephrinB2, ROR1, mesothelin, c-Met, GD-2, and MAGE A3 TCR, 4-1BB, adenocarcinoma antigen, alpha-fetoprotein, BAFF, B-lymphoma cell, C242 antigen, carbonic anhydrase 9 (CA-IX), CCR4, CD152, CD200, CD22, CD19, CD22, CD123, CD221, CD23 (IgE receptor), CD28, CD4, CD40, CD44, CD44 v6, CD51, CD52, CD56, CD74, CD80, CS-1, CNT0888, CTLA-4, DR5, EpCAM, CD3, fibronectin extra domain-B, folate receptor 1, glycoprotein 75, GPNMB, HGF, human scatter factor receptor kinase, IGF-1 receptor, IGF-I, IgGI, L1-CAM, IL-13, IL-6, insulin-like growth factor I receptor, integrin $\alpha 5\beta 1$, integrin $\alpha v\beta 3$, MORAb-009, MS4A1, mucin CanAg, N-glycolylneuraminic acid, NPC-1C, PDGF-R$\alpha$, PDL192, phosphatidylserine, prostatic carcinoma cells, RANKL, RON, SCH 900105, SDC1, SLAMF7, tenascin C, TGF beta 2, TGF-$\beta$, TRAIL-R1, TRAIL-R2, tumor antigen CTAA16.88, Vascular endothelial growth, factor (VEGF), VEGF-A, VEGFR-1, VEGFR2, vimentin, 5T4, CD5, CD19, CD20, CD21, CD25, CD37, CD30, CD33, CD45, CAMPATH-1 (CDw52), HLA-DR, Anti-idiotype, TAG-72, Ep-CAM, MUC1, Folate-binding protein, A33, G250, Prostate-specific membrane antigen, (PSMA), Prostate specific antigen (PSA), Ferritin, Gangliosides (e.g. GD2, GD3, GM2), $Le^y$, CA-125, CA19-9, Epidermal growth factor receptor (EGFR), p185HER2, IL-2 receptor, de2-7 EGFR, Fibroblast activation protein (FAP), Tenascin, metalloproteinases, Endosialin, Carbonic anhydrase, Galectin 9, Aldolase A, eIF$\gamma$4, Tyrosinase, Galectin 4, HERKV-K10, p53, NY-LU-12, Restin, NY-CO-38, MAGE-1, MAGE-4a, SSX2, NY-ESO-1, SCP-1, 707-AP, AFP, ART-4, BAGE, b-catenin/m, Bcr-abl, CAMEL, CAP-1, CASP-8, CDC27m, CDK4/m, CT, Cyp-B, DAM-6 (MAGE-B2) and DAM-10 (MAGE-B1), ELF2M, ETV6-AML1, G250, GAGE, GnT-V, Gp100, HAGE, HER-2/neu, HLA-A*0201-R170I, HPV-E7, HSP70-2M, HST-2, hTERT (hTRT), iCE, KIAA0205, LAGE, LDLR/FUT, MAGE, MART-1/Melan-A, MC1R, Myosin/m, MUC1, MUM-1, MUM-2, MUM-3, NA88-A, NY-ESO-1, P15, p190 minor bcr-abl, Pml/RARa, PRAME, RAGE, RU1, RU2, SAGE, SART-1, SART-3, TEL/AML1, TPI/m, TRP-1, protein 1, gp75, TRP-2, TRP-2/INT2 and WT1, preferably wherein the TAA-specific T-cell expresses a chimeric antigen receptor (CAR) and/or a transgenic T-cell receptor (TCR),

preferably wherein the cytokine is an interferon (IFN), IL-12 or granulocyte-macrophage colony-stimulating factor (GM-CSF),
preferably wherein the IFN is a type I IFN (preferably IFN$\alpha$ or IFN$\beta$), or a type II IFN (preferably IFN$\gamma$),
more preferably wherein the type I IFN is IFN$\alpha$,
preferably, wherein the TAA-specific T-cell expresses a CD19-specific CAR.

**11.** A TAA-specific T-cell for use according to claim 10 wherein the vector comprises:

(i) at least one mir-130a target sequence and at least one mir-126 target sequence, preferably wherein the vector comprises two mir-130a target sequences and two mir-126 target sequences; and/or

(ii) a tissue specific promoter operably linked to the nucleotide sequence encoding the cytokine, preferably wherein the tissue specific promoter is a TEK (Tie2) promoter.

**12.** A TAA-specific T-cell for use according to claim 10 or 11 wherein the cancer is a hematological malignancy or a solid tumor, preferably wherein the hematological malignancy is selected from the group consisting of acute myeloid leukemia (AML), lymphoblastic leukemia, acute lymphoblastic leukemia (ALL), myelodisplastic syndromes, lymphoma, multiple myeloma, non Hodgkin lymphoma and Hodgkin lymphoma; or preferably wherein the solid tumor is selected from the group consisting of lung cancer, breast cancer, oesophageal cancer, gastric cancer, colon cancer, cholangiocarcinoma, pancreatic cancer, ovarian cancer, head and neck cancers, synovial sarcoma, angiosarcoma, osteosarcoma, thyroid cancer, endometrial cancer, neuroblastoma, rabdomyosarcoma, liver cancer, melanoma, prostate cancer, renal cancer, soft tissue sarcoma, urothelial cancer, biliary cancer, glioblastoma, cervical cancer and colorectal cancer.

**13.** A product comprising: (a) a hematopoietic stem cell (HSC), a hematopoietic progenitor cell (HPC), a myeloid/monocyte-committed progenitor cell, a macrophage or a monocyte comprising a vector wherein the vector comprises at least one mir-130a and/or mir-126 target sequence operably linked to a nucleotide sequence encoding a cytokine; and (b) a tumor associated antigen (TAA)-specific T-cell,

preferably wherein the cytokine is an interferon (IFN), IL-12 or granulocyte-macrophage colony-stimulating factor (GM-CSF),
preferably wherein the IFN is a type I IFN (preferably IFN$\alpha$ or IFN$\beta$), or a type II IFN (preferably IFN$\gamma$),
more preferably wherein the type I IFN is IFN$\alpha$.

## Patentansprüche

**1.** Hämatopoetische Stammzelle (HSC), hämatopoetische Progenitorzelle (HPC), Myeloid/Monozyten-festgelegte Progenitorzelle, Makrophage oder Monozyt, umfassend einen Vektor, wobei der Vektor mindestens eine mir-130a- und/oder mir-126-Zielsequenz in funktioneller Verknüpfung mit einer für ein Zytokin codierenden Nukleotidsequenz umfasst, zur Verwendung bei der Behandlung oder Verhinderung eines Krebses in einem Patienten, wobei die HSC, die HPC, die Myeloid/Monozytenfestgelegte Progenitorzelle, der Makrophage oder der Monozyt in Kombination mit einer Tumor-assoziiertes-Antigen (TAA)-spezifischen T-Zelle verwendet wird,

vorzugsweise wobei das Zytokin ein Interferon (IFN), IL-12 oder Granulozyten-Makrophagen-Kolonie-Stimulierungsfaktor (GM-CSF) ist,
vorzugsweise wobei das IFN ein Typ-I-IFN (vorzugsweise IFN$\alpha$ oder IFN$\beta$) oder ein Typ-II-IFN (vorzugsweise IFN$\gamma$) ist,
wobei es sich stärker bevorzugt bei dem Typ-I-IFN um IFN$\alpha$ handelt.

**2.** HSC, HPC, Myeloid/Monozyten-festgelegte Progenitorzelle, Makrophage oder Monozyt zur Verwendung gemäß Anspruch 1, wobei die HSC, die HPC, die Myeloid/Monozyten-festgelegte Progenitorzelle, der Makrophage oder der Monozyt ferner in Kombination mit einem Immuncheckpoint-Inhibitor verwendet wird.

**3.** HSC, HPC, Myeloid/Monozyten-festgelegte Progenitorzelle, Makrophage oder Monozyt zur Verwendung gemäß Anspruch 1 oder 2, wobei die TAA-spezifische T-Zelle einen chimären Antigenrezeptor (CAR) und/oder einen transgenen T-Zell-Rezeptor (TCR) exprimiert.

**4.** HSC, HPC, Myeloid/Monozyten-festgelegte Progenitorzelle, Makrophage oder Monozyt zur Verwendung gemäß einem beliebigen vorhergehenden Anspruch, wobei das TAA ausgewählt wird aus der Gruppe bestehend aus karzinoembryonalem Antigen (CEA), Östrogenrezeptor, Progesteronrezeptor, EphrinB2, ROR1, Mesothelin, c-Met, GD-2 und MAGE-A3-TCR, 4-1BB, Adenokarzinom-Antigen, alpha-Fetoprotein, BAFF, B-Lymphom-Zelle, C242-Antigen, Carboanhydrase 9 (CA-IX), CCR4, CD152, CD200, CD22, CD19, CD22, CD123, CD221, CD23 (IgE-Rezeptor), CD28, CD4, CD40, CD44, CD44 v6, CD51, CD52, CD56, CD74, CD80, CS-1, CNT0888, CTLA-4, DR5, EpCAM, CD3, Fibronektin-Extra-Domäne-B, Folatrezeptor 1, Glykoprotein 75, GPNMB, HGF, Human-Scatter-Faktor-Rezeptor-Kinase, IGF-1-Rezeptor, IGF-I, IgGI, L1-CAM, IL-13, IL-6, Insulin-like-Wachstumsfaktor I-Rezeptor, Integrin $\alpha5\beta1$, Integrin $\alpha v\beta3$, MORAb-009, MS4A1, Mucin CanAg, N-Glykolylneuraminsäure, NPC-1C, PDGF-R$\alpha$, PDL192, Phosphatidylserin, Prostatakarzinom-Zellen, RANKL, RON, SCH 900105, SDC1, SLAMF7, Tenascin C, TGF beta 2, TGF-$\beta$, TRAIL-R1, TRAIL-R2, Tumorantigen CTAA16.88, Gefäß-Endothelwachstumsfaktor (VEGF),

VEGF-A, VEGFR-1, VEGFR2, Vimentin, 5T4, CD5, CD19, CD20, CD21, CD25, CD37, CD30, CD33, CD45, CAMPATH-1 (CDw52), HLA-DR, Anti-Idiotyp, TAG-72, Ep-CAM, MUC1, Folatbindungsprotein, A33, G250, Prostata-spezifischem Membranantigen (PSMA), Prostata-spezifischem Antigen (PSA), Ferritin, Gangliosiden (z. B. GD2, GD3, GM2), $Le^y$, CA-125, CA19-9, Epidermalwachstumsfaktor-Rezeptor (EGFR), p185HER2, IL-2-Rezeptor, de2-7 EGFR, Fibroblasten-Aktivations-Protein (FAP), Tenascin, Metalloproteinasen, Endosialin, Carboanhydrase, Galectin 9, Aldolase A, elFγ4, Tyrosinase, Galectin 4, HERKV-K10, p53, NY-LU-12, Restin, NY-CO-38, MAGE-1, MAGE-4a, SSX2, NY-ESO-1, SCP-1, 707-AP, AFP, ART-4, BAGE, b-Catenin/m, Bcr-abl, CAMEL, CAP-1, CASP-8, CDC27m, CDK4/m, CT, Cyp-B, DAM-6 (MAGE-B2) und DAM-10 (MAGE-B1), ELF2M, ETV6-AML1, G250, GAGE, GnT-V, Gp100, HAGE, HER-2/neu, HLA-A*0201-R170I, HPV-E7, HSP70-2M, HST-2, hTERT (hTRT), iCE, KIAA0205, LAGE, LDLR/FUT, MAGE, MART-1/Melan-A, MC1R, Myosin/m, MUC1, MUM-1, MUM-2, MUM-3, NA88-A, NY-ESO-1, P15, p190 minor bcr-abl, Pml/RARa, PRAME, RAGE, RU1, RU2, SAGE, SART-1, SART-3, TEL/AML1, TPI/m, TRP-1, Protein 1, gp75, TRP-2, TRP-2/INT2 und WT1,
vorzugsweise wobei die TAA-spezifische T-Zelle einen CD19-spezifischen CAR exprimiert.

**5.** HSC, HPC, Myeloid/Monozyten-festgelegte Progenitorzelle, Makrophage oder Monozyt zur Verwendung gemäß einem beliebigen vorhergehenden Anspruch, wobei die TAA-spezifische T-Zelle von einer aus dem Patienten isolierten Zelle abgeleitet ist.

**6.** HSC, HPC, Myeloid/Monozyten-festgelegte Progenitorzelle, Makrophage oder Monozyt zur Verwendung gemäß einem beliebigen vorhergehenden Anspruch, wobei die TAA-spezifische T-Zelle konstruiert wurde, um mindestens ein endogenes Gen zu disruptieren, vorzugsweise wobei das mindestens eine endogene Gen ausgewählt ist aus einem endogenen Gen, codierend eine TCR-α-Kette, eine TCR-β-Kette bzw. einen Haupthistokompatibilitätskomplex (MHC).

**7.** HSC, HPC, Myeloid/Monozyten-festgelegte Progenitorzelle, Makrophage oder Monozyt zur Verwendung gemäß einem der Ansprüche 2-6, wobei der Immuncheckpoint-Inhibitor:

(i) ein Antikörper ist, vorzugsweise wobei der Immuncheckpoint-Inhibitor-Antikörper ausgewählt ist aus der Gruppe bestehend aus einem Anti-CTLA4-Antikörper, einem Anti-PD1-Antikörper, einem Anti-PDL1-Antikörper, einem Anti-PDL2-Antikörper und einem Anti-LAG-3-Antikörper; und/oder
(ii) ein inhibitorisches Checkpoint-Molekül, ausgewählt aus der Gruppe bestehend aus A2AR (Adenosin-A2A-Rezeptor), B7-H3 (CD276), B7-H4 (VTCN1), BTLA (B and T Lymphocyte Attenuator; CD272), HVEM (Herpesvirus Entry Mediator), CTLA-4 (Cytotoxische-T-Lymphozyten-Assoziiertes-Protein 4; CD152), IDO (Indolamin-2,3-Dioxygenase), TDO (Tryptophan-2,3-Dioxygenase), KIR (Killerzell-Immunglobulin-like-Rezeptor), LAG3 (Lymphocyte Activation Gene-3), PD-1 (Programmed-Death-1-Rezeptor), PD-L1 (PD-1-Ligand 1), PD-L2 (PD-1-Ligand 2), TIM-3 (T-Cell Immunoglobulin Domain and Mucin Domain 3), VISTA (V-Domain Ig Suppressor of T Cell Activation), B7-1 (CD80) und B7-2 (CD86), inhibiert.

**8.** HSC, HPC, Myeloid/Monozyten-festgelegte Progenitorzelle, Makrophage oder Monozyt zur Verwendung gemäß einem beliebigen vorhergehenden Anspruch, wobei der Vektor umfasst:

(i) mindestens eine mir-130a-Zielsequenz und mindestens eine mir-126-Zielsequenz, vorzugsweise wobei der Vektor zwei mir-130a-Zielsequenzen und zwei mir-126-Zielsequenzen umfasst; und/oder
(ii) einen gewebespezifischen Promotor in funktioneller Verknüpfung mit der für das Zytokin codierenden Nukleotidsequenz, vorzugsweise wobei der gewebespezifische Promotor ein TEK(Tie2)-Promotor ist.

**9.** HSC, HPC, Myeloid/Monozyten-festgelegte Progenitorzelle, Makrophage oder Monozyt zur Verwendung gemäß einem beliebigen vorhergehenden Anspruch, wobei der Krebs eine hämatologische Malignität oder ein solider Tumor ist, wobei die hämatologische Malignität vorzugsweise ausgewählt ist aus der Gruppe bestehend aus akuter myeloischer Leukämie (AML), lymphoblastischer Leukämie, akuter lymphatischer Leukämie (ALL), myelodysplastischen Syndromen (MDS), myeloproliferativen Neoplasien (MPN), primärer Myelofibrose, essentieller Thrombozythämie, Polycythaemia vera, atypischer chronischer myeloischer Leukämie, chronischer myeloischer Leukämie (CML), Lymphom, multiplem Myelom, Non-Hodgkin-Lymphom und Hodgkin-Lymphom; oder vorzugsweise wobei der solide Tumor ausgewählt ist aus der Gruppe bestehend aus Lungenkrebs, Brustkrebs, Speiseröhrenkrebs, Magenkrebs, Dickdarmkrebs, Cholangiokarzinom, Bauchspeicheldrüsenkrebs, Eierstockkrebs, Krebs im Kopf-Hals-Bereich, Synovialsarkom, Angiosarkom, Osteosarkom, Schilddrüsenkrebs, Endometriumkrebs, Neuroblastom, Rhabdomyosarkom, Leberkrebs, Melanom, Prostatakrebs, Nierenkrebs, Weichteilsarkom, Urothelkrebs, Gallenkrebs, Glioblastom, Gebärmutterhalskrebs und Kolorektalkrebs.

**10.** Tumor-assoziiertes-Antigen (TAA)-spezifische T-Zelle zur Verwendung zur Behandlung oder Verhinderung eines Krebses in einem Patienten, wobei dem Patienten zuvor eine hämatopoetische Stammzelle (HSC), eine hämatopoetische Progenitorzelle (HPC), eine Myeloid/Monozyten-festgelegte Progenitorzelle, ein Makrophage oder ein Monozyt, umfassend einen Vektor, wobei der Vektor mindestens eine mir-130a- und/oder mir-126-Zielsequenz in funktioneller Verknüpfung mit einer für ein Zytokin codierenden Nukleotidsequenz umfasst, verabreicht wurde, vorzugsweise wobei das TAA ausgewählt wird aus der Gruppe bestehend aus karzinoembryonalem Antigen (CEA), Östrogenrezeptor, Progesteronrezeptor, EphrinB2, ROR1, Mesothelin, c-Met, GD-2 und MAGE-A3-TCR, 4-1BB, Adenokarzinom-Antigen, alpha-Fetoprotein, BAFF, B-Lymphom-Zelle, C242-Antigen, Carboanhydrase 9 (CA-IX), CCR4, CD152, CD200, CD22, CD19, CD22, CD123, CD221, CD23 (IgE-Rezeptor), CD28, CD4, CD40, CD44, CD44 v6, CD51, CD52, CD56, CD74, CD80, CS-1, CNT0888, CTLA-4, DR5, EpCAM, CD3, Fibronektin-Extra-Domäne-B, Folatrezeptor 1, Glykoprotein 75, GPNMB, HGF, Human-Scatter-Faktor-Rezeptor-Kinase, IGF-1-Rezeptor, IGF-I, IgGI, L1-CAM, IL-13, IL-6, Insulin-like-Wachstumsfaktor I-Rezeptor, Integrin $\alpha 5\beta 1$, Integrin $\alpha v\beta 3$, MORAb-009, MS4A1, Mucin CanAg, N-Glykolylneuraminsäure, NPC-1C, PDGF-R$\alpha$, PDL192, Phosphatidylserin, Prostatakarzinom-Zellen, RANKL, RON, SCH 900105, SDC1, SLAMF7, Tenascin C, TGF beta 2, TGF-$\beta$, TRAIL-R1, TRAIL-R2, Tumorantigen CTAA16.88, Gefäß-Endothelwachstumsfaktor (VEGF), VEGF-A, VEGFR-1, VEGFR2, Vimentin, 5T4, CD5, CD19, CD20, CD21, CD25, CD37, CD30, CD33, CD45, CAMPATH-1 (CDw52), HLA-DR, Anti-Idiotyp, TAG-72, Ep-CAM, MUC1, Folatbindungsprotein, A33, G250, Prostata-spezifischem Membranantigen (PSMA), Prostata-spezifischem Antigen (PSA), Ferritin, Gangliosiden (z. B. GD2, GD3, GM2), $Le^y$, CA-125, CA19-9, Epidermalwachstumsfaktor-Rezeptor (EGFR), p185HER2, IL-2-Rezeptor, de2-7 EGFR, Fibroblasten-Aktivations-Protein (FAP), Tenascin, Metalloproteinasen, Endosialin, Carboanhydrase, Galectin 9, Aldolase A, elF$\gamma$4, Tyrosinase, Galectin 4, HERKV-K10, p53, NY-LU-12, Restin, NY-CO-38, MAGE-1, MAGE-4a, SSX2, NY-ESO-1, SCP-1, 707-AP, AFP, ART-4, BAGE, b-Catenin/m, Bcr-abl, CAMEL, CAP-1, CASP-8, CDC27m, CDK4/m, CT, Cyp-B, DAM-6 (MAGE-B2) und DAM-10 (MAGE-B1), ELF2M, ETV6-AML1, G250, GAGE, GnT-V, Gp100, HAGE, HER-2/neu, HLA-A*0201-R170I, HPV-E7, HSP70-2M, HST-2, hTERT (hTRT), iCE, KIAA0205, LAGE, LDLR/FUT, MAGE, MART-1/Melan-A, MC1R, Myosin/m, MUC1, MUM-1, MUM-2, MUM-3, NA88-A, NY-ESO-1, P15, p190 minor bcr-abl, Pml/RARa, PRAME, RAGE, RU1, RU2, SAGE, SART-1, SART-3, TEL/AML1, TPI/m, TRP-1, Protein 1, gp75, TRP-2, TRP-2/INT2 und WT1, vorzugsweise wobei die TAA-spezifische T-Zelle einen chimären Antigenrezeptor (CAR) und/oder einen transgenen T-Zell-Rezeptor (TCR) exprimiert, vorzugsweise wobei das Zytokin ein Interferon (IFN), IL-12 oder Granulozyten-Makrophagen-Kolonie-Stimulierungsfaktor (GM-CSF) ist,

vorzugsweise wobei das IFN ein Typ-I-IFN (vorzugsweise IFN$\alpha$ oder IFN$\beta$) oder ein Typ-II-IFN (vorzugsweise IFN$\gamma$) ist,
wobei es sich stärker bevorzugt bei dem Typ-I-IFN um IFN$\alpha$ handelt.
vorzugsweise wobei die TAA-spezifische T-Zelle einen CD19-spezifischen CAR exprimiert.

**11.** TAA-spezifische T-Zelle zur Verwendung gemäß Anspruch 10, wobei der Vektor umfasst:

(i) mindestens eine mir-130a-Zielsequenz und mindestens eine mir-126-Zielsequenz, vorzugsweise wobei der Vektor zwei mir-130a-Zielsequenzen und zwei mir-126-Zielsequenzen umfasst; und/oder
(ii) einen gewebespezifischen Promotor in funktioneller Verknüpfung mit der für das Zytokin codierenden Nukleotidsequenz, vorzugsweise wobei der gewebespezifische Promotor ein TEK(Tie2)-Promotor ist.

**12.** TAA-spezifische T-Zelle zur Verwendung gemäß Anspruch 10 oder 11, wobei der Krebs eine hämatologische Malignität oder ein solider Tumor ist, vorzugsweise wobei die hämatologische Malignität ausgewählt ist aus der Gruppe bestehend aus akuter myeloischer Leukämie (AML), lymphoblastischer Leukämie, akuter lymphatischer Leukämie (ALL), myelodysplastischen Syndromen, Lymphom, multiplem Myelom, Non-Hodgkin-Lymphom und Hodgkin-Lymphom; oder vorzugsweise wobei der solide Tumor ausgewählt ist aus der Gruppe bestehend aus Lungenkrebs, Brustkrebs, Speiseröhrenkrebs, Magenkrebs, Dickdarmkrebs, Cholangiokarzinom, Bauchspeicheldrüsenkrebs, Eierstockkrebs, Krebs im Kopf-Hals-Bereich, Synovialsarkom, Angiosarkom, Osteosarkom, Schilddrüsenkrebs, Endometriumkrebs, Neuroblastom, Rhabdomyosarkom, Leberkrebs, Melanom, Prostatakrebs, Nierenkrebs, Weichteilsarkom, Urothelkrebs, Gallenkrebs, Glioblastom, Gebärmutterhalskrebs und Kolorektalkrebs.

**13.** Produkt, das umfasst: (a) eine hämatopoetische Stammzelle (HSC), eine hämatopoetische Progenitorzelle (HPC), eine Myeloid/Monozyten-festgelegte Progenitorzelle, einen Makrophagen oder einen Monozyt, umfassend einen Vektor, wobei der Vektor mindestens eine mir-130a- und/oder mir-126-Zielsequenz in funktioneller Verknüpfung mit einer für ein Zytokin codierenden Nukleotidsequenz umfasst; und (b) eine Tumor-assoziiertes-Antigen (TAA)-spezifische T-Zelle, vorzugsweise wobei das Zytokin ein Interferon (IFN), IL-12 oder Granulozyten-Makrophagen-Kolonie-Stimulierungsfaktor (GM-CSF) ist,

vorzugsweise wobei das IFN ein Typ-I-IFN (vorzugsweise IFNα oder IFNβ) oder ein Typ-II-IFN (vorzugsweise IFNγ) ist,
wobei es sich stärker bevorzugt bei dem Typ-I-IFN um IFNα handelt.

## Revendications

1. Cellule souche hématopoïétique (HSC), cellule progénitrice hématopoïétique (HPC), cellule progénitrice myéloïde/monocyte-engagée, macrophage ou monocyte comprenant un vecteur, le vecteur comprenant au moins une séquence cible mir-130a et/ou mir-126 liée de manière fonctionnelle à une séquence de nucléotides codant pour une cytokine pour une utilisation dans le traitement ou la prévention d'un cancer chez un patient, la HSC, la HPC, la cellule progénitrice myéloïde/monocyte-engagée, le macrophage ou le monocyte étant utilisé(e) en combinaison avec une cellule T spécifique à un antigène associé à une tumeur (TAA),

   préférablement, la cytokine étant un interféron (IFN), IL-12 ou un facteur de stimulation des colonies par granulocyte et macrophage (GM-CSF),
   préférablement l'IFN étant un IFN de type I (préférablement IFNα ou IFNβ), ou un IFN de type II (préférablement IFNγ),
   plus préférablement l'IFN de type I étant IFNα.

2. HSC, HPC, cellule progénitrice myéloïde/monocyte-engagée, macrophage ou monocyte pour une utilisation selon la revendication 1, la HSC, la HPC, la cellule progénitrice myéloïde/monocyte-engagée, le macrophage ou le monocyte étant en outre utilisé(e) en combinaison avec un inhibiteur de point de contrôle immunitaire.

3. HSC, HPC, cellule progénitrice myéloïde/monocyte-engagée, macrophage ou monocyte pour une utilisation selon la revendication 1 ou 2, la cellule T spécifique à TAA exprimant un récepteur antigénique chimérique (CAR) et/ou un récepteur de cellules T transgéniques (TCR).

4. HSC, HPC, cellule progénitrice myéloïde/monocyte-engagée, macrophage ou monocyte pour une utilisation selon l'une quelconque des revendications précédentes, le TAA étant choisi dans le groupe constitué par antigène carcinoembryonnaire (CEA), récepteur d'œstrogènes, récepteur de progestérone, éphrineB2, ROR1, mésothéline, c-Met, GD-2 et MAGE A3 TCR, 4-1BB, antigène d'adénocarcinome, alpha- foetoprotéine, BAFF, cellule de lymphome B, antigène C242, anhydrase carbonique 9 (CA-IX), CCR4, CD152, CD200, CD22, CD19, CD22, CD123, CD221, CD23 (récepteur IgE), CD28, CD4, CD40, CD44, CD44 v6, CD51, CD52, CD56, CD74, CD80, CS-1, CNT0888, CTLA-4, DR5, EpCAM, CD3, domaine supplémentaire B de la fibronectine, récepteur 1 du folate, glycoprotéine 75, GPNMB, HGF, récepteur kinase du facteur de dispersion humain, IGF-1 récepteur, IGF-I, IgGI, L1-CAM, IL-13, IL-6, récepteur du facteur de croissance analogue à l'insuline I, intégrine α5β1, intégrine αvβ3, MORAb-009, MS4A1, mucine CanAg, acide N-glycolylneuraminique, NPC-1C, PDGF-Rα, PDL192, phosphatidylsérine, cellules du carcinome prostatique, RANKL, RON, SCH 900105, SDC1, SLAMF7, ténascine C, TGF bêta 2, TGF-β, TRAIL-R1, TRAIL-R2, antigène tumoral CTAA16.88, croissance endothéliale vasculaire, facteur (VEGF), VEGF-A, VEGFR-1, VEGFR2, vimentine, 5T4, CD5, CD19, CD20, CD21, CD25, CD37, CD30, CD33, CD45, CAMPATH-1 (CDw52), HLA-DR, anti-idiotype, TAG-72, Ep-CAM, MUC1, protéine de liaison au folate, A33, G250, antigène membranaire spécifique de la prostate (PSMA), antigène spécifique de la prostate (PSA), ferritine, gangliosides (par exemple, GD2, GD3, GM2), $Le^y$, CA-125, CA19-9, récepteur du facteur de croissance épidermique (EGFR), p185HER2, récepteur de l'IL-2, de2-7 EGFR, protéine d'activation des fibroblastes (FAP), ténascine, métalloprotéinases, endosialine, anhydrase carbonique, galectine 9, aldolase A, eIFγ4, tyrosinase, galectine 4, HERKV-K10, p53, NY-LU-12, restine, NY-CO-38, MAGE-1, MAGE-4a, SSX2, NY-ESO-1, SCP-1, 707-AP, AFP, ART-4, BAGE, b-caténine/m, Bcr-abl, CAMEL, CAP-1, CASP-8, CDC27m, CDK4/m, CT, Cyp-B, DAM-6 (MAGE-B2) et DAM-10 (MAGE-B1), ELF2M, ETV6-AML1, G250, GAGE, GnT-V, Gp100, HAGE, HER-2/neu, HLA-A*0201-R170I, HPV-E7, HSP70-2M, HST-2, hTERT (hTRT), iCE, KIAA0205, LAGE, LDLR/FUT, MAGE, MART-1/Melan-A, MC1R, myosine/m, MUC1, MUM-1, MUM-2, MUM-3, NA88-A, NY-ESO-1, P15, p190 mineur bcr-abl, Pml/RARa, PRAME, RAGE, RU1, RU2, SAGE, SART-1, SART-3, TEL/AML1, TPI/m, TRP-1, protéine 1, gp75, TRP-2, TRP-2/INT2 et WT1, préférablement, la cellule T spécifique à TAA exprimant un CAR spécifique à CD19.

5. HSC, HPC, cellule progénitrice myéloïde/monocyte-engagée, macrophage ou monocyte pour une utilisation selon l'une quelconque des revendications précédentes, la cellule T spécifique à TAA étant dérivée d'une cellule isolée du patient.

6. HSC, HPC, cellule progénitrice myéloïde/monocyte-engagée, macrophage ou monocyte pour une utilisation selon l'une quelconque des revendications précédentes, la cellule T spécifique à TAA ayant été modifiée pour perturber au moins un gène endogène, préférablement, l'au moins un gène endogène étant choisi parmi un gène endogène codant une chaîne TCR a, une chaîne TCR β et un complexe majeur d'histocompatibilité (MHC).

7. HSC, HPC, cellule progénitrice myéloïde/monocyte-engagée, macrophage ou monocyte pour une utilisation selon l'une quelconque des revendications 2-6, l'inhibiteur du point de contrôle immunitaire :

(i) étant un anticorps, préférablement l'anticorps inhibiteur du point de contrôle immunitaire étant choisi dans le groupe constitué par un anticorps anti-CTLA4, un anticorps anti-PD1, un anticorps anti-PDL1, un anticorps anti-PDL2 et un anticorps anti-LAG-3 ; et/ou
(ii) inhibant une molécule de point de contrôle inhibiteur choisie dans le groupe constitué par A2AR (récepteur de l'adénosine A2A), B7-H3 (CD276), B7-H4 (VTCN1), BTLA (atténuateur des lymphocytes B et T ; CD272), HVEM (médiateur d'entrée du virus de l'herpès), CTLA-4 (protéine 4 associée aux lymphocytes T cytotoxiques ; CD152), IDO (indoleamine 2,3-dioxygénase), TDO (tryptophane 2,3-dioxygénase), KIR (récepteur de type immunoglobuline des cellules tueuses), LAG3 (gène d'activation des lymphocytes 3), PD-1 (récepteur de la mort programmée 1), PD-L1 (ligand 1 de PD-1), PD-L2 (ligand 2 de PD-1), TIM-3 (domaine d'immunoglobuline des cellules T et domaine 3 de la mucine), VISTA (suppresseur d'activation des cellules T du domaine V Ig), B7-1 (CD80) et B7-2 (CD86).

8. HSC, HPC, cellule progénitrice myéloïde/monocyte-engagée, macrophage ou monocyte pour une utilisation selon l'une quelconque des revendications précédentes, le vecteur comprenant :

(i) au moins une séquence cible mir-130a et au moins une séquence cible mir-126, préférablement le vecteur comprenant deux séquences cibles mir-130a et deux séquences cibles mir-126 ; et/ou
(ii) un promoteur spécifique à un tissu lié de manière fonctionnelle à la séquence de nucléotides codant pour la cytokine, préférablement le promoteur spécifique au tissu étant un promoteur TEK (Tie2).

9. HSC, HPC, cellule progénitrice myéloïde/monocyte-engagée, macrophage ou monocyte pour une utilisation selon une quelconque revendication précédente, le cancer étant une malignité hématologique ou une tumeur solide, préférablement, la malignité hématologique étant choisie dans le groupe constitué par la leucémie myéloïde aiguë (LMA), la leucémie lymphoblastique, la leucémie aiguë lymphoblastique (LAL), les syndromes myélodysplasiques (SMD), les néoplasmes myéloprolifératifs (NMP), la myélofibrose primaire, la thrombocytémie essentielle, la polycythémie vraie, la leucémie myéloïde chronique atypique, la leucémie myéloïde chronique (LMC), le lymphome, le myélome multiple, le lymphome non hodgkinien et le lymphome de Hodgkin ; ou préférablement, la tumeur solide étant choisie dans le groupe constitué par le cancer du poumon, le cancer du sein, le cancer de l'œsophage, le cancer gastrique, le cancer du côlon, le cholangiocarcinome, le cancer du pancréas, le cancer de l'ovaire, les cancers de la tête et du cou, le sarcome synovial, l'angiosarcome, l'ostéosarcome, le cancer de la thyroïde, le cancer de l'endomètre, le neuroblastome, le rabdomyosarcome, le cancer du foie, le mélanome, le cancer de la prostate, le cancer du rein, le sarcome des tissus mous, le cancer urothélial, le cancer biliaire, le glioblastome, le cancer du col de l'utérus et le cancer colorectal.

10. Cellule T spécifique à un antigène associé à une tumeur (TAA) pour une utilisation dans le traitement ou la prévention d'un cancer chez un patient, le patient ayant précédemment été administré avec une cellule souche hématopoïétique (HSC), une cellule progénitrice hématopoïétique (HPC), une cellule progénitrice myéloïde/monocyte-engagée, un macrophage ou un monocyte comprenant un vecteur, le vecteur comprenant au moins une séquence cible mir-130a et/ou mir-126 liée de manière fonctionnelle à une séquence de nucléotides codant pour une cytokine, préférablement le TAA étant choisi dans le groupe constitué par antigène carcinoembryonnaire (ACE), récepteur d'œstrogènes, récepteur de progestérone, éphrineB2, ROR1, mésothéline, c-Met, GD-2 et MAGE A3 TCR, 4-1BB, antigène d'adénocarcinome, alpha-fœtoprotéine, BAFF, cellule de lymphome B, antigène C242, anhydrase carbonique 9 (CA-IX), CCR4, CD152, CD200, CD22, CD19, CD22, CD123, CD221, CD23 (récepteur IgE), CD28, CD4, CD40, CD44, CD44 v6, CD51, CD52, CD56, CD74, CD80, CS-1, CNT0888, CTLA-4, DR5, EpCAM, CD3, domaine supplémentaire B de la fibronectine, récepteur 1 du folate, glycoprotéine 75, GPNMB, HGF, récepteur kinase du facteur de dispersion humain, IGF-1 récepteur, IGF-I, IgGI, L1-CAM, IL-13, IL-6, récepteur du facteur de croissance analogue à l'insuline I, intégrine α5β1, intégrine αvβ3, MORAb-009, MS4A1, mucine CanAg, acide N-glycolylneuraminique, NPC-1C, PDGF-Rα, PDL192, phosphatidylsérine, cellules du carcinome prostatique, RANKL, RON, SCH 900105, SDC1, SLAMF7, ténascine C, TGF bêta 2, TGF-β, TRAIL-R1, TRAIL-R2, antigène tumoral CTAA16.88, croissance endothéliale vasculaire, facteur (VEGF), VEGF-A, VEGFR-1, VEGFR2, vimentine, 5T4, CD5, CD19,

CD20, CD21, CD25, CD37, CD30, CD33, CD45, CAMPATH-1 (CDw52), HLA-DR, anti-idiotype, TAG-72, Ep-CAM, MUC1, protéine de liaison au folate, A33, G250, antigène membranaire spécifique de la prostate (PSMA), antigène spécifique de la prostate (PSA), ferritine, gangliosides (par exemple, GD2, GD3, GM2), $Le^y$, CA-125, CA19-9, récepteur du facteur de croissance épidermique (EGFR), p185HER2, récepteur de l'IL-2, de2-7 EGFR, protéine d'activation des fibroblastes (FAP), ténascine, métalloprotéinases, endosialine, anhydrase carbonique, galectine 9, aldolase A, elFy4, tyrosinase, galectine 4, HERKV-K10, p53, NY-LU-12, restine, NY-CO-38, MAGE-1, MAGE-4a, SSX2, NY-ESO-1, SCP-1, 707-AP, AFP, ART-4, BAGE, b-caténine/m, Bcr-abl, CAMEL, CAP-1, CASP-8, CDC27m, CDK4/m, CT, Cyp-B, DAM-6 (MAGE-B2) et DAM-10 (MAGE-B1), ELF2M, ETV6-AML1, G250, GAGE, GnT-V, Gp100, HAGE, HER-2/neu, HLA-A*0201-R170I, HPV-E7, HSP70-2M, HST-2, hTERT (hTRT), iCE, KIAA0205, LAGE, LDLR/FUT, MAGE, MART-1/Melan-A, MC1R, myosine/m, MUC1, MUM-1, MUM-2, MUM-3, NA88-A, NY-ESO-1, P15, p190 mineur bcr-abl, Pml/RARa, PRAME, RAGE, RU1, RU2, SAGE, SART-1, SART-3, TEL/AML1, TPI/m, TRP-1, protéine 1, gp75, TRP-2, TRP-2/INT2 et WT1, préférablement dans lequel la cellule T spécifique à TAA exprime un récepteur d'antigène chimérique (CAR) et/ou un récepteur de cellules T transgéniques (TCR), préférablement, la cytokine étant un interféron (IFN), IL-12 ou un facteur de stimulation des colonies par granulocyte et macrophage (GM-CSF),

préférablement l'IFN étant un IFN de type I (préférablement IFNa ou IFN$\beta$), ou un IFN de type II (préférablement IFN$\gamma$),
préférablement l'IFN de type I étant IFNa,
préférablement, la cellule T spécifique à TAA exprimant un CAR spécifique à CD19.

**11.** Cellule T spécifique à TAA pour une utilisation selon la revendication 10, le vecteur comprenant :

(i) au moins une séquence cible mir-130a et au moins une séquence cible mir-126, préférablement le vecteur comprenant deux séquences cibles mir-130a et deux séquences cibles mir-126 ; et/ou
(ii) un promoteur spécifique à un tissu lié de manière fonctionnelle à la séquence de nucléotides codant pour la cytokine, préférablement le promoteur spécifique au tissu étant un promoteur TEK (Tie2).

**12.** Cellule T spécifique à TAA pour une utilisation selon la revendication 10 ou 11, le cancer étant une malignité hématologique ou une tumeur solide, préférablement, la malignité hématologique étant choisie dans le groupe constitué par la leucémie myéloïde aiguë (LMA), la leucémie lymphoblastique, la leucémie aiguë lymphoblastique (LAL), les syndromes myélodysplasiques, le lymphome, le myélome multiple, le lymphome non hodgkinien et le lymphome de Hodgkin ; ou préférablement, la tumeur solide étant choisie dans le groupe constitué par le cancer du poumon, le cancer du sein, le cancer de l'œsophage, le cancer gastrique, le cancer du côlon, le cholangiocarcinome, le cancer du pancréas, le cancer de l'ovaire, les cancers de la tête et du cou, le sarcome synovial, l'angiosarcome, l'ostéosarcome, le cancer de la thyroïde, le cancer de l'endomètre, le neuroblastome, le rabdomyosarcome, le cancer du foie, le mélanome, le cancer de la prostate, le cancer du rein, le sarcome des tissus mous, le cancer urothélial, le cancer biliaire, le glioblastome, le cancer du col de l'utérus et le cancer colorectal.

**13.** Produit comprenant : (a) une cellule souche hématopoïétique (HSC), une cellule progénitrice hématopoïétique (HPC), une cellule progénitrice myéloïde/monocyte-engagée, un macrophage ou un monocyte comprenant un vecteur, le vecteur comprenant au moins une séquence cible mir-130a et/ou mir-126 liée de manière fonctionnelle à une séquence de nucléotides codant pour une cytokine ; et (b) une cellule T spécifique à un antigène associé à une tumeur (TAA),

préférablement, la cytokine étant un interféron (IFN), IL-12 ou un facteur de stimulation des colonies par granulocyte et macrophage (GM-CSF),
préférablement l'IFN étant un IFN de type I (préférablement IFNa ou IFN$\beta$), ou un IFN de type II (préférablement IFN$\gamma$),
plus préférablement l'IFN de type I étant IFNa.

# Figure 1

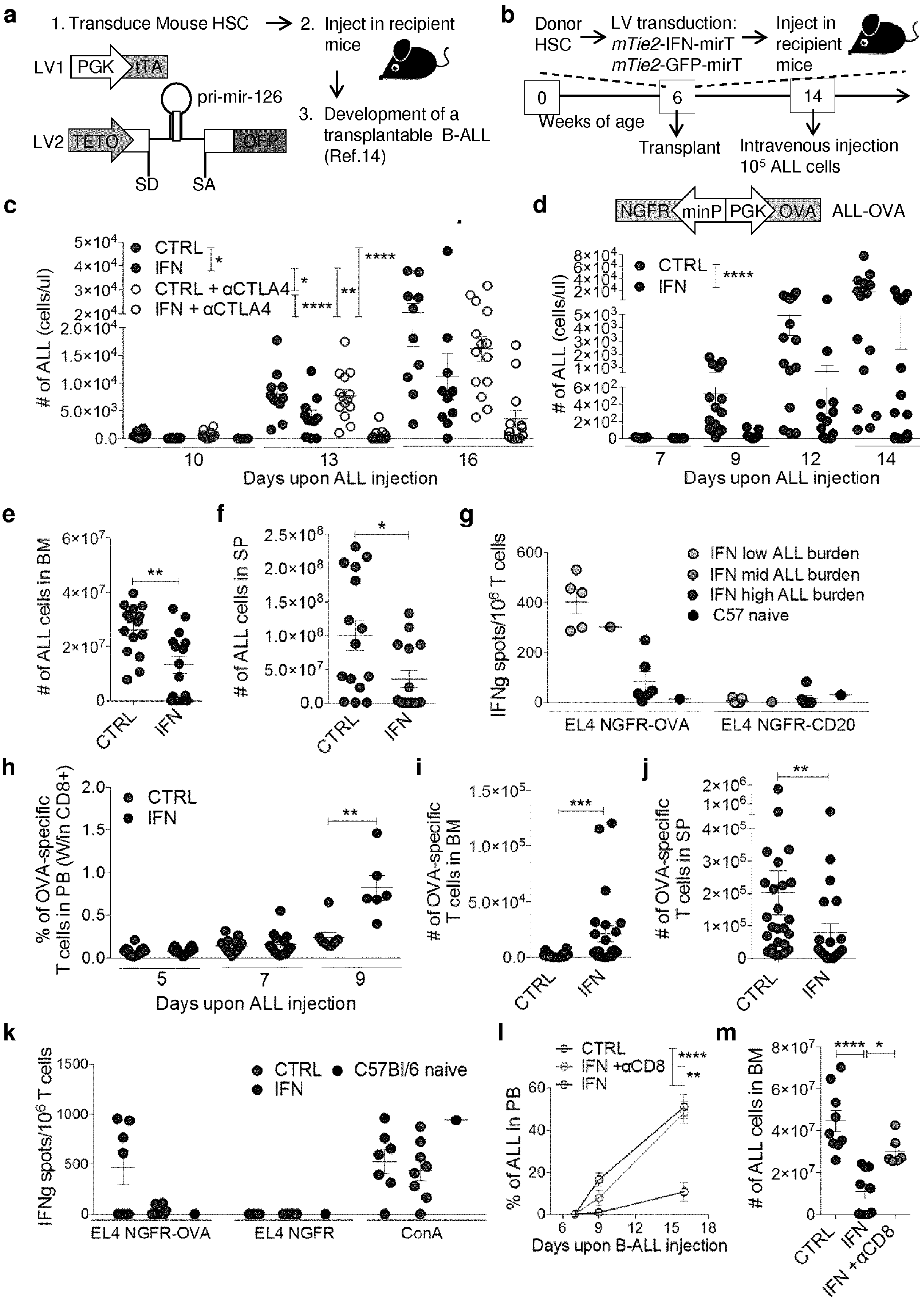
a
1. Transduce Mouse HSC
2. Inject in recipient mice
3. Development of a transplantable B-ALL (Ref.14)
LV1 PGK tTA
LV2 TETO OFP
pri-mir-126
SD
SA
b
Donor HSC
LV transduction: mTie2-IFN-mirT mTie2-GFP-mirT
Inject in recipient mice
Weeks of age
Transplant
Intravenous injection $10^5$ ALL cells
c
CTRL
IFN
CTRL + αCTLA4
IFN + αCTLA4
# of ALL (cells/ul)
Days upon ALL injection
d
NGFR minP PGK OVA ALL-OVA
e
# of ALL cells in BM
f
# of ALL cells in SP
g
IFNg spots/$10^6$ T cells
IFN low ALL burden
IFN mid ALL burden
IFN high ALL burden
C57 naive
EL4 NGFR-OVA
EL4 NGFR-CD20
h
% of OVA-specific T cells in PB (W/in CD8+)
i
# of OVA-specific T cells in BM
j
# of OVA-specific T cells in SP
k
C57Bl/6 naive
EL4 NGFR
ConA
l
IFN +αCD8
% of ALL in PB
Days upon B-ALL injection
m
IFN +αCD8

## Figure 2

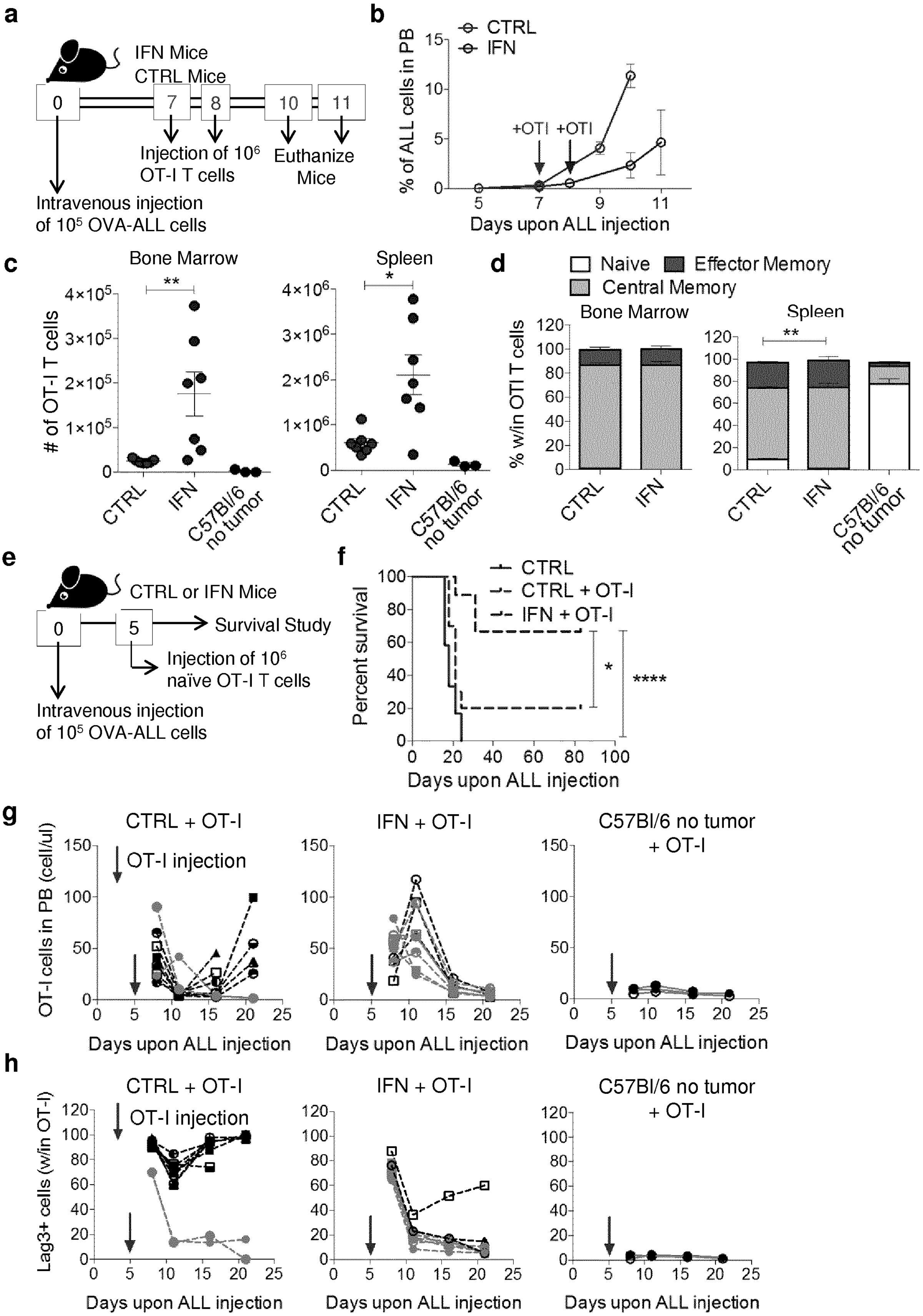
a
IFN Mice
CTRL Mice
0
7
8
10
11
Injection of 10^6 OT-I T cells
Euthanize Mice
Intravenous injection of 10^5 OVA-ALL cells
b
CTRL
IFN
% of ALL cells in PB
+OTI +OTI
Days upon ALL injection
c
Bone Marrow
Spleen
# of OT-I T cells
CTRL
IFN
C57Bl/6 no tumor
d
Naive
Effector Memory
Central Memory
Bone Marrow
Spleen
% w/in OTI T cells
e
CTRL or IFN Mice
0
5
Survival Study
Injection of 10^6 naïve OT-I T cells
Intravenous injection of 10^5 OVA-ALL cells
f
CTRL
CTRL + OT-I
IFN + OT-I
Percent survival
Days upon ALL injection
g
CTRL + OT-I
IFN + OT-I
C57Bl/6 no tumor + OT-I
OT-I injection
OT-I cells in PB (cell/ul)
Days upon ALL injection
h
CTRL + OT-I
IFN + OT-I
C57Bl/6 no tumor + OT-I
OT-I injection
Lag3+ cells (w/in OT-I)
Days upon ALL injection

Figure 3

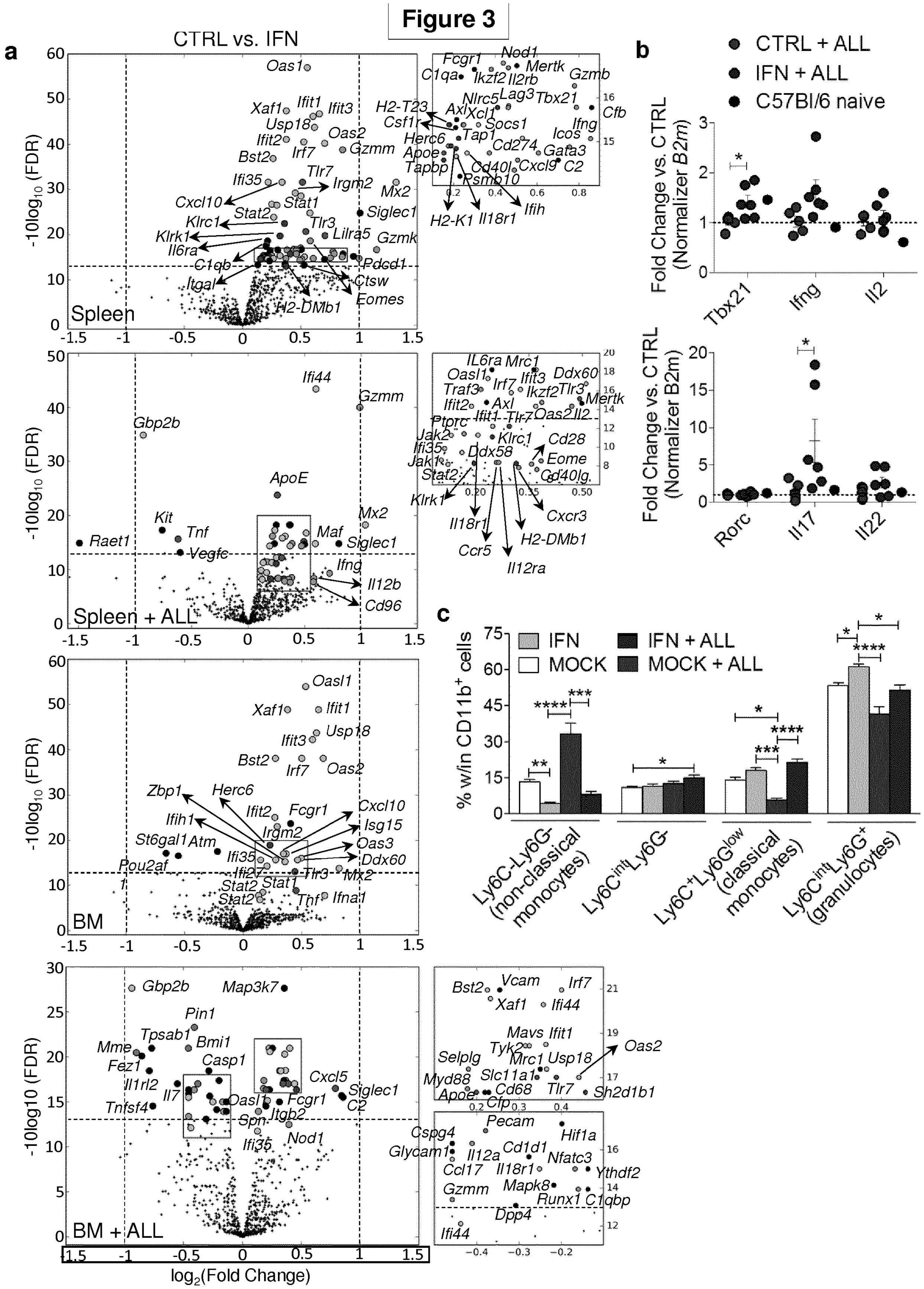
a
CTRL vs. IFN
-10log10 (FDR)
Spleen
Spleen + ALL
BM
BM + ALL
log2(Fold Change)
b
CTRL + ALL
IFN + ALL
C57Bl/6 naive
Fold Change vs. CTRL (Normalizer B2m)
Tbx21
Ifng
Il2
Rorc
Il17
Il22
c
IFN
MOCK
IFN + ALL
MOCK + ALL
% w/in CD11b+ cells
Ly6C-Ly6G- (non-classical monocytes)
Ly6CintLy6G-
Ly6C+Ly6Glow (classical monocytes)
Ly6CintLy6G+ (granulocytes)

## Figure 4

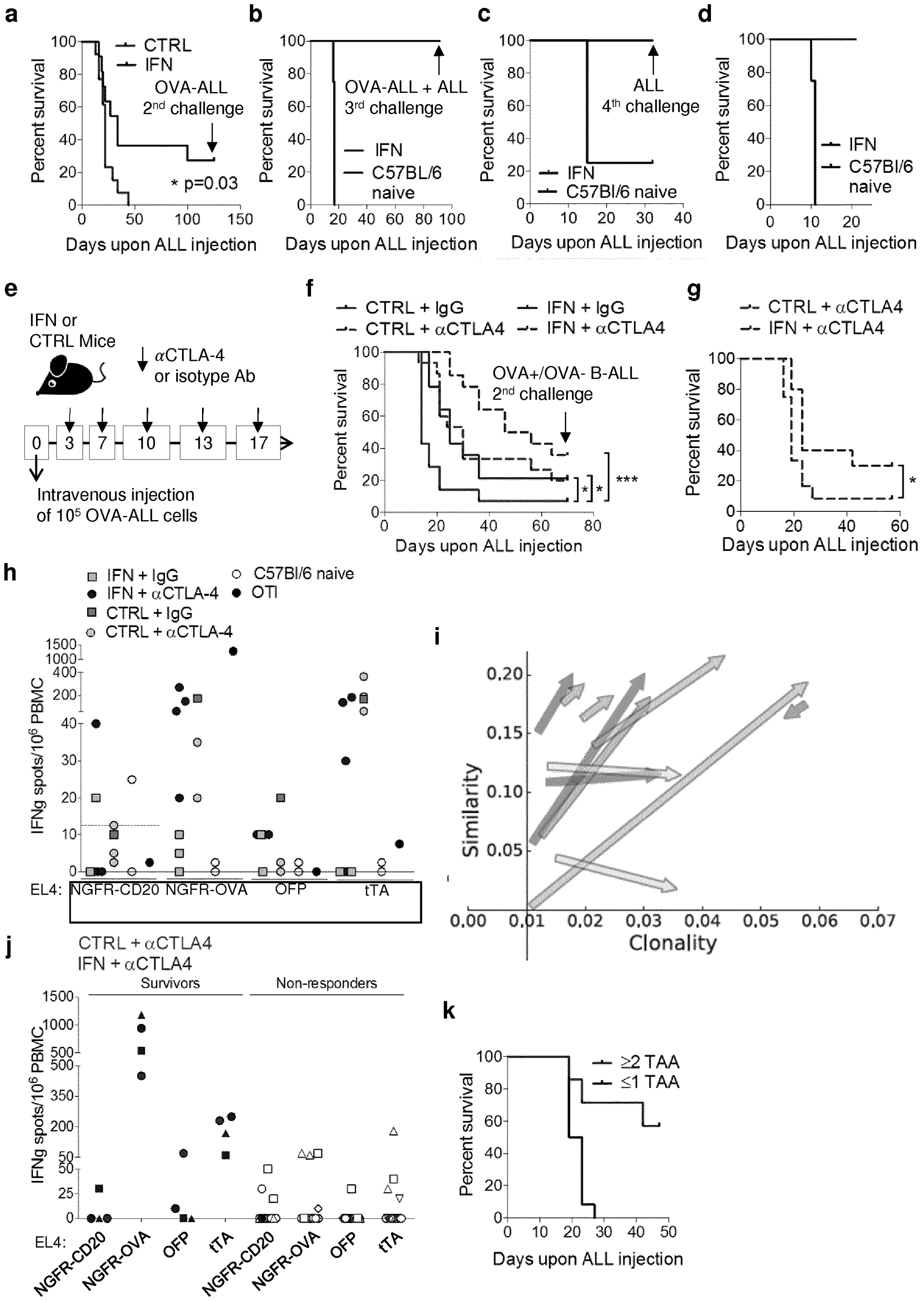

a
CTRL
IFN
OVA-ALL
2nd challenge
* p=0.03
Percent survival
Days upon ALL injection
b
OVA-ALL + ALL
3rd challenge
IFN
C57BL/6 naive
c
ALL
4th challenge
IFN
C57Bl/6 naive
d
IFN
C57Bl/6 naive
e
IFN or CTRL Mice
αCTLA-4 or isotype Ab
0 3 7 10 13 17
Intravenous injection of 10^5 OVA-ALL cells
f
CTRL + IgG
IFN + IgG
CTRL + αCTLA4
IFN + αCTLA4
OVA+/OVA- B-ALL
2nd challenge
g
CTRL + αCTLA4
IFN + αCTLA4
h
IFN + IgG
IFN + αCTLA-4
CTRL + IgG
CTRL + αCTLA-4
C57Bl/6 naive
OTI
IFNg spots/10^6 PBMC
EL4: NGFR-CD20 NGFR-OVA OFP tTA
i
Similarity
Clonality
j
CTRL + αCTLA4
IFN + αCTLA4
Survivors
Non-responders
IFNg spots/10^6 PBMC
EL4: NGFR-CD20 NGFR-OVA OFP tTA NGFR-CD20 NGFR-OVA OFP tTA
k
≥2 TAA
≤1 TAA
Percent survival
Days upon ALL injection

## Figure 5

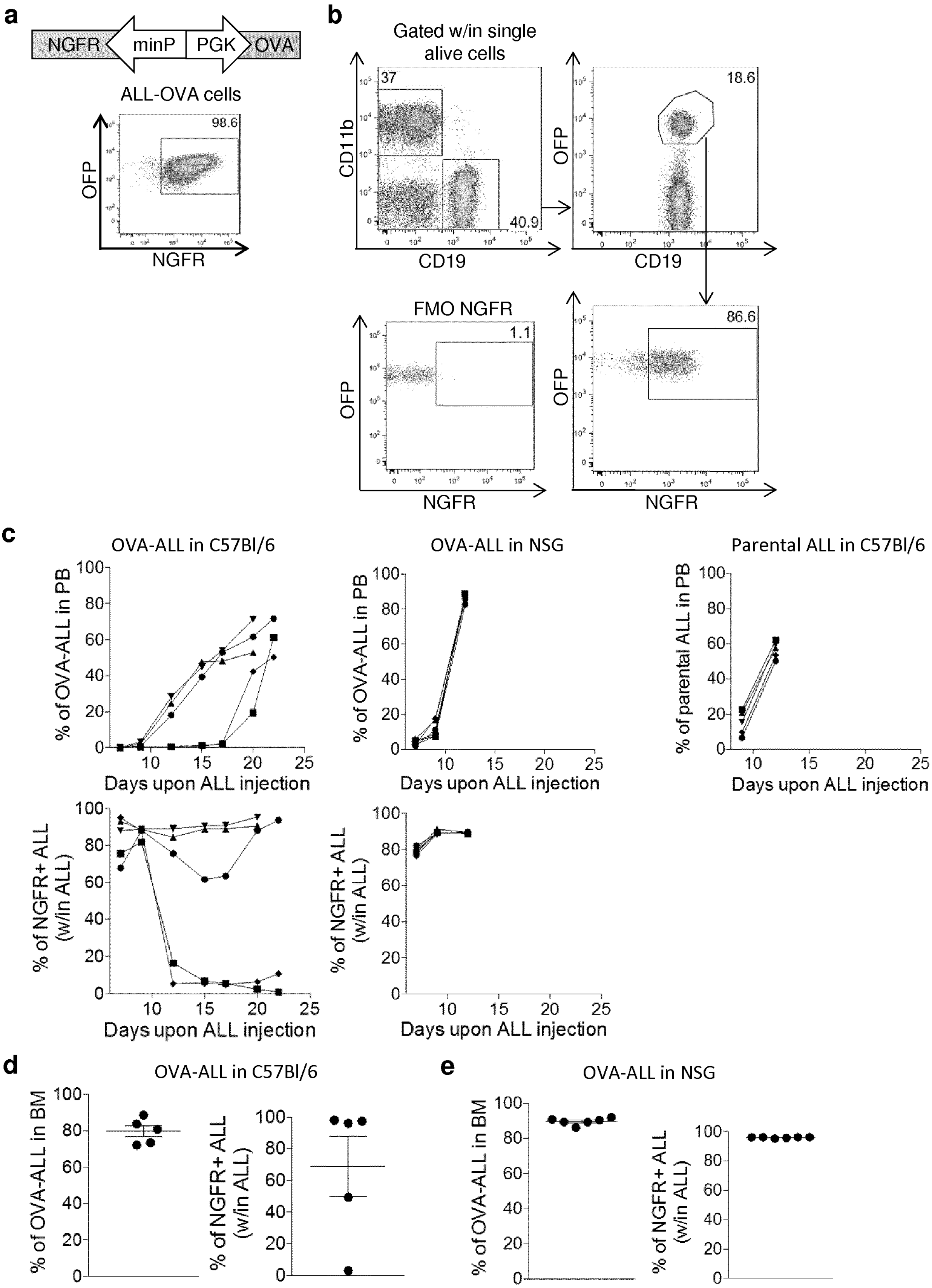
a
NGFR
minP
PGK
OVA
ALL-OVA cells
98.6
OFP
NGFR
b
Gated w/in single alive cells
37
40.9
CD11b
CD19
18.6
OFP
CD19
FMO NGFR
1.1
OFP
NGFR
86.6
OFP
NGFR
c
OVA-ALL in C57Bl/6
% of OVA-ALL in PB
Days upon ALL injection
OVA-ALL in NSG
% of OVA-ALL in PB
Days upon ALL injection
Parental ALL in C57Bl/6
% of parental ALL in PB
Days upon ALL injection
% of NGFR+ ALL (w/in ALL)
Days upon ALL injection
% of NGFR+ ALL (w/in ALL)
Days upon ALL injection
d
OVA-ALL in C57Bl/6
% of OVA-ALL in BM
% of NGFR+ ALL (w/in ALL)
e
OVA-ALL in NSG
% of OVA-ALL in BM
% of NGFR+ ALL (w/in ALL)

# Figure 6

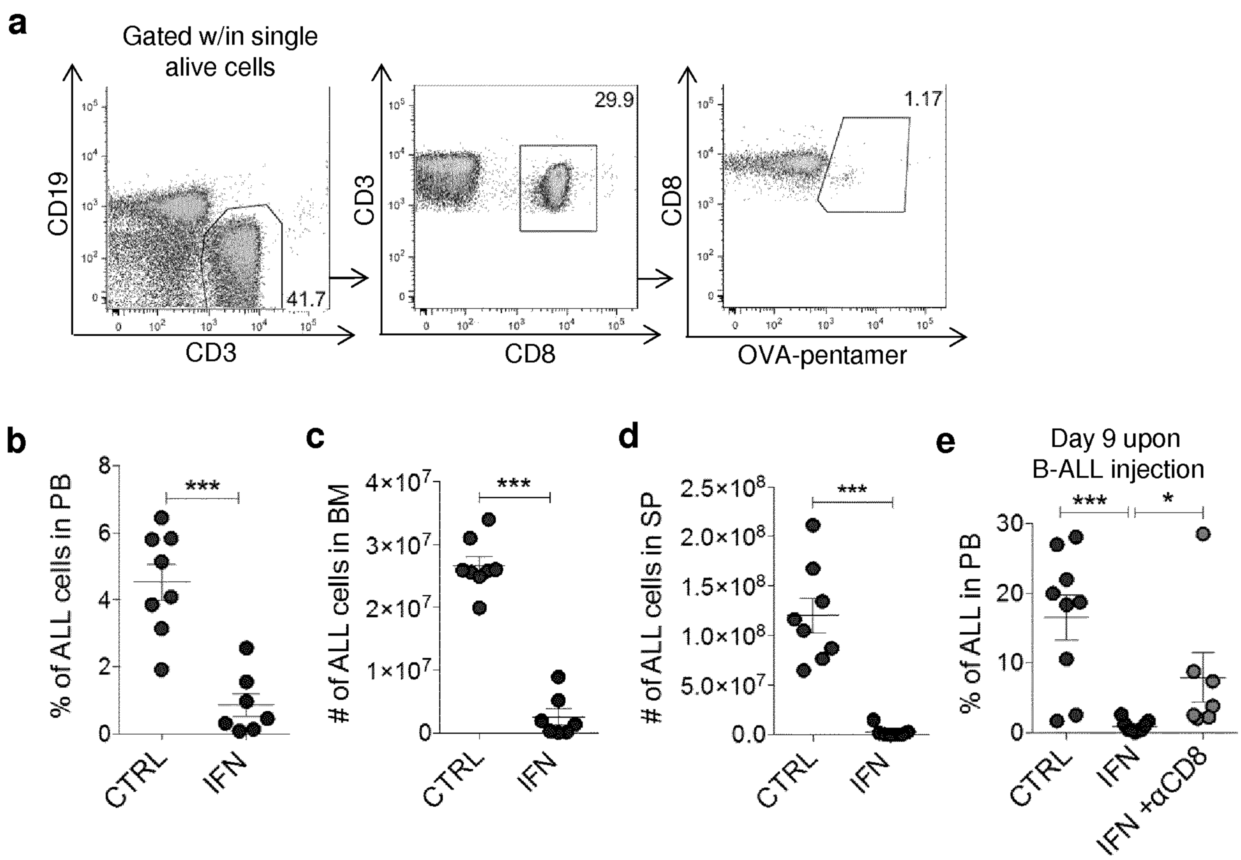
a
Gated w/in single alive cells
CD19
CD3
41.7
CD3
CD8
29.9
CD8
OVA-pentamer
1.17
b
% of ALL cells in PB
***
CTRL
IFN
c
# of ALL cells in BM
***
CTRL
IFN
d
# of ALL cells in SP
***
CTRL
IFN
e
Day 9 upon B-ALL injection
% of ALL in PB
***
*
CTRL
IFN
IFN +αCD8

# Figure 7

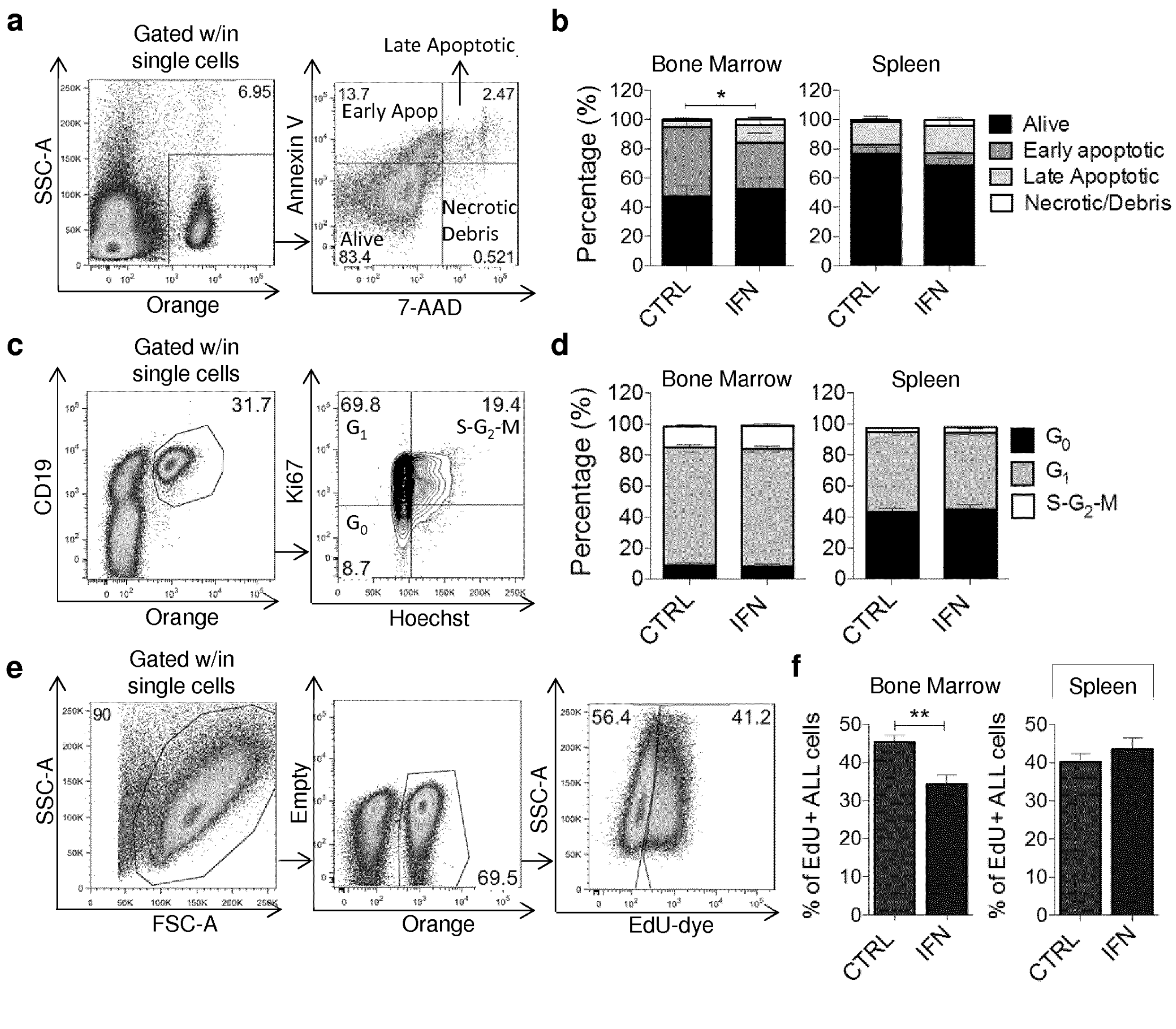
a
Gated w/in single cells
SSC-A
Orange
6.95
Late Apoptotic
Annexin V
7-AAD
13.7
Early Apop
2.47
Alive
83.4
Necrotic
Debris
0.521
b
Bone Marrow
Spleen
Percentage (%)
CTRL
IFN
*
Alive
Early apoptotic
Late Apoptotic
Necrotic/Debris
c
Gated w/in single cells
CD19
Orange
31.7
Ki67
Hoechst
69.8
G1
19.4
S-G2-M
G0
8.7
d
Bone Marrow
Spleen
Percentage (%)
G0
G1
S-G2-M
e
Gated w/in single cells
SSC-A
FSC-A
90
Empty
Orange
69.5
SSC-A
EdU-dye
56.4
41.2
f
Bone Marrow
Spleen
% of EdU+ALL cells
**
CTRL
IFN

# Figure 8

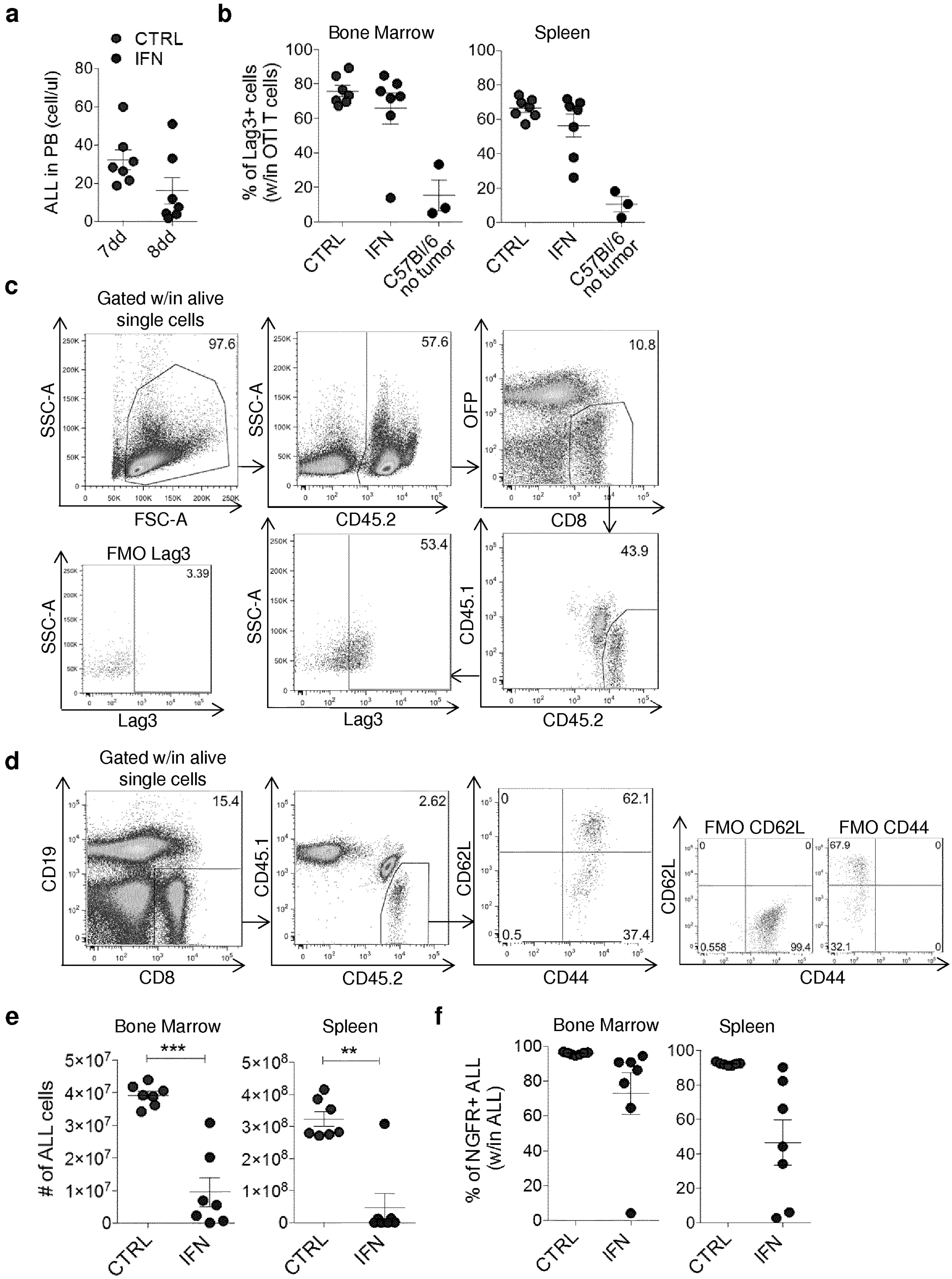
a
CTRL
IFN
ALL in PB (cell/ul)
7dd
8dd
b
Bone Marrow
Spleen
% of Lag3+ cells (w/in OTI T cells)
CTRL
IFN
C57Bl/6 no tumor
c
Gated w/in alive single cells
SSC-A
FSC-A
97.6
CD45.2
57.6
OFP
CD8
10.8
FMO Lag3
3.39
Lag3
53.4
CD45.1
43.9
d
CD19
15.4
CD45.1
2.62
CD62L
CD44
62.1
37.4
0.5
FMO CD62L
FMO CD44
0.558
99.4
67.9
32.1
e
# of ALL cells
***
**
f
% of NGFR+ ALL (w/in ALL)

# Figure 9

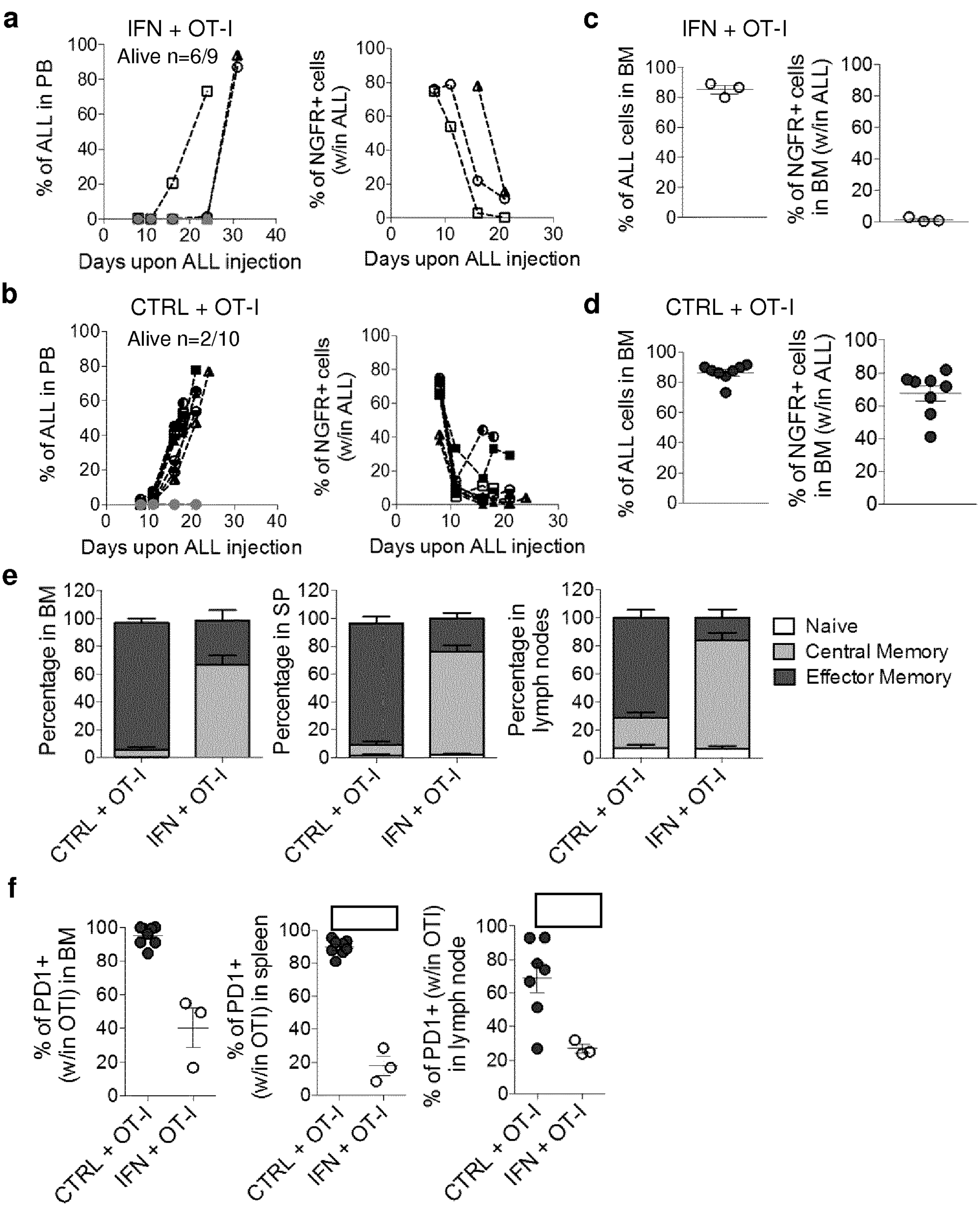
a
IFN + OT-I
Alive n=6/9
% of ALL in PB
Days upon ALL injection
% of NGFR+ cells (w/in ALL)
Days upon ALL injection
b
CTRL + OT-I
Alive n=2/10
% of ALL in PB
Days upon ALL injection
% of NGFR+ cells (w/in ALL)
Days upon ALL injection
c
IFN + OT-I
% of ALL cells in BM
% of NGFR+ cells in BM (w/in ALL)
d
CTRL + OT-I
% of ALL cells in BM
% of NGFR+ cells in BM (w/in ALL)
e
Percentage in BM
Percentage in SP
Percentage in lymph nodes
CTRL + OT-I
IFN + OT-I
Naive
Central Memory
Effector Memory
f
% of PD1+ (w/in OTI) in BM
% of PD1+ (w/in OTI) in spleen
% of PD1+ (w/in OTI) in lymph node
CTRL + OT-I
IFN + OT-I

# Figure 10

a

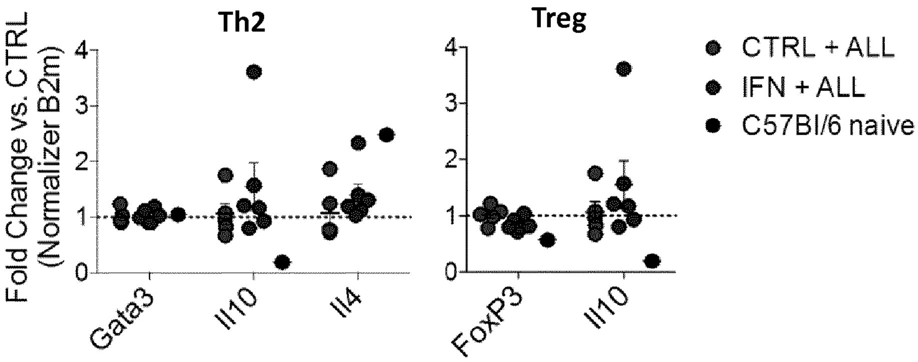
Th2
Treg
Fold Change vs. CTRL (Normalizer B2m)
4
3
2
1
0
Gata3
Il10
Il4
FoxP3
Il10
CTRL + ALL
IFN + ALL
C57Bl/6 naive

# Figure 11

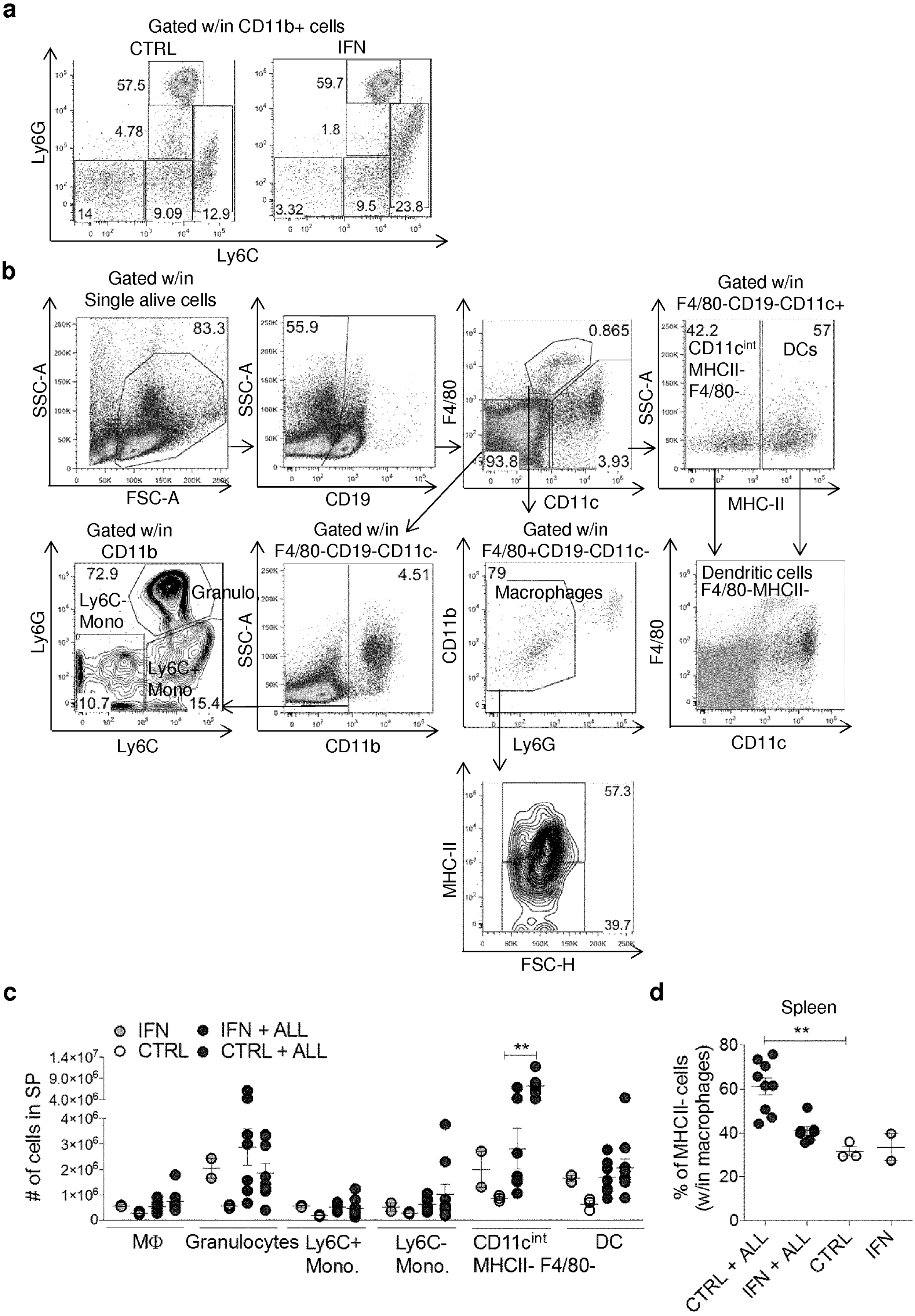
a
Gated w/in CD11b+ cells
CTRL
IFN
Ly6G
Ly6C
57.5
4.78
14
9.09
12.9
59.7
1.8
3.32
9.5
23.8
b
Gated w/in
Single alive cells
SSC-A
FSC-A
83.3
55.9
CD19
F4/80
CD11c
0.865
93.8
3.93
Gated w/in
F4/80-CD19-CD11c+
42.2
CD11c^int
MHCII-
F4/80-
57
DCs
MHC-II
Gated w/in
CD11b
72.9
Ly6C-
Mono
Granulo
Ly6C+
Mono
10.7
15.4
Gated w/in
F4/80-CD19-CD11c-
4.51
CD11b
Gated w/in
F4/80+CD19-CD11c-
79
Macrophages
Ly6G
Dendritic cells
F4/80-MHCII-
57.3
39.7
FSC-H
c
IFN
CTRL
IFN + ALL
CTRL + ALL
# of cells in SP
MΦ
Granulocytes
Ly6C+ Mono.
Ly6C- Mono.
CD11c^int MHCII- F4/80-
DC
d
Spleen
% of MHCII- cells (w/in macrophages)
CTRL + ALL
IFN + ALL
CTRL
IFN

# Figure 12

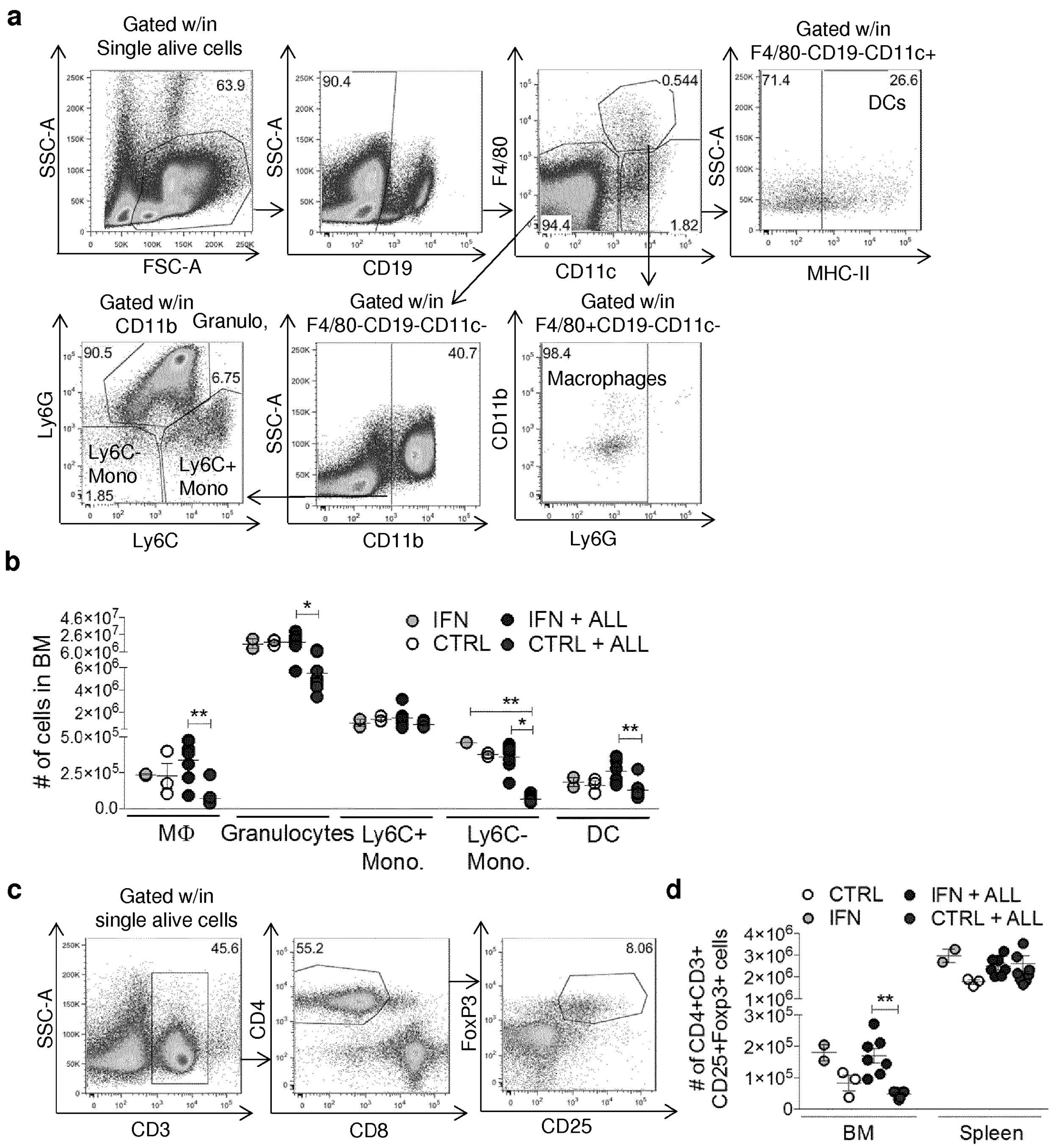
a
Gated w/in Single alive cells
SSC-A
FSC-A
63.9
90.4
CD19
F4/80
CD11c
0.544
94.4
1.82
Gated w/in F4/80-CD19-CD11c+
71.4
26.6
DCs
MHC-II
Gated w/in CD11b
Granulo,
90.5
6.75
Ly6G
Ly6C-
Mono
Ly6C+
Mono
1.85
Ly6C
Gated w/in F4/80-CD19-CD11c-
40.7
CD11b
Gated w/in F4/80+CD19-CD11c-
98.4
Macrophages
Ly6G
b
# of cells in BM
IFN
CTRL
IFN + ALL
CTRL + ALL
MΦ
Granulocytes
Ly6C+ Mono.
Ly6C- Mono.
DC
c
Gated w/in single alive cells
45.6
CD3
55.2
CD4
CD8
8.06
FoxP3
CD25
d
CTRL
IFN
IFN + ALL
CTRL + ALL
# of CD4+CD3+ CD25+Foxp3+ cells
BM
Spleen

# Figure 13

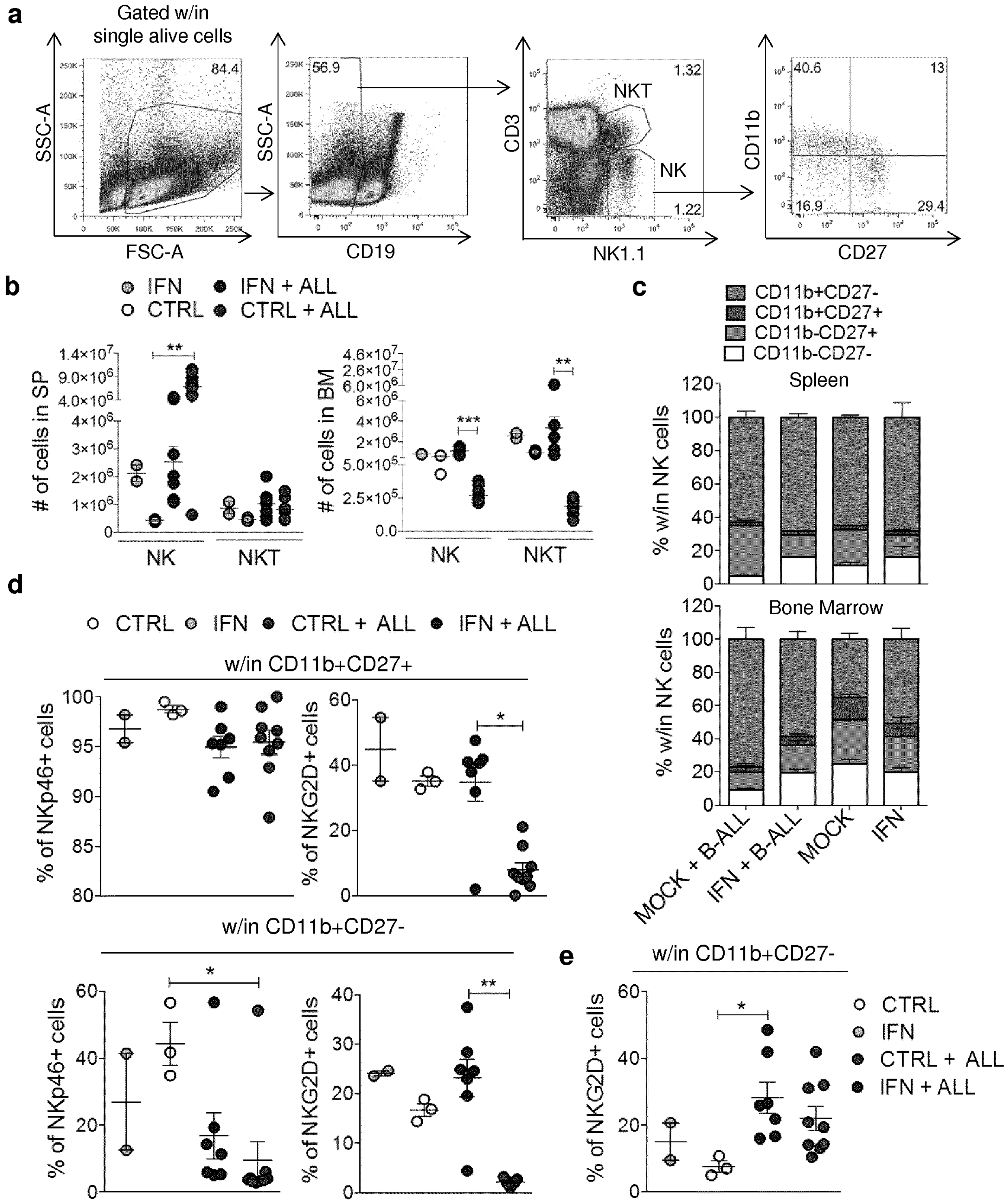
a
Gated w/in single alive cells
SSC-A
FSC-A
84.4
SSC-A
CD19
56.9
CD3
NK1.1
NKT
NK
1.32
1.22
CD11b
CD27
40.6
13
16.9
29.4
b
IFN
CTRL
IFN + ALL
CTRL + ALL
# of cells in SP
# of cells in BM
NK
NKT
c
CD11b+CD27-
CD11b+CD27+
CD11b-CD27+
CD11b-CD27-
Spleen
Bone Marrow
% w/in NK cells
MOCK + B-ALL
IFN + B-ALL
MOCK
IFN
d
CTRL
IFN
CTRL + ALL
IFN + ALL
w/in CD11b+CD27+
% of NKp46+ cells
% of NKG2D+ cells
w/in CD11b+CD27-
e
w/in CD11b+CD27-
% of NKG2D+ cells
CTRL
IFN
CTRL + ALL
IFN + ALL

# Figure 14

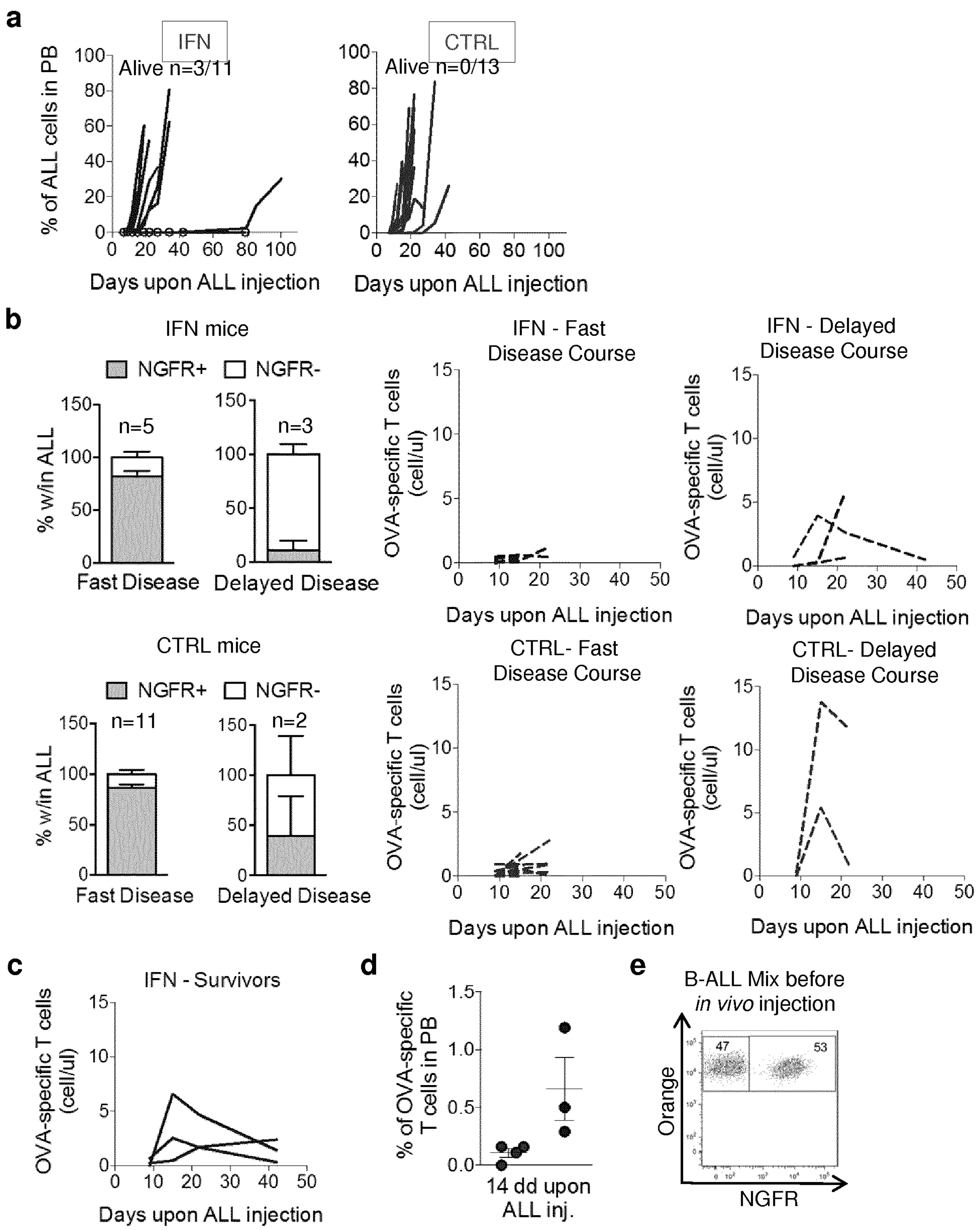
a
IFN
Alive n=3/11
% of ALL cells in PB
Days upon ALL injection
CTRL
Alive n=0/13
b
IFN mice
NGFR+
NGFR-
% w/in ALL
n=5
n=3
Fast Disease
Delayed Disease
IFN - Fast Disease Course
IFN - Delayed Disease Course
OVA-specific T cells (cell/ul)
CTRL mice
n=11
n=2
CTRL- Fast Disease Course
CTRL- Delayed Disease Course
c
IFN - Survivors
d
% of OVA-specific T cells in PB
14 dd upon ALL inj.
e
B-ALL Mix before *in vivo* injection
47
53
Orange
NGFR

# Figure 15

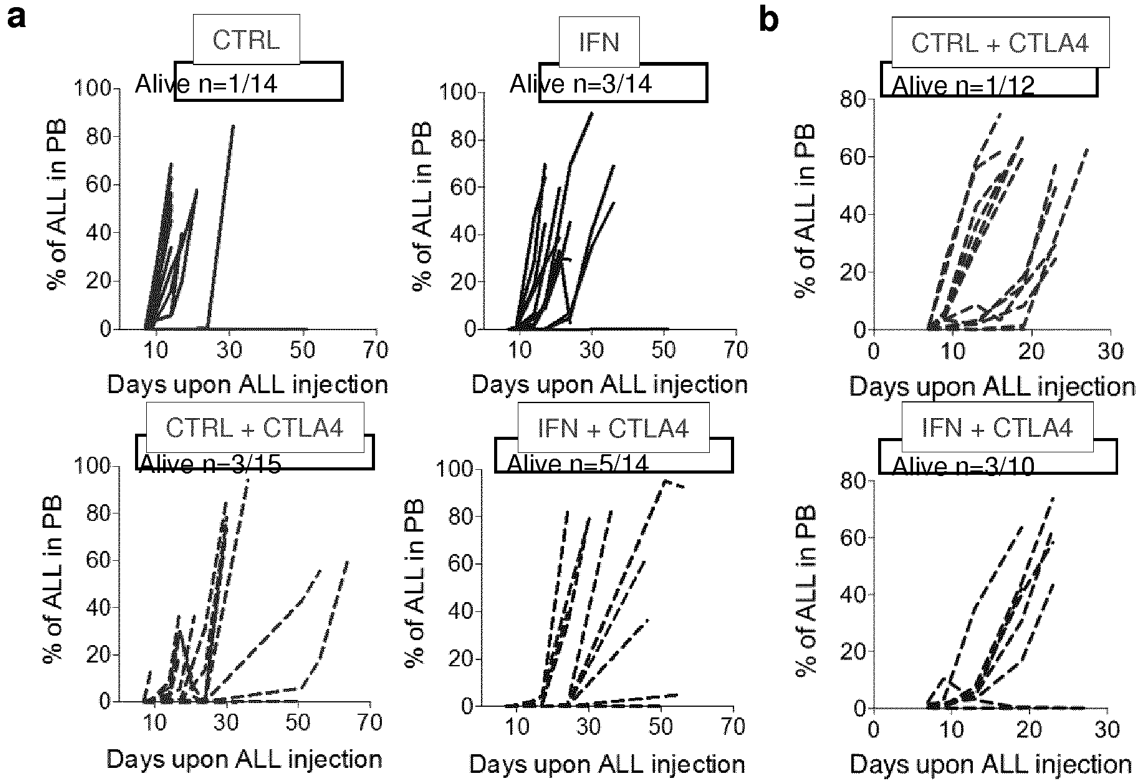
a
CTRL
Alive n=1/14
IFN
Alive n=3/14
CTRL + CTLA4
Alive n=3/15
IFN + CTLA4
Alive n=5/14
b
CTRL + CTLA4
Alive n=1/12
IFN + CTLA4
Alive n=3/10
% of ALL in PB
Days upon ALL injection

## Figure 16

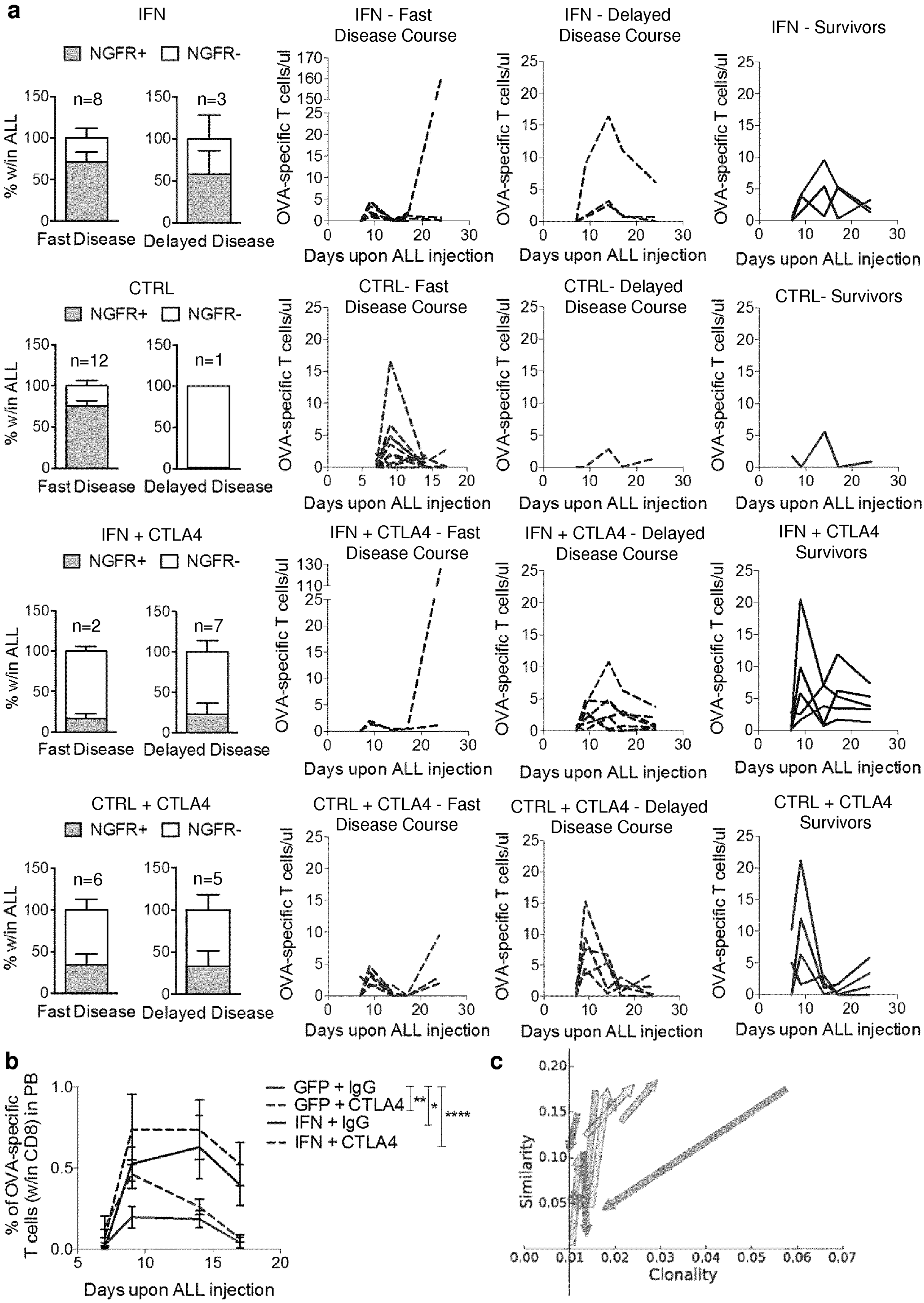
a
IFN
NGFR+ NGFR-
n=8
n=3
% w/in ALL
Fast Disease
Delayed Disease
IFN - Fast Disease Course
IFN - Delayed Disease Course
IFN - Survivors
OVA-specific T cells/ul
Days upon ALL injection
CTRL
n=12
n=1
CTRL- Fast Disease Course
CTRL- Delayed Disease Course
CTRL- Survivors
IFN + CTLA4
n=2
n=7
IFN + CTLA4 - Fast Disease Course
IFN + CTLA4 - Delayed Disease Course
IFN + CTLA4 Survivors
CTRL + CTLA4
n=6
n=5
CTRL + CTLA4 - Fast Disease Course
CTRL + CTLA4 - Delayed Disease Course
CTRL + CTLA4 Survivors
b
% of OVA-specific T cells (w/in CD8) in PB
GFP + IgG
GFP + CTLA4
IFN + IgG
IFN + CTLA4
**
*
****
Days upon ALL injection
c
Similarity
Clonality

## Figure 17

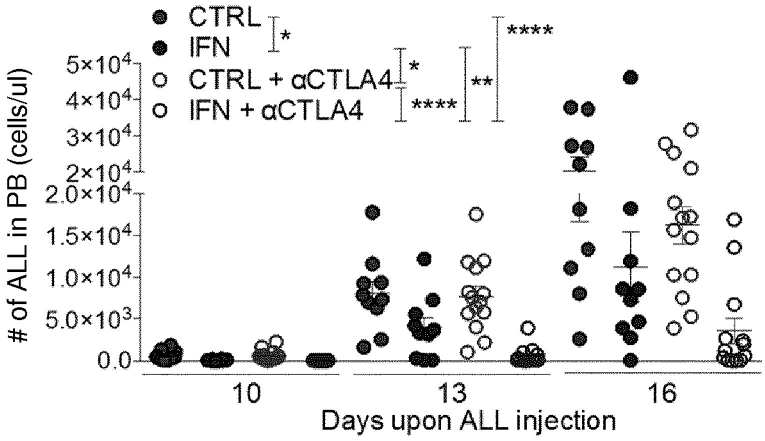


## Figure 18

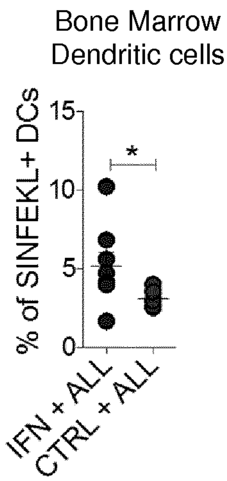

# Figure 19

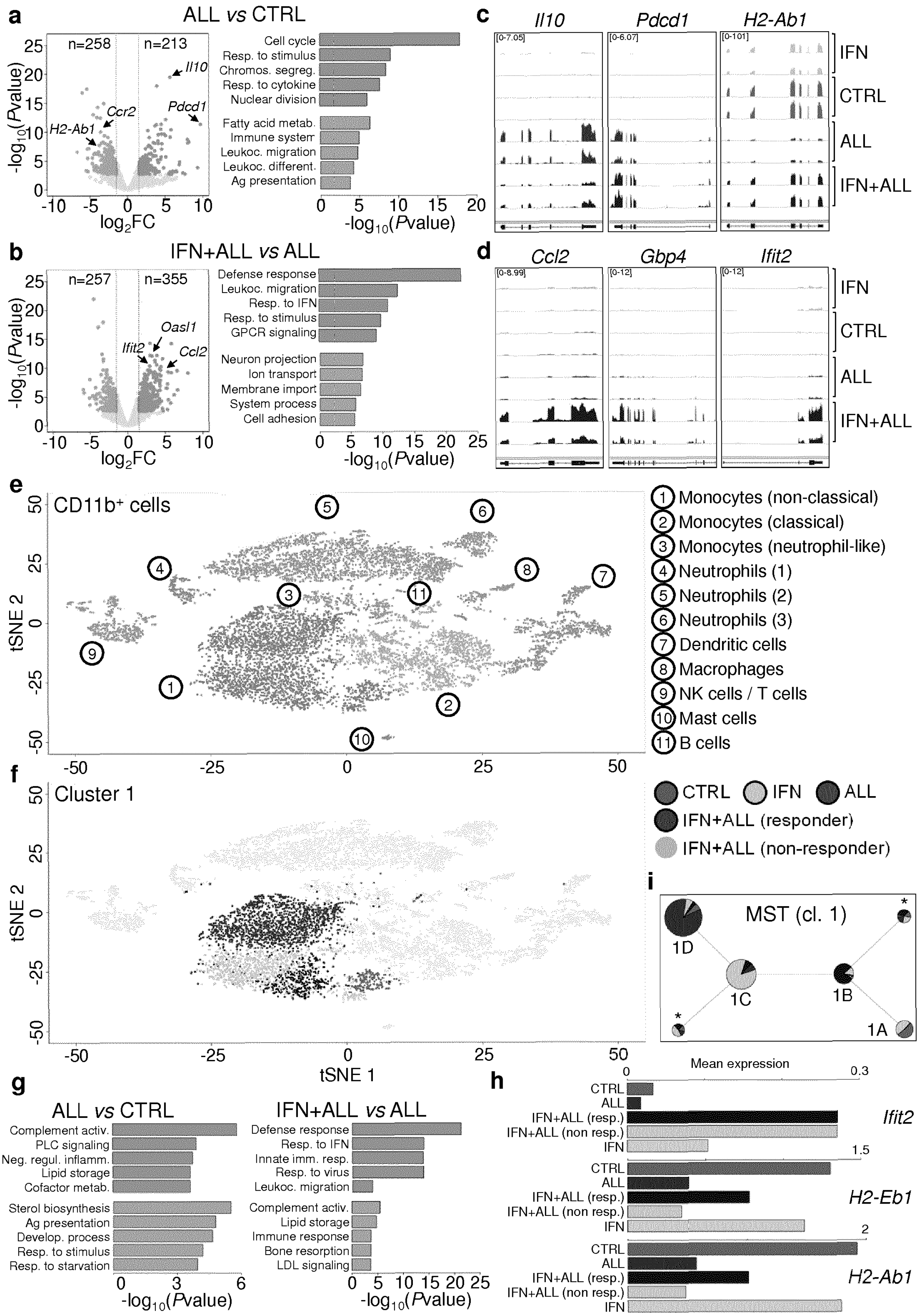
a
ALL vs CTRL
n=258
n=213
Il10
Ccr2
Pdcd1
H2-Ab1
-log10(Pvalue)
log2FC
Cell cycle
Resp. to stimulus
Chromos. segreg.
Resp. to cytokine
Nuclear division
Fatty acid metab.
Immune system
Leukoc. migration
Leukoc. different.
Ag presentation
-log10(Pvalue)
b
IFN+ALL vs ALL
n=257
n=355
Oasl1
Ifit2
Ccl2
Defense response
Leukoc. migration
Resp. to IFN
Resp. to stimulus
GPCR signaling
Neuron projection
Ion transport
Membrane import
System process
Cell adhesion
c
Il10
Pdcd1
H2-Ab1
IFN
CTRL
ALL
IFN+ALL
d
Ccl2
Gbp4
Ifit2
e
CD11b+ cells
tSNE 2
1 Monocytes (non-classical)
2 Monocytes (classical)
3 Monocytes (neutrophil-like)
4 Neutrophils (1)
5 Neutrophils (2)
6 Neutrophils (3)
7 Dendritic cells
8 Macrophages
9 NK cells / T cells
10 Mast cells
11 B cells
f
Cluster 1
tSNE 1
CTRL IFN ALL
IFN+ALL (responder)
IFN+ALL (non-responder)
i
MST (cl. 1)
1D
1C
1B
1A
g
ALL vs CTRL
Complement activ.
PLC signaling
Neg. regul. inflamm.
Lipid storage
Cofactor metab.
Sterol biosynthesis
Ag presentation
Develop. process
Resp. to stimulus
Resp. to starvation
IFN+ALL vs ALL
Defense response
Resp. to IFN
Innate imm. resp.
Resp. to virus
Leukoc. migration
Complement activ.
Lipid storage
Immune response
Bone resorption
LDL signaling
h
Mean expression
CTRL
ALL
IFN+ALL (resp.)
IFN+ALL (non resp.)
IFN
Ifit2
H2-Eb1
H2-Ab1

# Figure 20

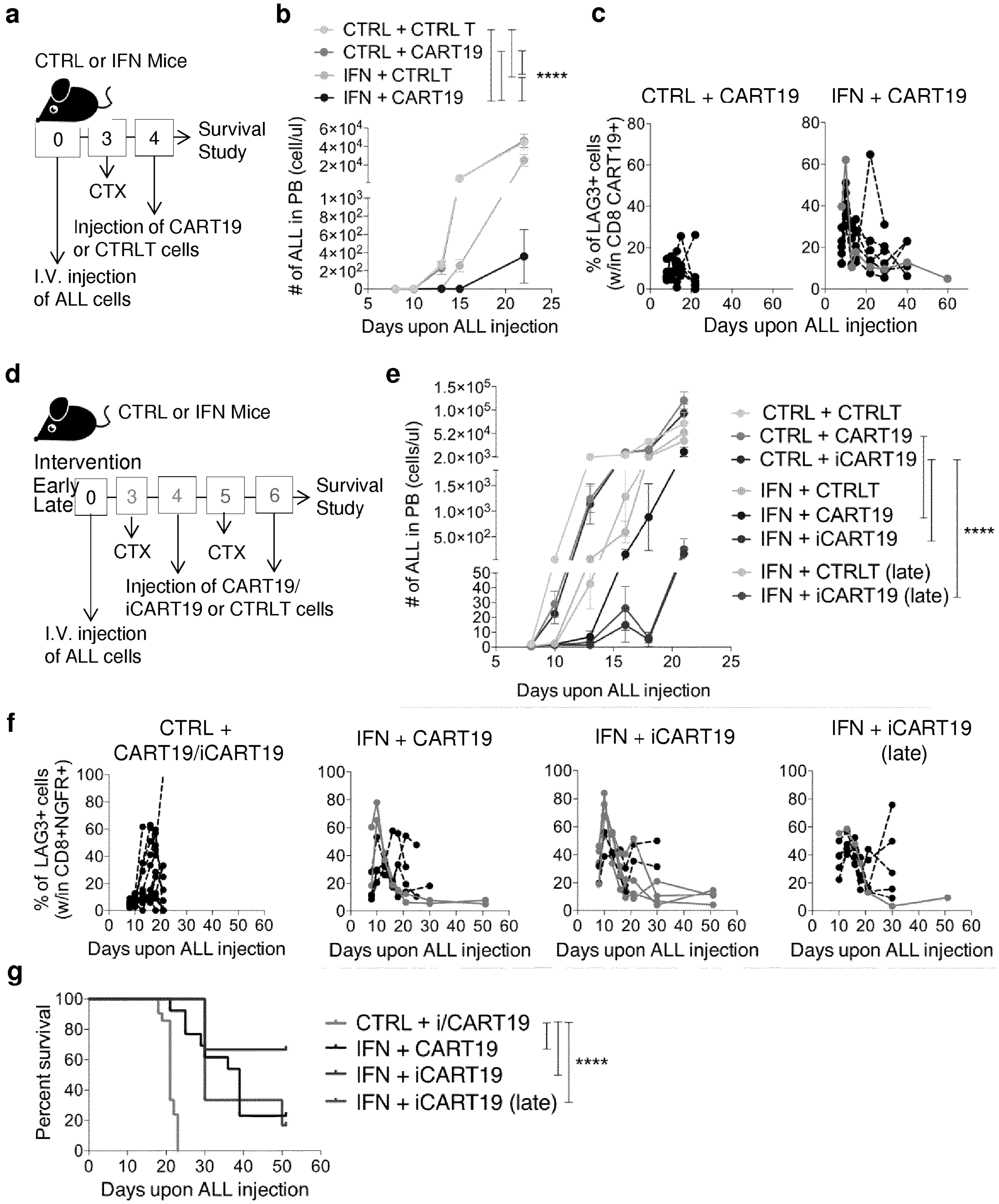
a
CTRL or IFN Mice
0
3
4
Survival Study
CTX
Injection of CART19 or CTRLT cells
I.V. injection of ALL cells
b
CTRL + CTRL T
CTRL + CART19
IFN + CTRLT
IFN + CART19
****
# of ALL in PB (cell/ul)
Days upon ALL injection
c
CTRL + CART19
IFN + CART19
% of LAG3+ cells (w/in CD8 CART19+)
Days upon ALL injection
d
CTRL or IFN Mice
Intervention
Early
Late
0
3
4
5
6
Survival Study
CTX
CTX
Injection of CART19/ iCART19 or CTRLT cells
I.V. injection of ALL cells
e
CTRL + CTRLT
CTRL + CART19
CTRL + iCART19
IFN + CTRLT
IFN + CART19
IFN + iCART19
IFN + CTRLT (late)
IFN + iCART19 (late)
****
# of ALL in PB (cells/ul)
Days upon ALL injection
f
CTRL + CART19/iCART19
IFN + CART19
IFN + iCART19
IFN + iCART19 (late)
% of LAG3+ cells (w/in CD8+NGFR+)
Days upon ALL injection
g
CTRL + i/CART19
IFN + CART19
IFN + iCART19
IFN + iCART19 (late)
****
Percent survival
Days upon ALL injection

# Figure 21

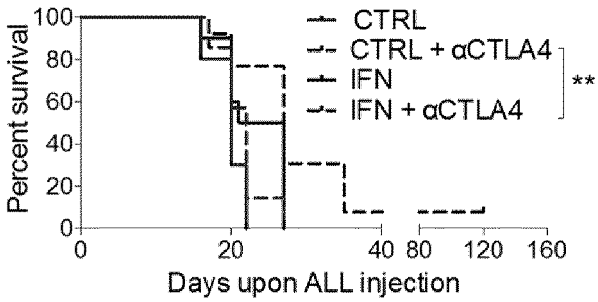
Percent survival
100
80
60
40
20
0
0
20
40
80
120
160
Days upon ALL injection
CTRL
CTRL + αCTLA4
IFN
IFN + αCTLA4
**

Figure 22

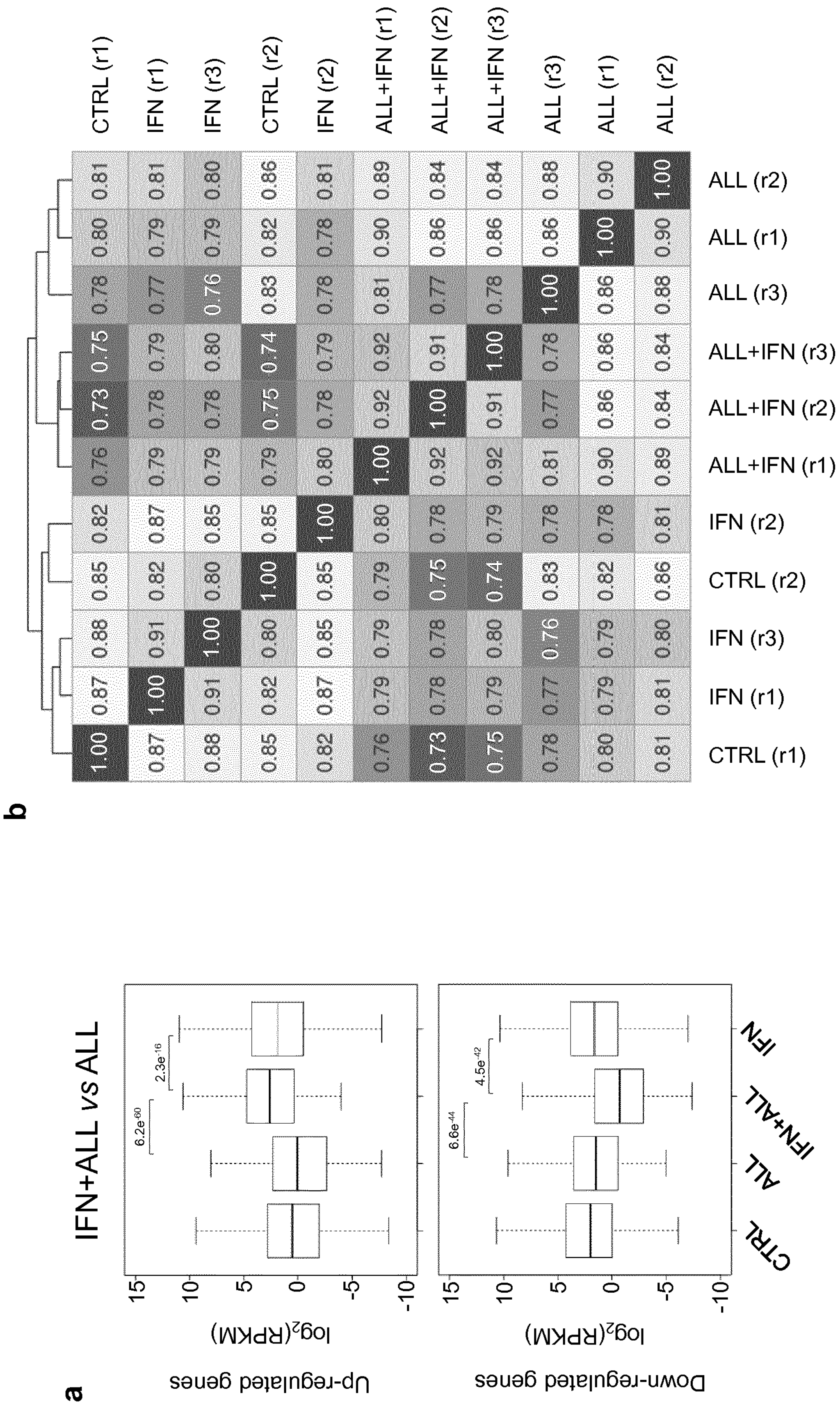
a
IFN+ALL vs ALL
Up-regulated genes
Down-regulated genes
log2(RPKM)
6.2e-60
2.3e-16
6.6e-44
4.5e-42
CTRL
ALL
IFN+ALL
IFN
b
CTRL (r1)
IFN (r1)
IFN (r3)
CTRL (r2)
IFN (r2)
ALL+IFN (r1)
ALL+IFN (r2)
ALL+IFN (r3)
ALL (r3)
ALL (r1)
ALL (r2)

## Figure 23

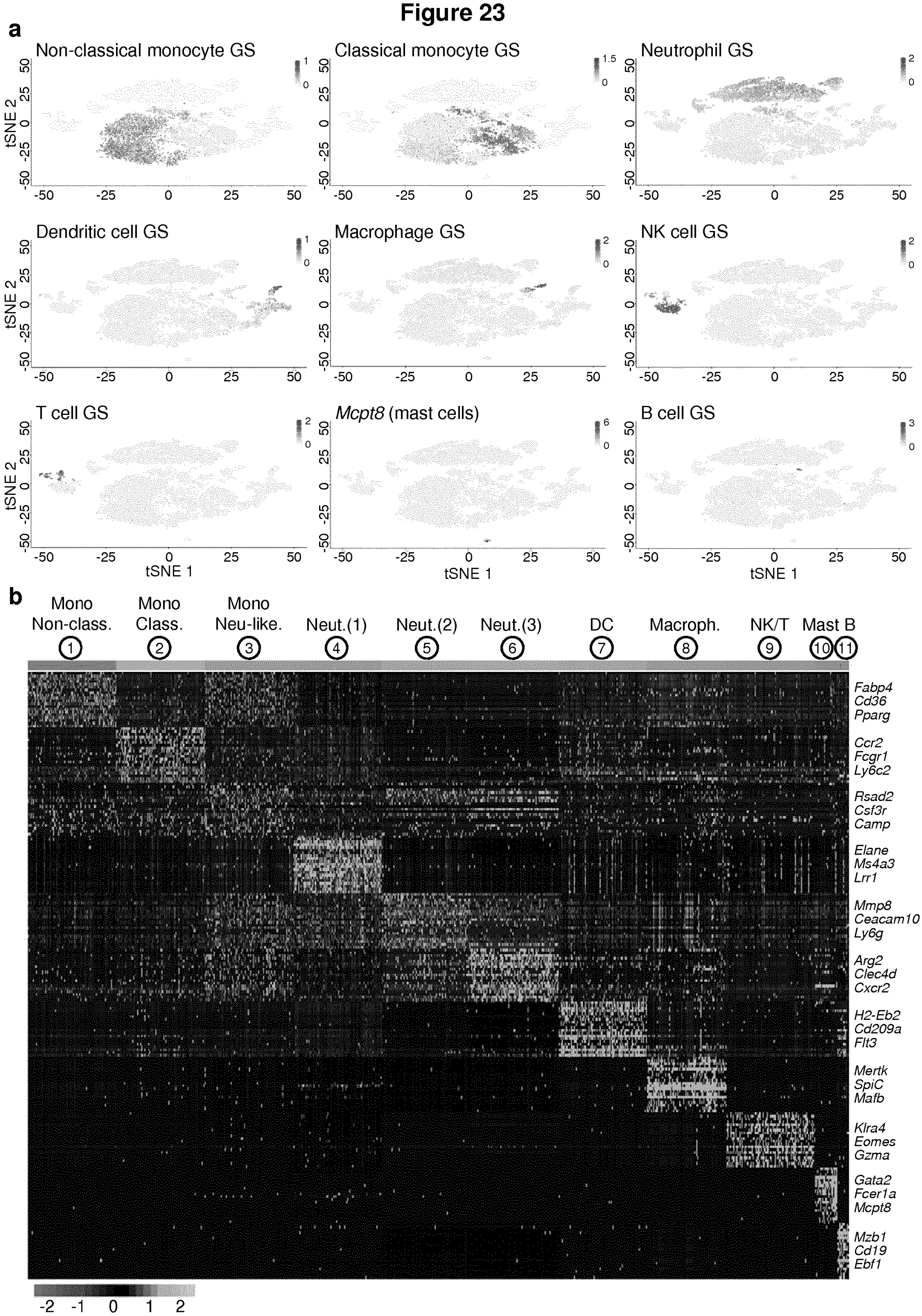
a
Non-classical monocyte GS
Classical monocyte GS
Neutrophil GS
Dendritic cell GS
Macrophage GS
NK cell GS
T cell GS
Mcpt8 (mast cells)
B cell GS
tSNE 2
tSNE 1
b
Mono Non-class.
Mono Class.
Mono Neu-like.
Neut.(1)
Neut.(2)
Neut.(3)
DC
Macroph.
NK/T
Mast
B
1
2
3
4
5
6
7
8
9
10
11
Fabp4
Cd36
Pparg
Ccr2
Fcgr1
Ly6c2
Rsad2
Csf3r
Camp
Elane
Ms4a3
Lrr1
Mmp8
Ceacam10
Ly6g
Arg2
Clec4d
Cxcr2
H2-Eb2
Cd209a
Flt3
Mertk
SpiC
Mafb
Klra4
Eomes
Gzma
Gata2
Fcer1a
Mcpt8
Mzb1
Cd19
Ebf1
-2 -1 0 1 2

## Figure 24

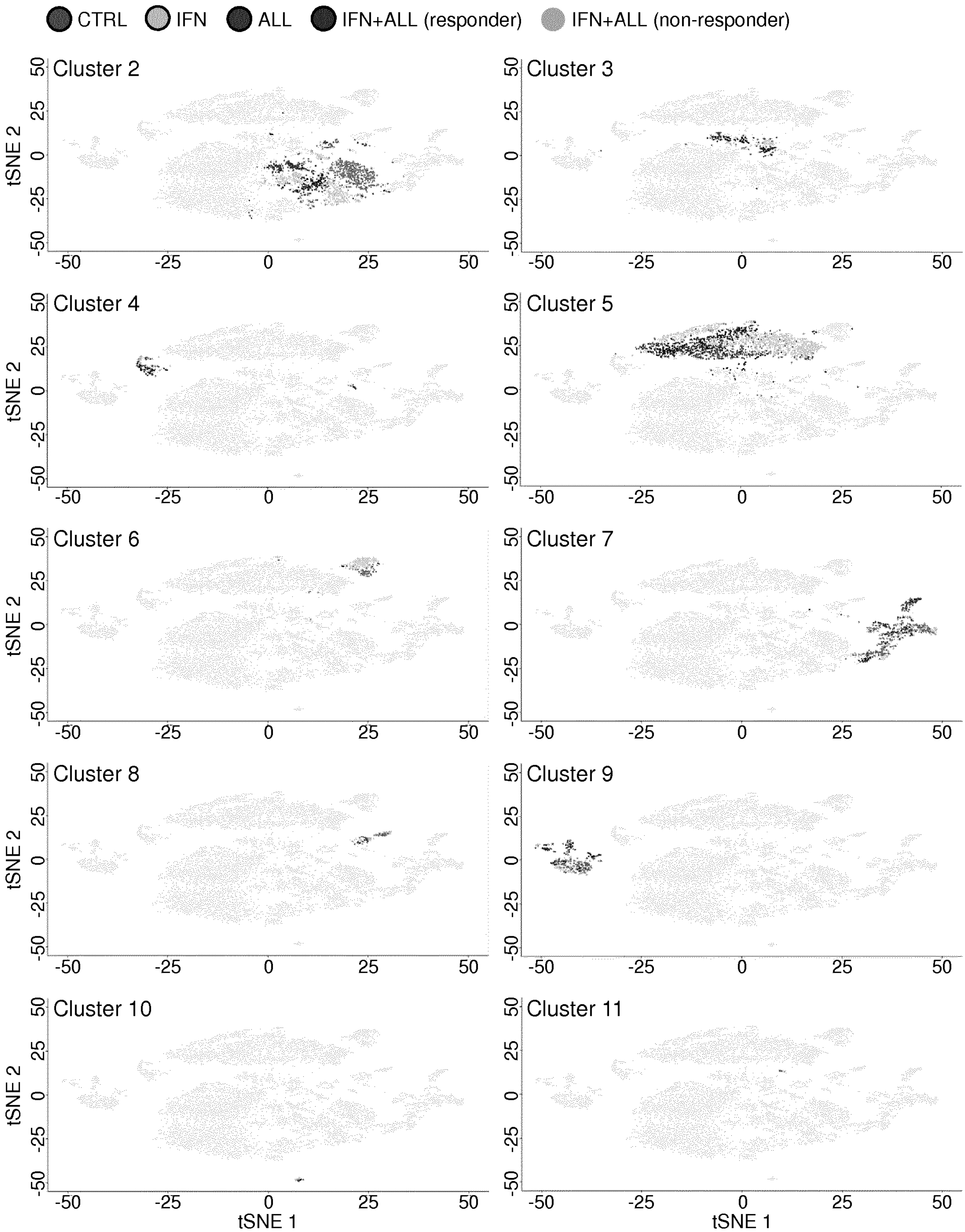
CTRL
IFN
ALL
IFN+ALL (responder)
IFN+ALL (non-responder)
Cluster 2
Cluster 3
Cluster 4
Cluster 5
Cluster 6
Cluster 7
Cluster 8
Cluster 9
Cluster 10
Cluster 11
tSNE 2
tSNE 1
-50
-25
0
25
50

## Figure 25

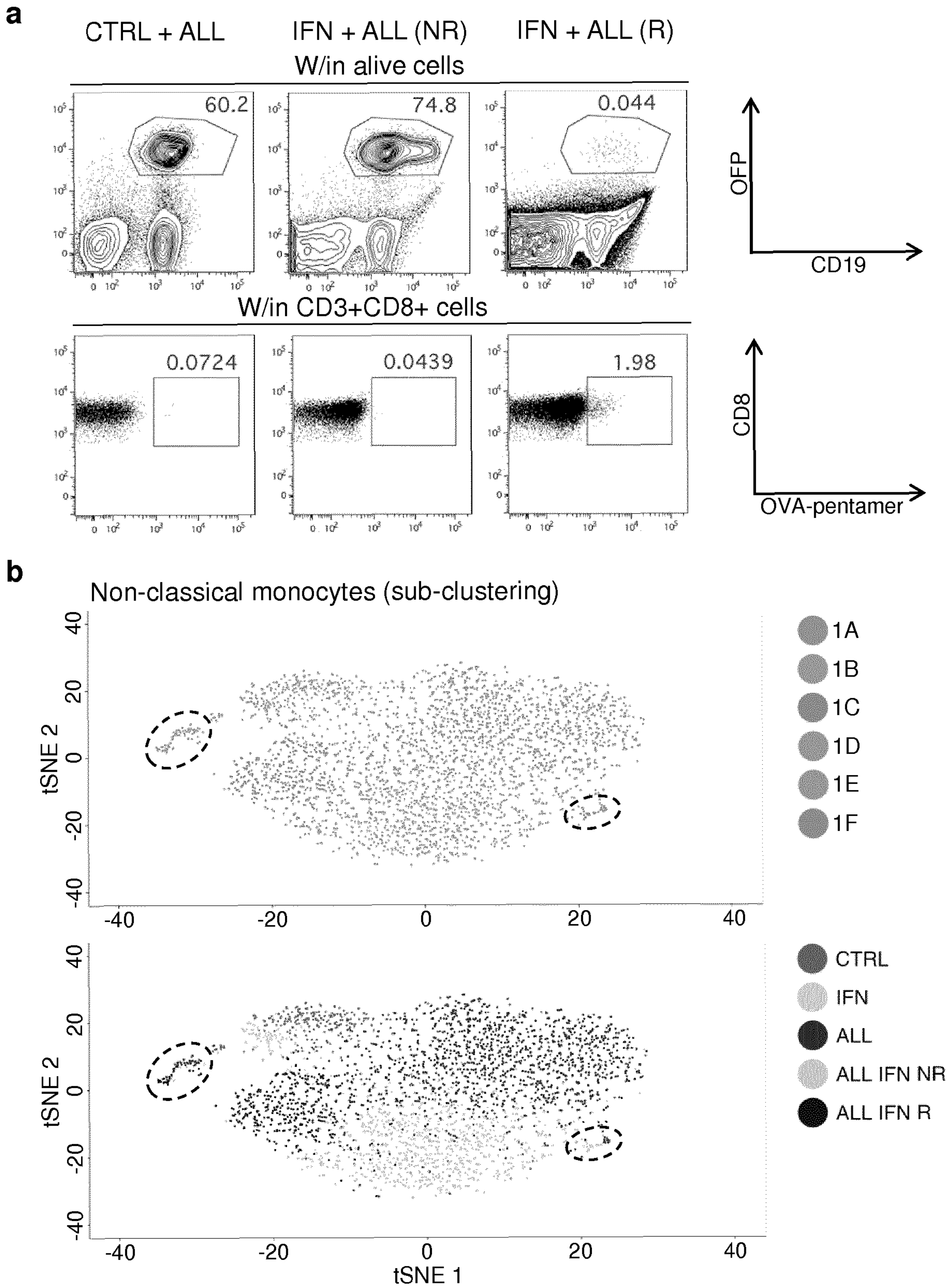
a
CTRL + ALL
IFN + ALL (NR)
IFN + ALL (R)
W/in alive cells
60.2
74.8
0.044
OFP
CD19
W/in CD3+CD8+ cells
0.0724
0.0439
1.98
CD8
OVA-pentamer
b
Non-classical monocytes (sub-clustering)
tSNE 2
1A
1B
1C
1D
1E
1F
CTRL
IFN
ALL
ALL IFN NR
ALL IFN R
tSNE 1

## Figure 26

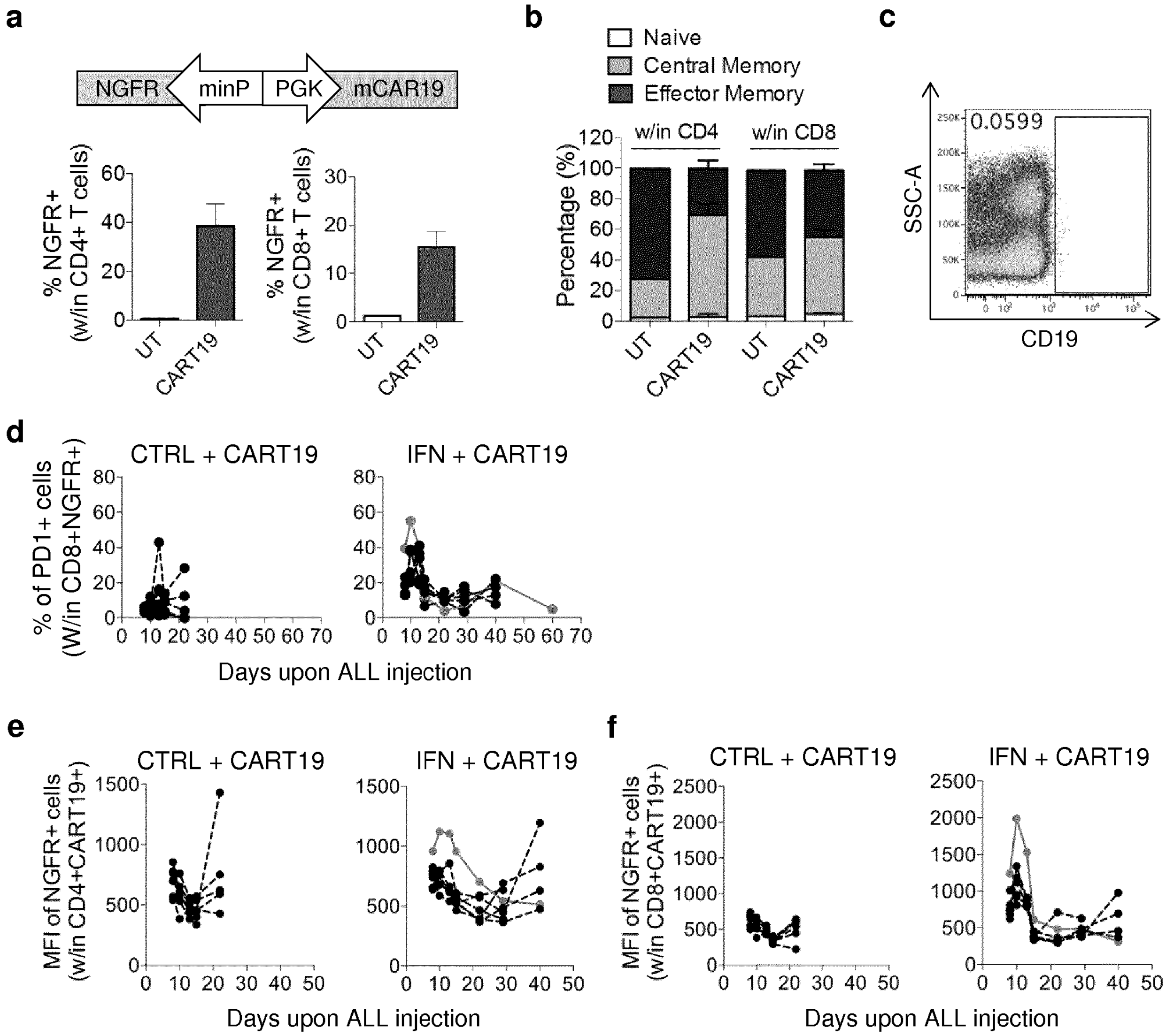
a
NGFR
minP
PGK
mCAR19
% NGFR+ (w/in CD4+ T cells)
% NGFR+ (w/in CD8+ T cells)
UT
CART19
b
Naive
Central Memory
Effector Memory
w/in CD4
w/in CD8
Percentage (%)
c
0.0599
SSC-A
CD19
d
CTRL + CART19
IFN + CART19
% of PD1+ cells (W/in CD8+NGFR+)
Days upon ALL injection
e
MFI of NGFR+ cells (w/in CD4+CART19+)
f
MFI of NGFR+ cells (w/in CD8+CART19+)

# Figure 27

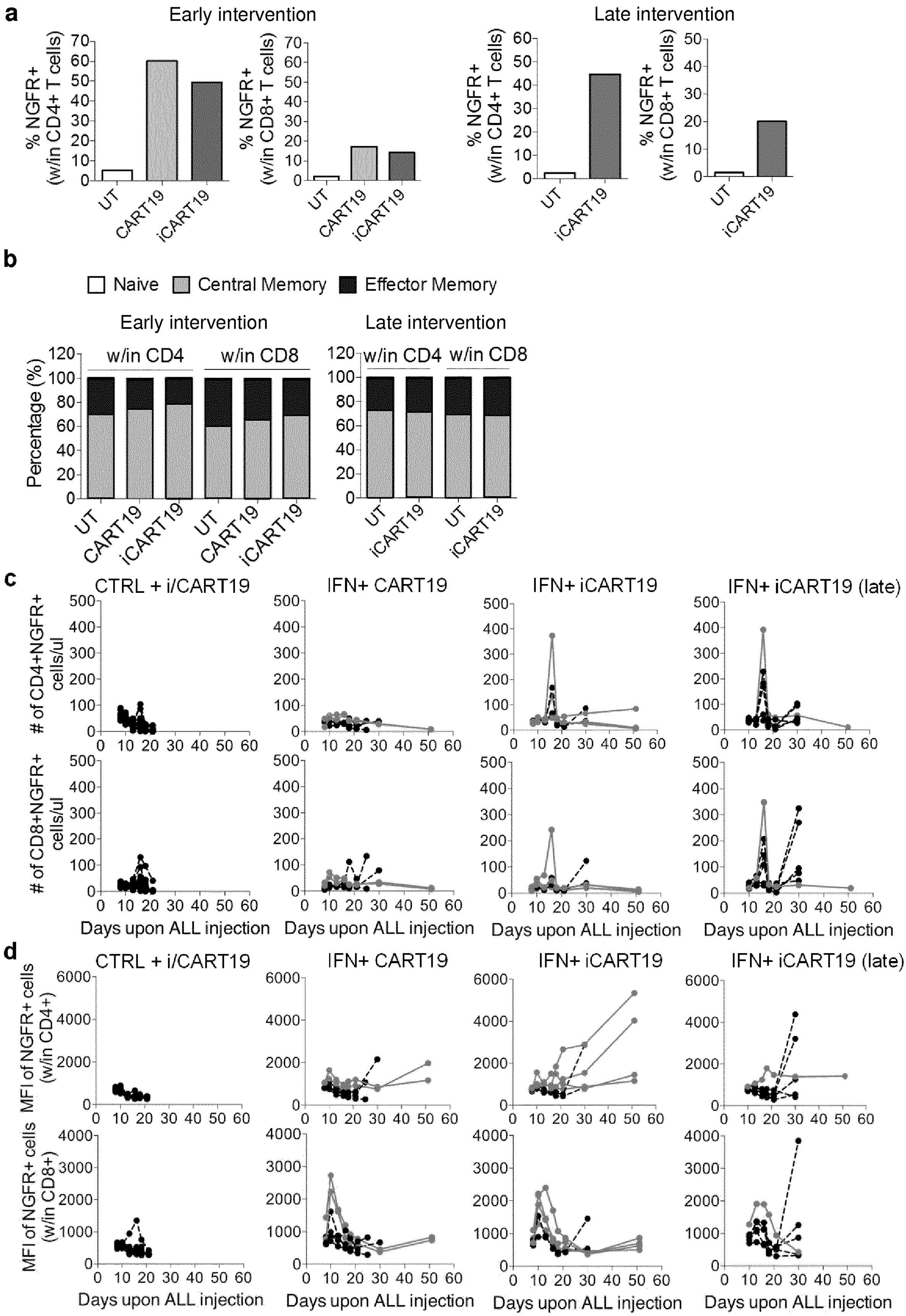
a
Early intervention
Late intervention
% NGFR+ (w/in CD4+ T cells)
% NGFR+ (w/in CD8+ T cells)
UT
CART19
iCART19
b
Naive
Central Memory
Effector Memory
Early intervention
Late intervention
w/in CD4
w/in CD8
Percentage (%)
c
CTRL + i/CART19
IFN+ CART19
IFN+ iCART19
IFN+ iCART19 (late)
# of CD4+NGFR+ cells/ul
# of CD8+NGFR+ cells/ul
Days upon ALL injection
d
MFI of NGFR+ cells (w/in CD4+)
MFI of NGFR+ cells (w/in CD8+)

# Figure 28

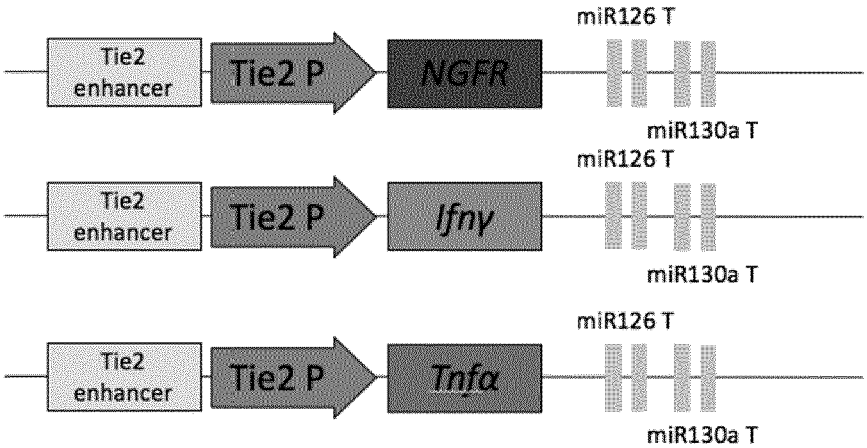
miR126 T
Tie2
enhancer
Tie2 P
NGFR
miR130a T
miR126 T
Tie2
enhancer
Tie2 P
Ifnγ
miR130a T
miR126 T
Tie2
enhancer
Tie2 P
Tnfα
miR130a T

**Figure 29**

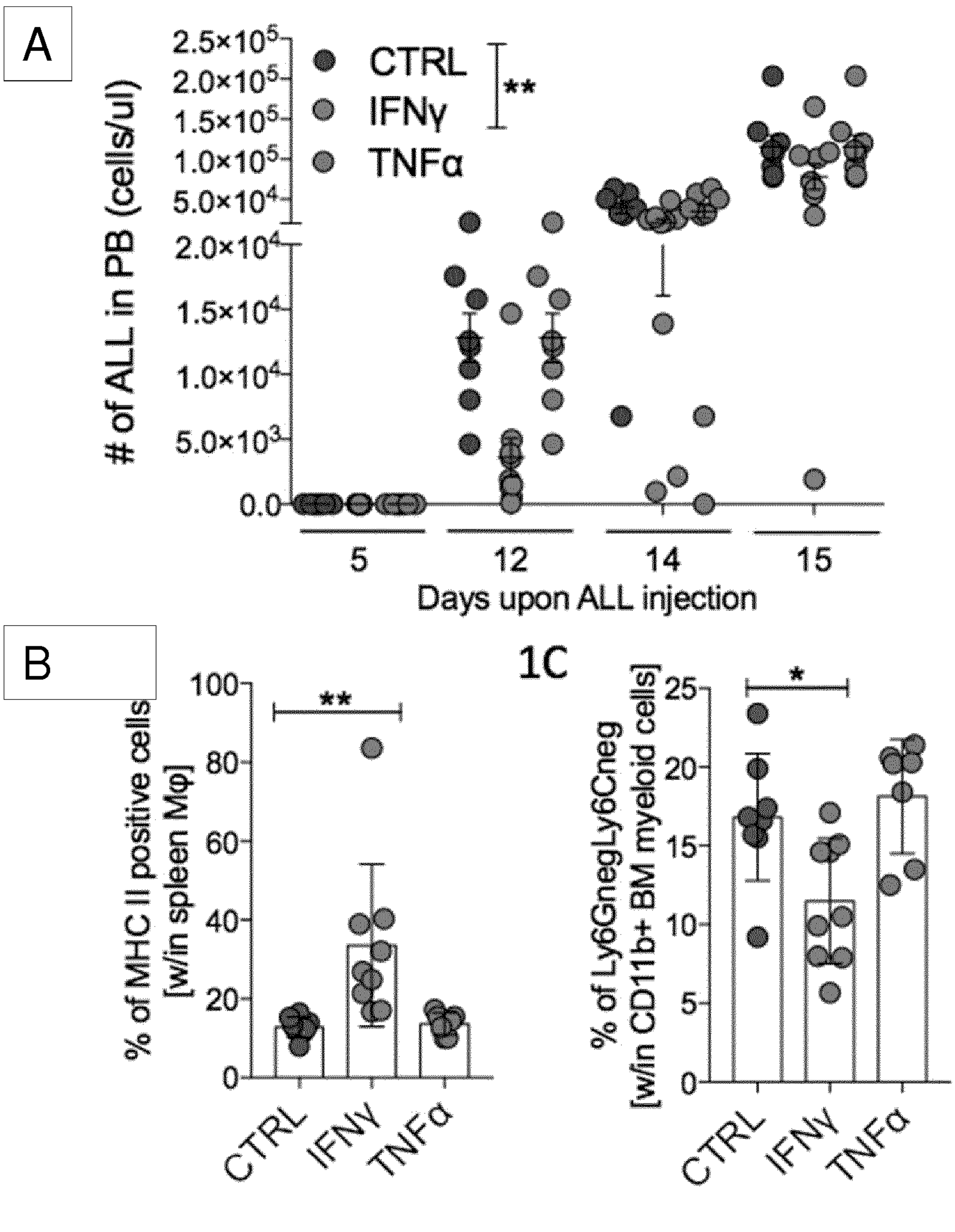
A
# of ALL in PB (cells/ul)
2.5×10^5
2.0×10^5
1.5×10^5
1.0×10^5
5.0×10^4
2.0×10^4
1.5×10^4
1.0×10^4
5.0×10^3
0.0
CTRL
IFNγ
TNFα
**
5
12
14
15
Days upon ALL injection
B
% of MHC II positive cells [w/in spleen Mφ]
100
80
60
40
20
0
**
CTRL
IFNγ
TNFα
1C
% of Ly6GnegLy6Cneg [w/in CD11b+ BM myeloid cells]
25
20
15
10
5
0
*
CTRL
IFNγ
TNFα

# Figure 30

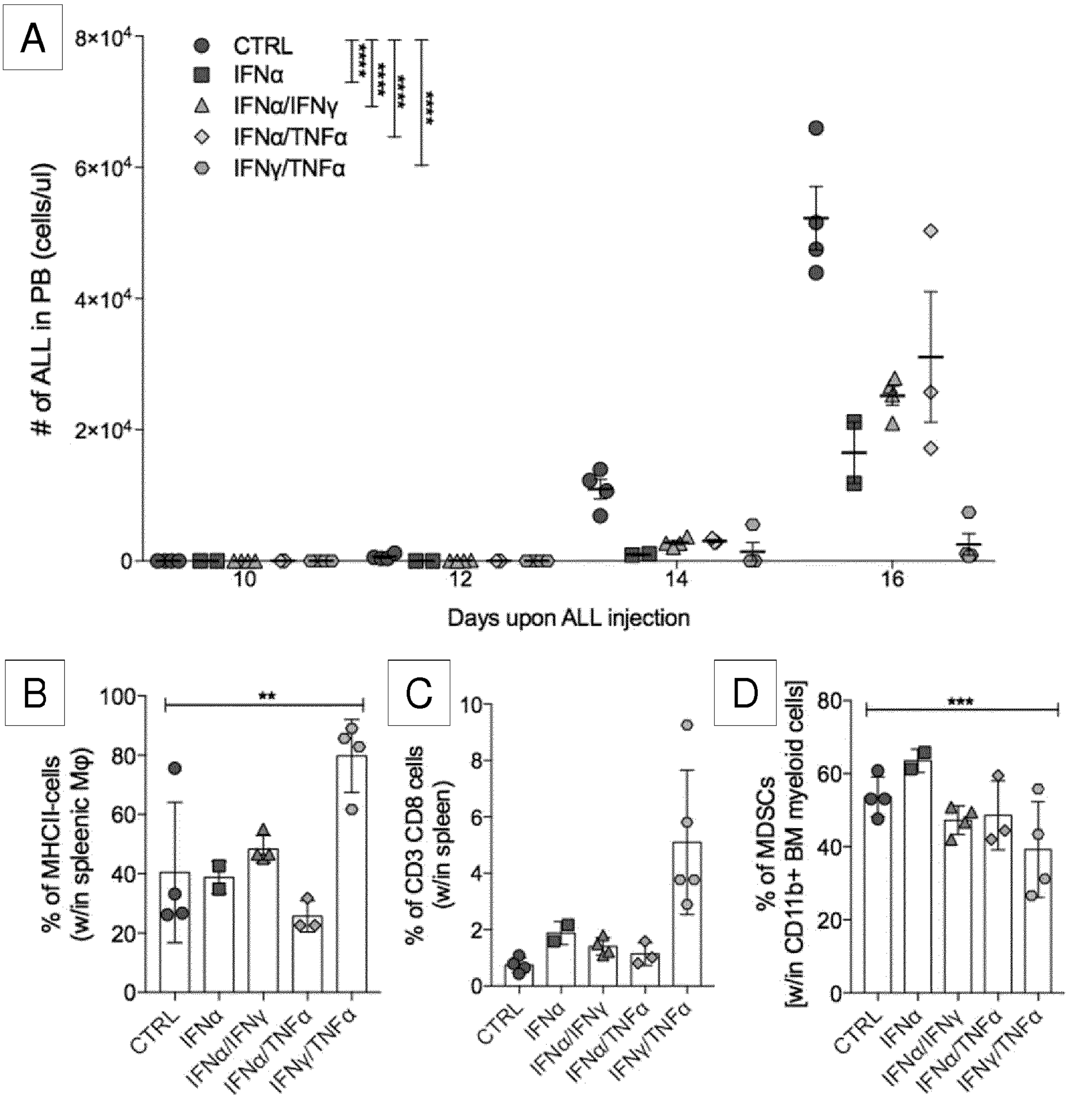
A
8×10⁴
6×10⁴
4×10⁴
2×10⁴
0
# of ALL in PB (cells/ul)
CTRL
IFNα
IFNα/IFNγ
IFNα/TNFα
IFNγ/TNFα
****
****
****
****
10
12
14
16
Days upon ALL injection
B
% of MHCII-cells (w/in spleenic Mφ)
**
C
% of CD3 CD8 cells (w/in spleen)
D
% of MDSCs [w/in CD11b+ BM myeloid cells]
***
CTRL
IFNα
IFNα/IFNγ
IFNα/TNFα
IFNγ/TNFα

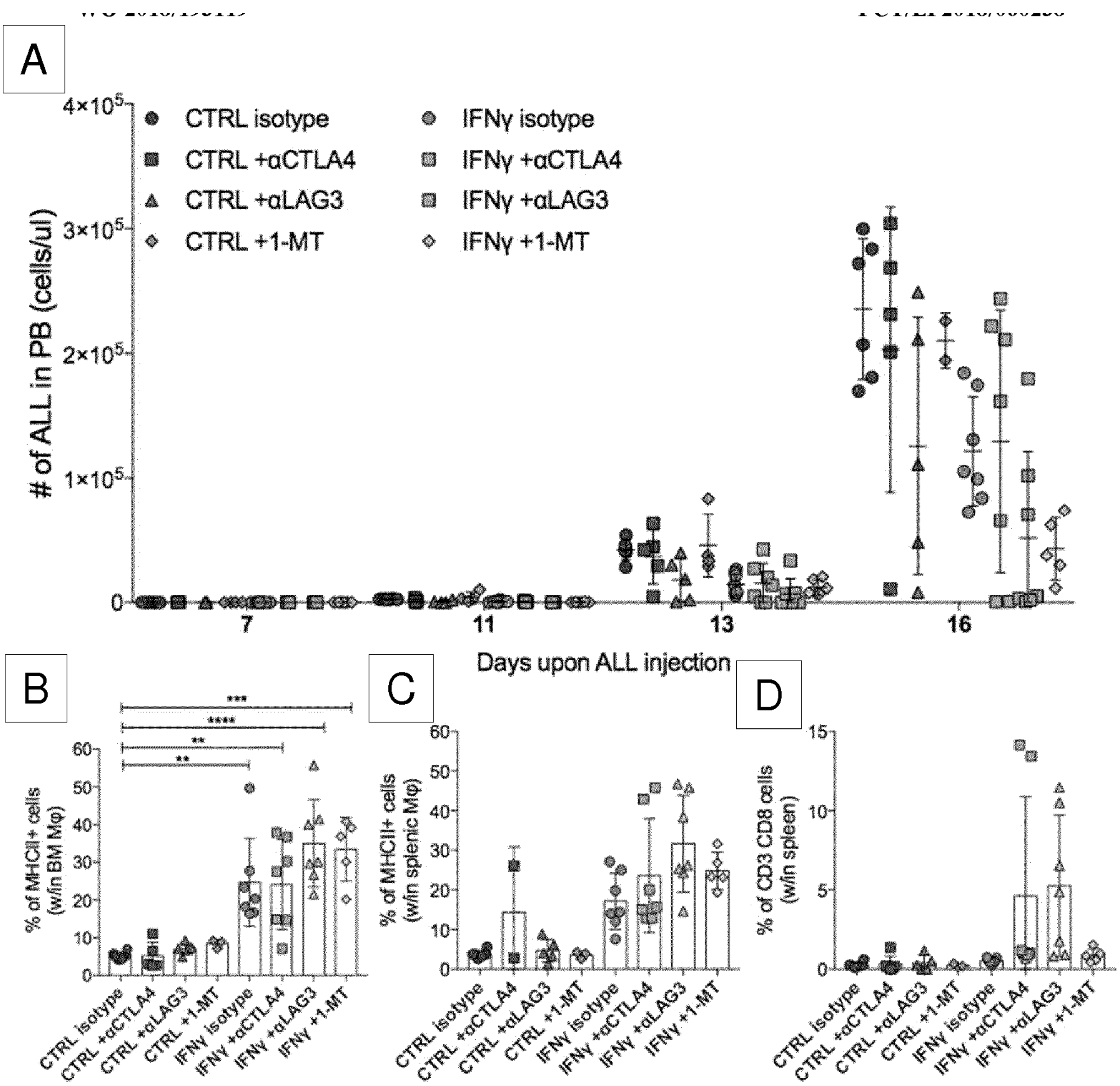
A
CTRL isotype
CTRL +αCTLA4
CTRL +αLAG3
CTRL +1-MT
IFNγ isotype
IFNγ +αCTLA4
IFNγ +αLAG3
IFNγ +1-MT
# of ALL in PB (cells/ul)
4×10^5
3×10^5
2×10^5
1×10^5
0
7
11
13
16
Days upon ALL injection
B
% of MHCII+ cells (w/in BM Mφ)
***
****
**
**
C
% of MHCII+ cells (w/in splenic Mφ)
D
% of CD3 CD8 cells (w/in spleen)
CTRL isotype
CTRL +αCTLA4
CTRL +αLAG3
CTRL +1-MT
IFNγ isotype
IFNγ +αCTLA4
IFNγ +αLAG3
IFNγ +1-MT

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2856876 A1
- WO 199941397 A
- WO 200179518 A

### Non-patent literature cited in the description


- **BERGE et al.** *J Pharm Sci,* 1977, vol. 66, 1-19
- *Nature Biotechnology,* 1996, vol. 14, 556
- **COFFIN et al.** Retroviruses. Cold Spring Harbour Laboratory Press, 1997, 758-763
- **LEWIS et al.** *EMBO J.,* 1992, 3053-3058
- **ZILBERBERG et al.** *Biology of Blood and Marrow Transplantation,* June 2015, vol. 21 (6), 1000-1007
- **LINNEMANN et al.** *Nature Medicine,* 2013, vol. 19, 1534-1541
- **OKAMOTO S.** *Cancer research,* 2009, vol. 69, 9003-9011
- **PROVASI E. ; GENOVESE P.** *Nature Medicine,* May 2012, vol. 18 (5), 807-15
- **LOMBARDO A.** *Nature biotechnology,* 2007, vol. 25, 1298-1306
- **SOMMERMEYER ; UCKERT.** *J Immunol,* 2010, vol. 184, 6223-6231
- **PULE et al.** *Mol Ther,* November 2005, vol. 12 (5), 933-41
- **BRENTJENS et al.** *CCR,* 15 September 2007, vol. 13, 5426-35
- **CASUCCI et al.** *Blood,* 14 November 2013, vol. 122 (20), 3461-72
- **SAVOLDO B.** *Blood,* 18 June 2009, vol. 113 (25), 6392-402
- **MAHER et al.** *Nat Biotechnol,* January 2002, vol. 20 (1), 70-5
- **IMAI C.** *Leukemia,* April 2004, vol. 18 (4), 676-84
- **MILONE et al.** *Mol Ther,* August 2009, vol. 17 (8), 1453-64
- **TODOROVSKA et al.** *J Immunol Methods,* 2001, vol. 248 (1), 47-66
- **HUDSON ; KORTT.** *J Immunol Methods,* 1999, vol. 231 (1), 177-189
- **POLJAK.** *Structure,* 1994, vol. 2 (12), 1121-1123
- **RONDON ; MARASCO.** *Annual Review of Microbiology,* 1997, vol. 21, 257-283
- **GREENFIELD.** Antibodies: A Laboratory Manual. 2014, 201-221
- **DEVEREUX et al.** Nucleic Acids Res. University of Wisconsin, 1984, vol. 12, 387
- **AUSUBEL et al.** ibid. 1999
- **ATSCHUL et al.** *J. Mol. Biol.,* 1990, 403-410
- **AUSUBEL et al.** *ibid,* 1999, 7-58, 7-60
- *FEMS Microbiol. Lett.,* 1999, vol. 174, 247-50
- *FEMS Microbiol. Lett.,* 1999, vol. 177, 187-8
- **SAMBROOK, J. ; FRITSCH, E.F. ; MANIATIS, T.** Molecular Cloning: A Laboratory Manual. Cold Spring Harbor Laboratory Press, 1989
- **AUSUBEL, F.M. et al.** Current Protocols in Molecular Biology. John Wiley & Sons, 1995
- **ROE, B. ; CRABTREE, J ; KAHN, A.** DNA Isolation and Sequencing: Essential Techniques. John Wiley & Sons, 1996
- **POLAK, J.M. ; MCGEE, J.O'D.** Situ Hybridization: Principles and Practice. Oxford University Press, 1990
- **GAIT, M.J.** Oligonucleotide Synthesis: A Practical Approach. IRL Press, 1984
- DNA Structures Part A: Synthesis and Physical Analysis of DNA. **LILLEY, D.M. ; DAHLBERG, J.E.** Methods in Enzymology. Academic Press, 1992
- **DE PALMA, M. et al.** *Cancer Cell,* 2008, vol. 14, 299-311
- **ESCOBAR, G. et al.** *Sci. Transl. Med.,* 2014, vol. 6, 217-3
- **CATARINELLA, M. et al.** *EMBO Mol. Med.,* 2016, vol. 8, 155-170
- **ZHENG, G.X. et al.** *Nat. Commun.,* 2017, vol. 8, 14049
- **LAVIN, Y. et al.** *Cell,* 2014, vol. 159, 1312-1326
- **VAROL, D. et al.** *Immunity,* 2017, vol. 46, 1030-1044
- **YÁÑEZ, A. et al.** *Immunity,* 2017, vol. 47, 890-902
- **KOCHENDERFER, J.N. et al.** *Blood,* 2010, vol. 116, 3875-3886
- **CASUCCI, M. et al.** *Blood,* 2013, vol. 122, 3461-3472
- **DAVILA, M.L. et al.** *PLoS One,* 2013, vol. 8, 1-14

- **FOLLENZI, A. et al.** *Nat. Genet.,* 2000, vol. 25, 217-222
- **DE PALMA et al.** *Blood,* 2005
- **PICELLI, S. et al.** *Nat. Protoc.,* 2014, vol. 9, 171-181
- **ESCOBAR, G. et al.** *Sci Transl Med,* 2014, vol. 6, 217-3
- **DE PALMA, M. et al.** *Cancer Cell,* 2005, vol. 8, 211-226
- **NUCERA, S. et al.** *Cancer Cell,* 2016, vol. 29, 905-921
- **KHALIL, D. N. ; SMITH, E. L. ; BRENTJENS, R. J. ; WOLCHOK, J. D.** The future of cancer treatment: immunomodulation, CARs and combination immunotherapy. *Nat. Rev. Clin. Oncol.,* 2016, vol. 13, 273-290
- **TOPALIAN, S. L. ; DRAKE, C. G. ; PARDOLL, D. M.** Immune Checkpoint Blockade : A Common Denominator Approach to Cancer Therapy. *Cancer Cell,* 2015, vol. 27, 450-461
- **SCHADENDORF, D. et al.** Pooled Analysis of Long-Term Survival Data From Phase II and Phase III Trials of Ipilimumab in Unresectable or Metastatic Melanoma. *J. Clin. Oncol.,* 2015, vol. 33, 1889-94
- **PITT, J. M. et al.** Resistance Mechanisms to Immune-Checkpoint Blockade in Cancer: Tumor-Intrinsic and -Extrinsic Factors. *Immunity,* 2016, vol. 44, 1255-1269
- **PARKER, B. S. ; RAUTELA, J. ; HERTZOG, P. J.** Antitumour actions of interferons: implications for cancer therapy. *Nat. Rev. Cancer,* 2016, vol. 16, 131-44
- **HERVAS-STUBBS, S. et al.** Direct effects of type I interferons on cells of the immune system. *Clin. Cancer Res.,* 2011, vol. 17, 2619-27
- **FUERTES, M. B. ; WOO, S. R. ; BURNETT, B. ; FU, Y. X. ; GAJEWSKI, T. F.** Type I interferon response and innate immune sensing of cancer. *Trends Immunol.,* 2013, vol. 34, 67-73
- **DE PALMA, M. et al.** Tumor-Targeted Interferon-Alpha Delivery by Tie2-Expressing Monocytes Inhibits Tumor Growth and Metastasis. *Cancer Cell,* 2008, vol. 14, 299-311
- **ESCOBAR, G. et al.** Genetic engineering of hematopoiesis for targeted IFN-$\alpha$ delivery inhibits breast cancer progression. *Sci. Transl. Med.,* 2014, vol. 6, 217-3
- **CATARINELLA, M. et al.** IFN$\alpha$ gene/cell therapy curbs colorectal cancer colonization of the liver by acting on the hepatic microenvironment. *EMBO Mol. Med.,* 2016, vol. 8, 155-70
- **FESNAK, A. D. ; JUNE, C. H. ; LEVINE, B. L.** Engineered T cells: the promise and challenges of cancer immunotherapy. *Nat. Rev. Cancer,* 2016, vol. 16, 566-81
- **ROSENBERG, S. A. ; RESTIFO, N. P.** Adoptive cell transfer as personalized immunotherapy for human cancer. *Science,* 2015, vol. 348 (80), 62-68
- **SOTILLO, E. et al.** Convergence of acquired mutations and alternative splicing of CD19 enables resistance to CART-19 immunotherapy. *Cancer Discov.,* 2015, vol. 5, 1282-1295
- **NUCERA, S. et al.** miRNA-126 Orchestrates an Oncogenic Program in B Cell Precursor Acute Lymphoblastic Leukemia. *Cancer Cell,* 2016, vol. 29, 905-921
- **DE PALMA, M. ; VENNERI, M. A. ; ROCA, C. ; NALDINI, L.** Targeting exogenous genes to tumor angiogenesis by transplantation of genetically modified hematopoietic stem cells. *Nat. Med,* 2003, vol. 9, 789-95
- **DE PALMA, M. et al.** Tie2 identifies a hematopoietic lineage of proangiogenic monocytes required for tumor vessel formation and a mesenchymal population of pericyte progenitors. *Cancer Cell,* 2005, vol. 8, 211-26
- **MELLMAN, I. ; COUKOS, G. ; DRANOFF, G.** Cancer immunotherapy comes of age. *Nature,* 2011, vol. 480, 480-9
- **KUMAR, V. ; PATEL, S. ; TCYGANOV, E. ; GABRILOVICH, D. I.** The Nature of Myeloid-Derived Suppressor Cells in the Tumor Microenvironment. *Trends Immunol.,* 2016, vol. 37, 208-220
- **RUFFELL, B. ; COUSSENS, L. M.** Macrophages and therapeutic resistance in cancer. *Cancer Cell,* 2015, vol. 27, 462-472
- **JOYCE JOHANNA A. ; FEARON DOUGLAS T.** T cell exclusion, immune privilege, and the tumor microenvironment. *Science,* 2015, vol. 348 (80
- **VERDEGAAL, E. M. E. et al.** Neoantigen landscape dynamics during human melanoma-T cell interactions. *Nature,* 2016, vol. 536, 91-5
- **MATSUSHITA, H. et al.** Cancer exome analysis reveals a T-cell-dependent mechanism of cancer immunoediting. *Nature,* 2012, vol. 482, 400-4
- **LINNEMANN, C. et al.** High-throughput epitope discovery reveals frequent recognition of neo-antigens by CD4+ T cells in human melanoma. *Nat Med,* 2015, vol. 21, 81-85
- **LU, Y. C. et al.** Efficient identification of mutated cancer antigens recognized by T cells associated with durable tumor regressions. *Clin. Cancer Res.,* 2014, vol. 20, 3401-3410
- **MCGRANAHAN, N. et al.** Clonal neoantigens elicit T cell immunoreactivity and sensitivity to immune checkpoint blockade. *Science,* 2016, vol. 351 (80), 1463-1469
- **SCHUMACHER, T. N. ; SCHREIBER, R. D.** Neoantigens in cancer immunotherapy. *Science,* 2015, vol. 348 (80), 69-74

- **SHARMA, P. ; ALLISON, J. P.** Immune Checkpoint Targeting in Cancer Therapy : Toward Combination Strategies with Curative Potential. *Cell,* 2015, vol. 161, 205-214
- **SCHILDBERG, F. A. ; KLEIN, S. R. ; FREEMAN, G. J. ; SHARPE, A. H.** Coinhibitory Pathways in the B7-CD28 Ligand-Receptor Family. *Immunity,* 2016, vol. 44, 955-972
- **YANG, X. et al.** Targeting the Tumor Microenvironment with Interferon-β Bridges Innate and Adaptive Immune Responses. *Cancer Cell,* 2014, vol. 25, 37-48
- **HUANG, T.-H. ; CHINTALACHARUVU, K. R. ; MORRISON, S. L.** Targeting IFN-alpha to B Cell Lymphoma by a Tumor-Specific Antibody Elicits Potent Antitumor Activities. *J. Immunol.,* 2007, vol. 179, 6881-6888
- **WILSON, E. B. et al.** Blockade of chronic type I interferon signaling to control persistent LCMV infection. *Science,* 2013, vol. 340, 202-7
- **SANDLER, N. G. et al.** Type I interferon responses in rhesus macaques prevent SIV infection and slow disease progression. *Nature,* 2014, vol. 511, 601-605
- **JOSEPH BENCI, A. L. et al.** Tumor Interferon Signaling Regulates a Multigenic Resistance Program to Immune Checkpoint Blockade. *Cell,* 2016, vol. 167, 1540-1554
- **GENTLES, A. J. et al.** The prognostic landscape of genes and infiltrating immune cells across human cancers. *Nat. Med.,* 2015, vol. 21, 938-945
- **MLECNIK, B. et al.** Integrative Analyses of Colorectal Cancer Show Immunoscore Is a Stronger Predictor of Patient Survival Than Microsatellite Instability. *Immunity,* 2016, vol. 44, 698-711
- **BACHIREDDY, P. ; BURKHARDT, U. E. ; RAJASAGI, M. ; WU, C. J.** Haematological malignancies: at the forefront of immunotherapeutic innovation. *Nat. Rev. Cancer,* 2015, vol. 15, 201-215
- **MÁTRAI, J. et al.** Hepatocyte-targeted expression by integrase-defective lentiviral vectors induces antigen-specific tolerance in mice with low genotoxic risk. *Hepatology,* 2011, vol. 53, 1696-1707
- **AMENDOLA, M. ; VENNERI, M. A. ; BIFFI, A. ; VIGNA, E. ; NALDINI, L.** Coordinate dual-gene transgenesis by lentiviral vectors carrying synthetic bidirectional promoters. *Nat. Biotechnol.,* 2005, vol. 23, 108-116
- **TUMEH, P. ; HARVIEW, C. ; YEARLEY, J. ; AL, E.** PD-1 blockade induces responses by inhibiting adaptive immune resistance. *Nature,* 2014, vol. 515, 568-571
- **WAGGOTT, D. et al.** NanoStringNorm: An extensible R package for the pre-processing of nanostring mRNA and miRNA data. *Bioinformatics,* 2012, vol. 28, 1546-1548
- **RITCHIE, M. E. et al.** limma powers differential expression analyses for RNA742 sequencing and microarray studies. *Nucleic Acids Res,* 2015, vol. 43, e47
- R: A Language and Environment for Statistical Computing. R Foundation for Statistical Computing, 2016, vol. 0
- **PESARIN, F. ; SALMASO, L.** Permutation Tests for Complex Data. *Permutation Tests for Complex Data: Theory, Applications and Software,* 2010
- **BRUNNER, E. ; PURI, M. L.** Nonparametric methods in factorial designs. *Stat. Pap.,* 2001, vol. 42, 1-52
- **BRUNNER, E. ; DOMHOF, S. ; LANGER, F.** Nonparametric analysis of longitudinal data in factorial experiments. *Wiley Ser. Probab. Stat.,* 2002, vol. xvii, 261
- **NOGUCHI, K. ; GEL, Y. R. ; BRUNNER, E. ; KONIETSCHKE, F.** nparLD: An R software package for the nonparametric analysis of longitudinal data in factorial experiments. *J. Stat. Softw.,* 2012, vol. 50, 1-23
- **FEYS, J.** *New Nonparametric Rank Tests for Interactions in Factorial Designs with Repeated Measures New Nonparametric Rank Tests for.,* 2016, vol. 15
- **THERNEAU, T. M.** A Package for Survival Analysis. *S. Survival,* 2015